# EUROPEAN PATENT APPLICATION

(11) **EP 4 116 304 A1**
(43) Date of publication of application: **11.01.2023**
(21) Application number: 21764551.4
(22) Date of filing: 04.03.2021
(51) Int. Cl.: C07D 495/04, H01L 51/05, H01L 51/30, H01L 29/786

(54) **AROMATIC COMPOUND, ORGANIC SEMICONDUCTOR LAYER AND ORGANIC THIN FILM TRANSISTOR**

(30) Priority: 04.03.2020 JP 2020036737
(71) Applicant: Tosoh Corporation, Yamaguchi 746-8501 (JP)
(72) Inventor: MIYASHITA Masato, Yokkaichi-shi, Mie 510-8540 (JP); WATANABE Makoto, Yokkaichi-shi, Mie 510-8540 (JP); OKU Shinya, Yokkaichi-shi, Mie 510-8540 (JP)
(74) Representative: Novagraaf International SA
(86) International application number: PCT/JP2021/008511
(87) International publication number: WO 2021/177417

(57) **Abstract**

The purpose of the present invention is to provide: an aromatic compound having a specific substituent, said aromatic compound being a coating-type organic semiconductor material that has high carrier mobility, high heat resistance and adequate solubility; an organic semiconductor layer which uses this aromatic compound; and an organic thin film transistor. The purpose is achieved by an aromatic compound which is represented by formula (1-I) or (1-II).

In formulae (1-I) and (1-II), Ar represents a monocyclic ring or the like; each of X¹ and X² represents an oxygen atom or the like; each of Y¹ and Y² represents CR⁶ or the like; each of R¹ to R⁶ represents a hydrogen atom or the like; and at least one of the R¹ to R⁶ moieties is a group that is represented by formula (2).

In formula (2), A represents a specific alkenyl group or the like; each of 1 and n represents 0 or 1; m represents an integer from 1 to 20; and each of Z¹ and Z² represents a hydrogen atom or the like.

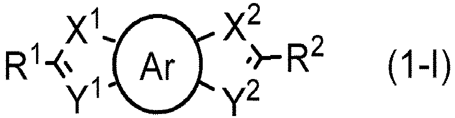

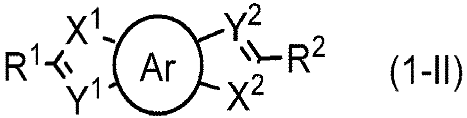

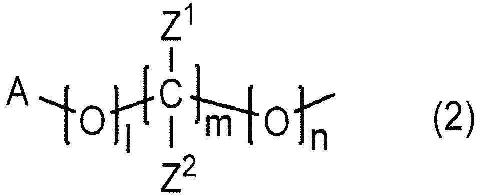

## Description

### TECHNICAL FIELD

The present invention relates to a novel aromatic compound that can be developed into an electronic material such as an organic semiconductor material; and an organic semiconductor layer and an organic thin film transistor using the aromatic compound. In particular, the present invention relates to an aromatic compound having a specific substituent that is applicable to various device fabrication processes because of its excellent solubility and heat resistance; and an organic semiconductor layer and an organic thin film transistor using the aromatic compound.

### BACKGROUND ART

An organic semiconductor device typified by an organic thin film transistor has been attracting attention in recent years because of its characteristics that are not found in an inorganic semiconductor device such as energy saving, low cost, and flexibility.

The organic semiconductor device is composed of several types of materials including an organic semiconductor layer, a substrate, an insulating layer, and an electrode. Among them, the organic semiconductor layer which is responsible for charged-carrier transfer plays a central role in the device. Since performance of the organic semiconductor device depends on carrier mobility of an organic semiconductor material that makes up the organic semiconductor layer, an organic semiconductor material that provides high carrier mobility has been desired to be developed.

A vacuum deposition method in which an organic material is vaporized at a high temperature and under vacuum and a coating method in which an organic material is dissolved in an appropriate solvent and the resulting solution is applied are generally known as methods for fabricating the organic semiconductor layer.

Among them, the coating method is an economically preferable process because it can also be performed using printing technology without using a high-temperature and high-vacuum condition, which is expected to significantly reduce a manufacturing cost for fabricating the device.

An organic semiconductor material for use in such a coating method preferably has, from the viewpoints of high carrier mobility and a device fabrication process, heat resistance of 130°C or more and solubility at room temperature of 0.1% by weight or more.

Furthermore, for a transistor for use in electronic paper or organic EL applications, the carrier mobility is preferably 1.0 cm²/V·sec or more.

Here, it is generally known that a small molecule semiconductor with a rod-shaped molecular long axis composed of a fused ring system can easily exhibit high carrier mobility due to its higher crystallinity than that of a polymer semiconductor.

However, the small molecule semiconductor generally has a problem of low solubility. Although a semiconductor into which an alkyl group is introduced in order to improve solubility has been reported, it has a problem with reduced carrier mobility and heat resistance.

A semiconductor into which an aromatic substituent is introduced for the purpose of achieving high carrier mobility by π-stacking has also been reported, but it has been reported to have significantly decreased solubility in exchange for the high mobility.

Therefore, at present, few small molecule organic semiconductor materials that combine high carrier mobility, high heat resistance, and appropriate solubility are known. Currently, small molecule materials such as 2,7-dialkyl-substituted benzothienobenzothiophene (e.g., see Patent Document 1 and Non-Patent Document 1), 2,7-diphenyl[1]benzothieno[3,2-b][1]benzothiophene (e.g., see Non-Patent Document 2), and a dithienobiphenylene derivative (e.g., see Patent Document 2) have been proposed.

However, the dialkyl-substituted benzothienobenzothiophene described in Patent Document 1 and Non-Patent Document 1 has a heat resistance problem in that transistor operation is lost when heated to 130°C or more.

The 2,7-diphenyl[1]benzothieno[3,2-b][1]benzothiophene described in Non-Patent Document 2 is generally almost insoluble in an organic solvent and thus has a solubility problem.

Furthermore, the alkyl-substituted dithienobiphenylene derivative described in Patent Document 2 combines high heat resistance and proper solubility, making it suitable for use in an organic semiconductor. However, a compound with even higher carrier mobility has been desired.
Patent Document 1: PCT International Publication No. WO2008/047896
Patent Document 2: Japanese Unexamined Patent Application, Publication No. 2018/174322
Non-Patent Document 1: Journal of the American Chemical Society, 2007, No. 129, pp. 15732-15733
Non-Patent Document 2: Journal of the American Chemical Society, 2006, No. 128, pp. 12604-12605

### DISCLOSURE OF THE INVENTION

### Problems to be Solved by the Invention

The present invention has been made in view of the above-described problem, and an object thereof is to provide a novel coating-type organic semiconductor material with high carrier mobility, high heat resistance, and appropriate solubility. Means for Solving the Problems

The present inventors conducted extensive studies to solve the above problem and have completed the present invention by finding that a novel aromatic compound having a specific substituent provides high carrier mobility and results in an organic semiconductor material with high heat resistance and appropriate solubility.

In other words, the present invention relates to an aromatic compound represented by Formula (1-I) or Formula (1-II) below; an organic semiconductor layer including the aromatic compound; and an organic thin film transistor including the semiconductor layer: 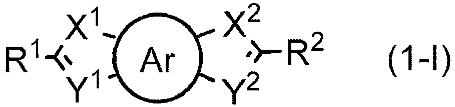 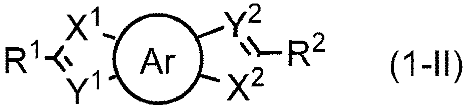
wherein Ar denotes a monocyclic ring or 2 to 6 fused ring;
X¹ and X² each independently denote one of the group consisting of an oxygen atom, a sulfur atom, a selenium atom, NR³, or
CR⁴=CR⁵;
Y¹ and Y² each independently denote CR⁶ or a nitrogen atom;
R¹ to R⁶ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by Formula (2) below; and at least one of R¹ to R⁶ is a group represented by Formula (2) below; 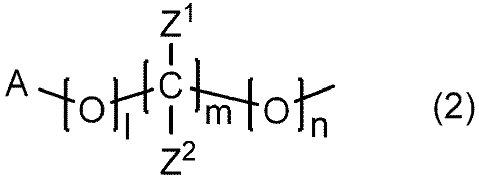
wherein A denotes one of the group consisting of an alkenyl group having 2 to 20 carbon atoms, an alkynyl group having 2 to 20 carbon atoms, or an aryl group having 4 to 26 carbon atoms;
1 and n each independently denote 0 or 1;
m denotes an integer of 1 to 20; and
Z¹ and Z² are the same or different at each occurrence and denote one of the group consisting of a hydrogen atom, a halogen atom, and an alkyl group having 1 to 20 carbon atoms.

### Effects of the Invention

A novel aromatic compound of the present invention provides high carrier mobility and has high heat resistance and appropriate solubility.

This makes it possible to provide an organic thin film transistor exhibiting an excellent semiconductor property via coating and its effect is extremely high.

### BRIEF DESCRIPTION OF THE DRAWINGS

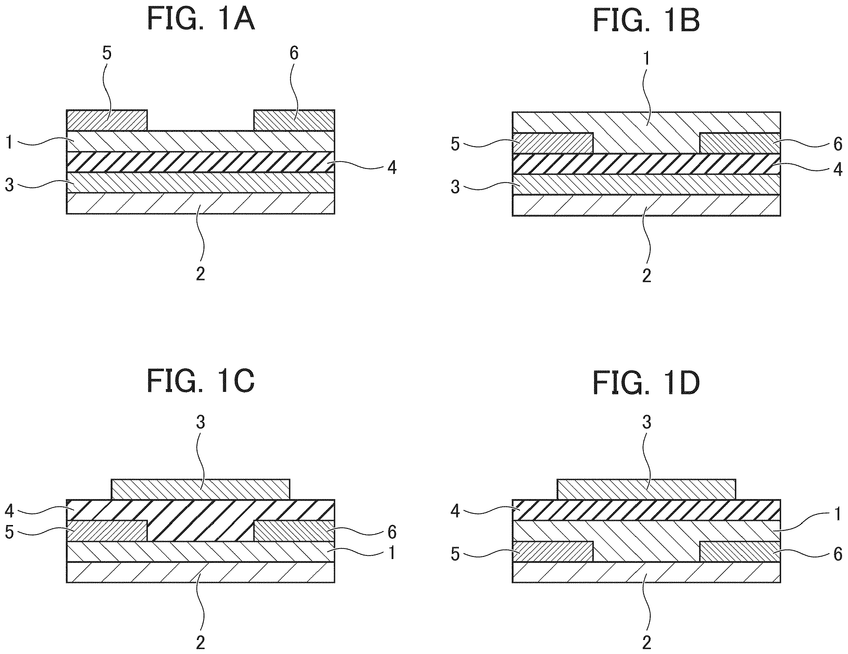

FIGS. 1A to 1D show cross-sectional configurations of an organic thin film transistor.

### PREFERRED MODE FOR CARRYING OUT THE INVENTION

The present invention will be described in detail.

The present invention is an aromatic compound represented by Formula (1-I) or Formula (1-II) above (hereinafter referred to as "compound of the present invention").

In Formula (1-I) or Formula (1-II), Ar denotes a monocyclic ring or 2 to 6 fused ring.

In order to exhibit higher carrier mobility, Ar is preferably a 2 to 4 fused ring. In order to exhibit higher solubility, Ar is more preferably a 2 or 3 fused ring.

Individual ring constituting the monocyclic ring or the 2 to 6 fused ring is 4 to 8-membered ring, with a 4 to 6-membered ring being preferred due to easier π-stacking.

Specific examples of the monocyclic ring or the 2 to 6 fused ring represented by Ar may include a monocyclic ring such as a cyclobutene ring, a thiophene ring, a furan ring, a selenophene ring, a thiazole ring, an oxazole ring, a pyrrole ring, an imidazole ring, a benzene ring, a pyridine ring, etc.; and a fused ring such as a thienothiophene ring, a naphthalene ring, a biphenylene ring, an anthracene ring, a dithienothiophene ring, a dithienobenzo ring, a benzothienobenzothiophene ring, a tetracene ring, a bis(dithieno)benzo ring, a bis(benzothieno)benzo ring, etc.; in order to exhibit higher carrier mobility, with a 2 to 4 fused ring such as a thienothiophene ring, a naphthalene ring, a biphenylene ring, an anthracene ring, a dithienothiophene ring, and a benzothienobenzothiophene ring being preferred, and with a 2 or 3 fused ring such as a thienothiophene ring, a naphthalene ring, a biphenylene ring, and an anthracene ring being more preferred.

In Formula (1-I) or Formula (1-II), X¹ and X² each independently denote one of the group consisting of an oxygen atom, a sulfur atom, a selenium atom, NR³, or CR⁴=CR⁵.

In order for the compound of the present invention to exhibit higher stability, at least one of X¹ and X² is preferably a sulfur atom or CR⁴=CR⁵ and further preferably a sulfur atom. In Formula (1-I) or Formula (1-II), Y¹ and Y² each independently denote CR⁶ or a nitrogen atom.

In order for the compound of the present invention to exhibit higher stability, at least one of Y¹ and Y² is preferably CR⁶.

In Formula (1-I) or Formula (1-II), R¹ to R⁶ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or an aryl group having 4 to 26 carbon atoms, or a group represented by Formula (2); and at least one of R¹ to R⁶ is a group represented by Formula (2).

The halogen atom in R¹ to R⁶ denotes, for example, a fluorine atom, a chlorine atom, a bromine atom, or an iodine atom, with a fluorine atom or a chlorine atom being preferred due to stability.

Examples of the alkyl group having 1 to 20 carbon atoms in R¹ to R⁶ include a linear, branched, or cyclic alkyl group such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, a 3-ethyldecyl group, a 2-hexyldecyl group, a cyclopentyl group, a cyclohexyl group, a cycloheptyl group, etc.

Among them, since an aromatic compound having high carrier mobility and high solubility is obtained, an alkyl group having 1 to 14 carbon atoms is preferred and a linear alkyl group having 1 to 14 carbon atoms including a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group, and an n-tetradecyl group is further preferred.

Examples of the alkenyl group having 2 to 20 carbon atoms in R¹ to R⁶ include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonel group, a 2-ethylheptenyl group, an n-decenyl group, an n-dodecenyl group, a cyclopentenyl-1-group, a cyclohexenyl-1-group, a cycloheptenyl-1-group, etc. Examples of the alkynyl group having 2 to 20 carbon atoms in R¹ to R⁶ include an ethynyl group, a propynyl group, a butynyl group, an n-pentynyl group, an n-hexynyl group, an n-heptynyl group, an n-octynyl group, an n-nonynyl group, an n-decynyl group, an n-dodecynyl group, etc.

Examples of the alkadienyl group having 4 to 22 carbon atoms in R¹ to R⁶ include a butadienyl group, a pentadienyl group, a hexadienyl group, an n-heptadienyl group, an n-octadienyl group, an n-nonadienyl group, an n-decadienyl group, an n-dodecadienyl group, an n-tridecadienyl group, etc.; with an alka-1,3-dienyl group having 4 to 22 carbon atoms being preferred and with a hexa-1,3-dienyl group, an n-hepta-1,3-dienyl group, an n-octa-1,3-dienyl group, an n-nona-1,3-dienyl group, or an n-deca-1,3-dienyl group being further preferred.

Examples of the alkadiynyl group having 4 to 22 carbon atoms in R¹ to R⁶ include a butadiynyl group, a pentadiynyl group, a hexadiynyl group, an n-heptadiynyl group, an n-octadiynyl group, an n-nonadiynyl group, an n-decadiynyl group, an n-dodecadiynyl group, an n-tridecadiynyl group, etc., with a 1,3-alkadiynyl group having 4 to 22 carbon atoms being preferred and with a hexa-1,3-diynyl group, an n-hepta-1,3-diynyl group, an n-octa-1,3-diynyl group, an n-nona-1,3-diynyl group, or an n-deca-1,3-diynyl group being further preferred. The aryl group having 4 to 26 carbon atoms in R¹ to R⁶ includes a heteroaryl group having 4 to 24 carbon atoms.

Examples of the aryl group having 4 to 26 carbon atoms may include a phenyl group; an alkyl-substituted phenyl group such as a p-tolyl group, a p-(n-hexyl)phenyl group, a p-(n-octyl)phenyl group, a p-(2-ethylhexyl)phenyl group, etc.; a 2-furyl group, a 2-thienyl group; an alkyl-substituted heteroaryl group such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group, a 5-(2-ethylhexyl)-2-thienyl group, etc.

For R¹ to R⁶, one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an aryl group having 4 to 26 carbon atoms, or a group represented by Formula (2) is preferred from the viewpoint of stability; and a hydrogen atom, an alkyl group having 1 to 20 carbon atoms, or a group represented by Formula (2) is more preferred from the viewpoint of high solubility. It is further preferred that only one or both of R¹ and R² denote a group represented by Formula (2) and R³ to R⁶ be a hydrogen atom from the viewpoint of high carrier mobility.

In Formula (2), A denotes one of the group consisting of an alkenyl group having 2 to 20 carbon atoms, an alkynyl group having 2 to 20 carbon atoms, or an aryl group having 4 to 26 carbon atoms, with an aryl group having 4 to 26 carbon atoms being more preferred in order to exhibit higher carrier mobility.

Examples of the alkenyl group having 2 to 20 carbon atoms in Formula (2) include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonel group, a 2-ethylheptenyl group, an n-decenyl group, an n-dodecenyl group, a cyclopentenyl-1-group, a cyclohexenyl-1-group, a cycloheptenyl-1-group, a 2-phenylethenyl group, a 1-phenylethenyl group, 2-(4-methylphenyl)ethenyl, 2-(4-n-butyl)ethenyl, etc.

Examples of the alkynyl group having 2 to 20 carbon atoms in Formula (2) include an ethynyl group, a propynyl group, a butynyl group, an n-pentynyl group, an n-hexynyl group, an n-heptynyl group, an n-octynyl group, an n-nonynyl group, an n-decynyl group, an n-dodecynyl group, a 2-phenylethynyl group, a 1-phenylethynyl group, 2-(4-methylphenyl)ethynyl, 2-(4-n-butyl)ethynyl, etc.

The aryl group having 4 to 26 carbon atoms in Formula (2) includes a heteroaryl group having 4 to 24 carbon atoms. Examples of the aryl group having 4 to 26 carbon atoms include a phenyl group; a alkyl-substituted phenyl group such as a p-tolyl group, a p-ethylphenyl group, a p-(n-propyl)phenyl group, a p-(isopropyl)phenyl group, a p-(n-butyl)phenyl group, a p-(2-methylpropyl)phenyl group, a p-(n-pentyl)phenyl group, a p-(3-methylbutyl)phenyl group, a p-(n-hexyl)phenyl group, a p-(4-methylpentyl)phenyl group, a p-(n-heptyl)phenyl group, a p-(n-octyl)phenyl group, a p-(2-ethylhexyl)phenyl group, an methylphenyl group, an m-(n-propyl)phenyl group, an m-(n-butyl)phenyl group, an m-(2-methylpropyl)phenyl group, an o-ethylphenyl group, an o-(n-propyl)phenyl group, an indanyl-5-yl group, etc.; an alkyloxy-substituted phenyl group such as a p-methoxyphenyl group, a p-ethoxyphenyl group, a p-(n-propyloxy)phenyl group, a p-(n-butylphenyloxy)phenyl group, a p-(n-pentyloxy)phenyl group, a p-(n-hexyloxy)phenyl group, a p-(n-heptyloxy)phenyl group, a p-(n-octyloxy)phenyl group, an m-methoxyphenyl group, an m-(n-propyloxy)phenyl group, an o-methoxyphenyl group, an o-(n-propyloxy)phenyl group, a 3,4-methylenedioxyphenyl group, a 3,4-ethylenedioxyphenyl group, 2,3-dihydrobenzofuran-5-yl, 2,3-dihydrobenzofuran-6-yl, etc.; a fluorine-substituted phenyl group such as a p-fluorophenyl group, an m-fluorophenyl group, an o-fluorophenyl group, a 3,5-difluorophenyl group, a perfluorophenyl group, a p-(trifluoromethyl)phenyl group, an m-(trifluoromethyl)phenyl group, an o-(trifluoromethyl)phenyl group, etc.; a 2-furyl group, a 2-thienyl group; an alkyl-substituted heteroaryl group such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group, a 5-(2-ethylhexyl)-2-thienyl group, thieno[3,2-b]thiophene-2-yl, 5-methylthieno[3,2-b]thiophene-2-yl, 5-ethylthieno[3,2-b]thiophene-2-yl, 5-(n-butyl)thieno[3,2-b]thiophene-2-yl, etc.

Among them, a phenyl group or an alkyl-substituted phenyl group is more preferred since it exhibits high carrier mobility and high solubility.

In Formula (2), 1 and n each independently denote 0 or 1, preferably n is 0 from the viewpoint of easy synthesis, and more preferably both of 1 and n are 0 from the viewpoint of high carrier mobility.

In Formula (2), m denotes an integer of 1 to 20, preferably m is an integer of 1 to 8 from the viewpoint of high solubility, more preferably m is an integer of 1 to 4 from the viewpoint of high carrier mobility, and further preferably n is 2.

In Formula (2), Z¹ and Z² are the same or different at each occurrence and denote one of the group consisting of a hydrogen atom, a halogen atom, and an alkyl group having 1 to 20 carbon atoms.

The halogen atom in Z¹ and Z² denotes, for example, a fluorine atom, a chlorine atom, a bromine atom, or an iodine atom, with a fluorine atom or a chlorine atom being preferred due to stability.

Examples of the alkyl group having 1 to 20 carbon atoms in Z¹ and Z² include a linear, branched, or cyclic alkyl group such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, 3-ethyldecyl, a 2-hexyldecyl group, a cyclopentyl group, a cyclohexyl group, a cycloheptyl group, etc.

Among them, since an aromatic compound having high carrier mobility and high solubility is obtained, an alkyl group having 1 to 8 carbon atoms is preferred and a methyl group, an ethyl group, or an n-propyl group is further preferred.

Z¹ and Z² are preferably a hydrogen atom or a halogen atom and more preferably a hydrogen atom from the viewpoint of high carrier mobility.

In Formula (2), it is preferable that A is a phenyl group or an alkyl-substituted phenyl group, both of 1 and n are 0, m is an integer of 1 to 4, and Z¹ and Z² are a hydrogen atom or a halogen atom.

This makes it possible for the compound of the present invention to exhibit higher carrier mobility.

The group represented by Formula (2) is preferably a 2-phenylethyl group or a 2-alkyl-substituted phenylethyl group from the viewpoint of high carrier mobility; more preferably a 2-phenylethyl group, a 2-(4-methylphenyl)ethyl group, a 2-(4-ethylphenyl)ethyl group, a 2-(4-n-propylphenyl)ethyl group, a 2-(4-n-butylphenyl)ethyl group, a 2-(4-n-pentylphenyl)ethyl group, a 2-(4-n-hexylphenyl)ethyl group, a 2-(4-n-heptylphenyl)ethyl group, a 2-(4-n-octylphenyl)ethyl group, a 2-(4-n-nonylphenyl)ethyl group, or a 2-(4-n-decylphenyl)ethyl group from the viewpoints of high heat resistance and high solubility; and further preferably a 2-phenylethyl group, a 2-(4-n-propylphenyl)ethyl group, a 2-(4-n-butylphenyl)ethyl group, a 2-(4-n-pentylphenyl)ethyl group, a 2-(4-n-hexylphenyl)ethyl group, or a 2-(4-n-heptylphenyl)ethyl group.

The aromatic compound of Formula (1-I) or Formula (1-II) is preferably an aromatic compound represented by one of the group consisting of Formulae (3-1) to (3-6) below from the viewpoint of easy synthesis: 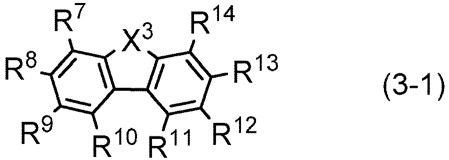 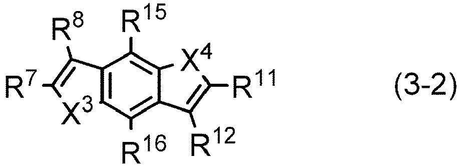 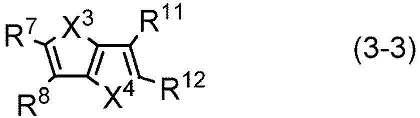 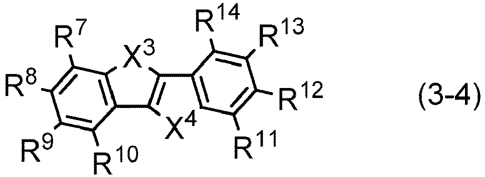 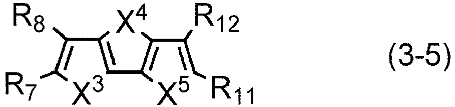 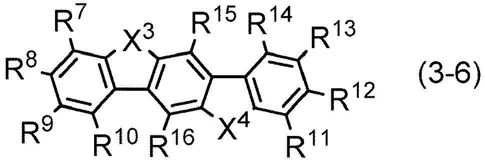
wherein X³, X⁴, and X⁵ each independently denote one of the group consisting of an oxygen atom, a sulfur atom, a selenium atom, a single bond, NR¹⁷, or CR¹⁸=CR¹⁹;
only one of combinations consisting of adjacent two of R⁷ to R¹⁰ constitutes Formula (4) below and only one of combinations consisting of adjacent two of R¹¹ to R¹⁴ constitutes Formula (4-2) below, each of which forms a 5- or 6-membered ring; and remainders of R⁷ to R¹⁴ that do not constitute Formula (4) or Formula (4-2) below and R¹⁵ to R¹⁹ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by the above Formula (2); 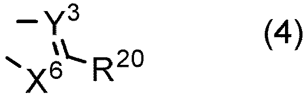
wherein X⁶ denotes one of the group consisting of an oxygen atom, a sulfur atom, a selenium atom, CR²¹=CR²², or NR²³;
Y³ denotes CR²⁴ or a nitrogen atom;
R²¹ to R²⁴ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by the above Formula (2); and R²⁰ is a group represented by the above Formula (2); 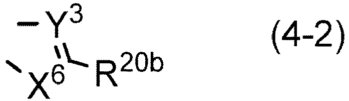
wherein X⁶, Y³, and R²¹ to R²⁴ have the same meaning as for X⁶, Y³, and R²¹ to R²⁴ in the above Formula (4); and R^{20b} denotes one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2).

In Formulae (3-1) to (3-6), only one of combinations consisting of adjacent two of R⁷ to R¹⁰ constitutes Formula (4) above and only one of combinations consisting of adjacent two of R¹¹ to R¹⁴ constitutes Formula (4-2) above, each of which forms a 5- or 6-membered ring.

Remainders of R⁷ to R¹⁴ that do not constitute Formula (4) or Formula (4-2), R¹⁵ to R¹⁹, and R²¹ to R²⁴ in Formulae (4) and (4-2) each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or an aryl group having 4 to 26 carbon atoms, or a group represented by Formula (2).

R²⁰ is a group represented by Formula (2).

R^{20b} is a group represented by one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by Formula (2), with one of the group consisting of a hydrogen atom, a fluorine atom, or a group represented by Formula (2) being preferred due to high mobility.

In the compound represented by one of the group consisting of Formulae (3-1) to (3-6), Formula (4-2) is preferably Formula (4-3) below: 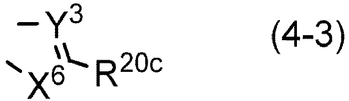 wherein X⁶, Y³, and R²¹ to R²⁴ have the same meaning as for X⁶, Y³, and R²¹ to R²⁴ in the above Formula (4); and R^{20c} denotes one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or a group represented by an aryl group having 4 to 26 carbon atoms.

R^{20c} is one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or a group represented by an aryl group having 4 to 26 carbon atoms, with a hydrogen atom or a fluorine atom being preferred and with a hydrogen atom being more preferred due to high mobility.

In the compound represented by one of the group consisting of Formulae (3-1) to (3-6), Formula (4-2) is also preferably Formula (4-4) below: 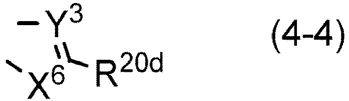 wherein X⁶, Y³, and R²¹ to R²⁴ have the same meaning as for X⁶, Y³, and R²¹ to R²⁴ in the above Formula (4); and R^{20d} is a group represented by the above Formula (2).

Note that, the group represented by Formula (2) in Formulae (3-1) to (3-6) is defined in the same way as for Formula (2) in Formulae (1-I) and (1-II) mentioned above.

The halogen atom in R⁷ to R¹⁹, R²¹ to R²⁴, R^{20b}, and R^{20c} denotes, for example, a fluorine atom, a chlorine atom, a bromine atom, or an iodine atom, with a fluorine atom or a chlorine atom being preferred due to stability.

Examples of the alkyl group having 1 to 20 carbon atoms in R⁷ to R¹⁹, R²¹ to R²⁴, R^{20b}, and R^{20c} include a linear, branched, or cyclic alkyl group such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, 3-ethyldecyl, a 2-hexyldecyl group, a cyclopentyl group, a cyclohexyl group, a cycloheptyl group, etc.

Among them, since an aromatic compound having high carrier mobility and high solubility is obtained, an alkyl group having 1 to 14 carbon atoms is preferred and a linear alkyl group having 1 to 14 carbon atoms including a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group, and an n-tetradecyl group is further preferred.

Examples of the alkenyl group having 2 to 20 carbon atoms in R⁷ to R²⁴ include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonel group, a 2-ethylheptenyl group, an n-decenyl group, an n-dodecenyl group, a cyclopentenyl-1-group, a cyclohexenyl-1-group, a cycloheptenyl-1-group, etc. Examples of the alkynyl group having 2 to 20 carbon atoms in R⁷ to R¹⁹, R²¹ to R²⁴, R^{20b}, and R^{20c} include an ethynyl group, a propynyl group, a butynyl group, an n-pentynyl group, an n-hexynyl group, an n-heptynyl group, an n-octynyl group, an n-nonynyl group, an n-decynyl group, an n-dodecynyl group, etc.

Examples of the alkadienyl group having 4 to 22 carbon atoms in R⁷ to R¹⁹, R²¹ to R²⁴, R^{20b}, and R^{20c} include a butadienyl group, a pentadienyl group, a hexadienyl group, an n-heptadienyl group, an n-octadienyl group, an n-nonadienyl group, an n-decadienyl group, an n-dodecadienyl group, an n-tridecadienyl group, etc.; with an alka-1,3-dienyl group having 4 to 22 carbon atoms being preferred and with a hexa-1,3-dienyl group, an n-hepta-1,3-dienyl group, an n-octa-1,3-dienyl group, an n-nona-1,3-dienyl group, or an n-deca-1,3-dienyl group being further preferred.

Examples of the alkadiynyl group having 4 to 22 carbon atoms in R⁷ to R¹⁹, R²¹ to R²⁴, R^{20b}, and R^{20c} include a butadiynyl group, a pentadiynyl group, a hexadiynyl group, an n-heptadiynyl group, an n-octadiynyl group, an n-nonadiynyl group, an n-decadiynyl group, an n-dodecadiynyl group, an n-tridecadiynyl group, with a 1,3-alkadiynyl group having 4 to 22 carbon atoms being preferred and with a hexa-1,3-diynyl group, an n-hepta-1,3-diynyl group, an n-octa-1,3-diynyl group, an n-nona-1,3-diynyl group, or an n-deca-1,3-diynyl group being further preferred.

The aryl group having 4 to 26 carbon atoms in R⁷ to R¹⁹, R²¹ to R²⁴, R^{20b}, and R^{20c} includes a heteroaryl group having 4 to 24 carbon atoms.

Examples of the aryl group having 4 to 26 carbon atoms include a phenyl group; an alkyl-substituted phenyl group such as a p-tolyl group, a p-(n-hexyl)phenyl group, a p-(n-octyl)phenyl group, a p-(2-ethylhexyl)phenyl group, etc.; a 2-furyl group, a 2-thienyl group; and an alkyl-substituted heteroaryl group such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group, a 5-(2-ethylhexyl)-2-thienyl group, etc.

R⁷ to R¹⁹ are preferably one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an aryl group having 4 to 26 carbon atoms, or a group represented by Formula (2) from the viewpoint of stability and more preferably a hydrogen atom or an alkyl group having 1 to 20 carbon atoms from the viewpoint of high solubility.

R²¹ to R²⁴ are preferably one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an aryl group having 4 to 26 carbon atoms, or the group represented by Formula (2) from the viewpoint of stability, and R²¹ to R²⁴ are more preferably selected from one of the group consisting of a hydrogen atom and a methyl group and further more preferably a hydrogen atom from the viewpoint of high carrier mobility.

In Formulae (3-1) to (3-6), X³, X⁴, and X⁵ denote one of the group consisting of an oxygen atom, a sulfur atom, a selenium atom, a single bond, NR¹⁷, or CR¹⁸=CR¹⁹, with a sulfur atom, a single bond, or CR¹⁸=CR¹⁹ being preferred, with a sulfur atom or CR¹⁸=CR¹⁹ being more preferred, and with a sulfur atom being further more preferred from the viewpoint of high carrier mobility.

In Formulae (4) and (4-2) to (4-3), X⁶ denotes one of the group consisting of an oxygen atom, a sulfur atom, a selenium atom, CR²¹=CR²², or NR²³, with a sulfur atom or CR²¹=CR²² being preferred and with a sulfur atom being more preferred from the viewpoint of high carrier mobility.

In Formulae (4) and (4-2) to (4-3), Y³ denotes CR²⁴ or a nitrogen atom, with CR²⁴ being preferred from the viewpoint of stability.

The aromatic compounds represented by Formulae (3-1) to (3-6) preferably have point-symmetric or axial-symmetric structure and more preferably point-symmetric structure from the viewpoint of high carrier mobility.

The aromatic compounds represented by Formulae (3-1) to (3-6) are preferably represented by Formula (3-1) or (3-2) from the viewpoints of high solubility and high heat resistance.

The aromatic compound represented by Formula (1-I) or Formula (1-II) is preferably an aromatic compound represented by Formula (5) or (5-2) below: 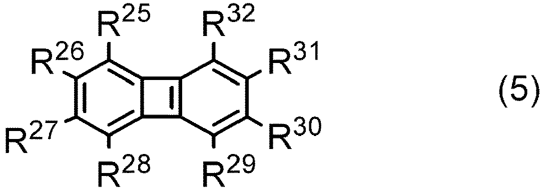 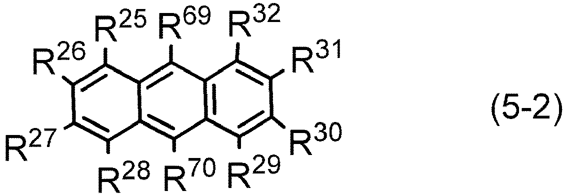
wherein only one of combinations consisting of adjacent two of R²⁵ to R²⁸ constitutes Formula (6) below and only one of combinations consisting of adjacent two of R²⁹ to R³² constitutes Formula (6-2) below, each of which forms a 5- or 6-membered ring; and
remainders of R²⁵ to R³² that do not constitute Formula (6) or Formula (6-2) below and R⁶⁹ and R⁷⁰ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2); 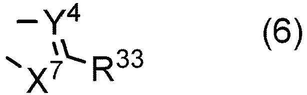
wherein X⁷ denotes an oxygen atom, a sulfur atom, a selenium atom, CR³⁴=CR³⁵, or NR³⁶;
Y⁴ denotes CR³⁷ or a nitrogen atom;
R³⁴ to R³⁷ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by the above Formula (2); and R³³ is a group represented by the above Formula (2); 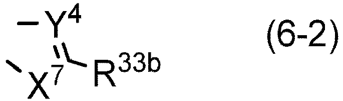
wherein X⁷, Y⁴, and R³⁴ to R³⁷ have the same meaning as for X⁷, Y⁴, and R³⁴ to R³⁷ in the above Formula (6); and R^{33b} denotes one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by the above Formula (2).

In Formulae (5) and (5-2), only one of combinations consisting of adjacent two of R²⁵ to R²⁸ constitutes Formula (6) above and only one of combinations consisting of adjacent two of R²⁹ to R³² constitutes Formula (6-2) above, each of which forms a 5- or 6-membered ring.

Remainders of R²⁵ to R³² that do not constitute Formula (6) or Formula (6-2), R⁶⁹, R⁷⁰, and R³⁴ to R³⁷ in Formulae (6) and (6-2) each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or an aryl group having 4 to 26 carbon atoms, or a group represented by Formula (2).

R³³ is a group represented by Formula (2).

R^{33b} is a group represented by one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by Formula (2), with one of the group consisting of a hydrogen atom, a fluorine atom, and a group represented by Formula (2) being preferred due to high mobility.

In the aromatic compound represented by Formula (5) or (5-2), Formula (6-2) is preferably Formula (6-3) below: 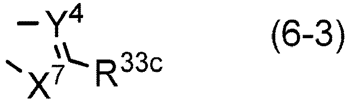 wherein X⁷, Y⁴, and R³⁴ to R³⁷ have the same meaning as for X⁷, Y⁴, and R³⁴ to R³⁷ in the above Formula (6); and R^{33c} denotes one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or an aryl group having 4 to 26 carbon atoms.

R^{33c} is one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or a group represented by an aryl group having 4 to 26 carbon atoms, with a hydrogen atom or a fluorine atom being preferred and with a hydrogen atom being more preferred due to high mobility.

In the aromatic compound represented by Formula (5) or (5-2), Formula (6-2) is also preferably Formula (6-4) below: 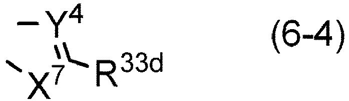 wherein X⁷, Y⁴, and R³⁴ to R³⁷ have the same meaning as for X⁷, Y⁴, and R³⁴ to R³⁷ in the above Formula (6); and R^{33d} is a group represented by the above Formula (2).

Note that, the group represented by Formula (2) in Formulae (5) and (5-2) is defined in the same way as for Formula (2) in Formulae (1-I) and (1-II) mentioned above.

The halogen atom in R²⁵ to R³², R³⁴ to R³⁷, R⁶⁹, R⁷⁰, R^{33b}, and R^{33c} denotes, for example, a fluorine atom, a chlorine atom, a bromine atom, or an iodine atom, with a fluorine atom or a chlorine atom being preferred due to stability.

Examples of the alkyl group having 1 to 20 carbon atoms in R²⁵ to R³², R³⁴ to R³⁷, R⁶⁹, R⁷⁰, R^{33b}, and R^{33c} include a linear, branched, or cyclic alkyl group such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, 3-ethyldecyl, a 2-hexyldecyl group, a cyclopentyl group, a cyclohexyl group, a cycloheptyl group, etc.

Among them, since an aromatic compound having high carrier mobility and high solubility is obtained, an alkyl group having 1 to 14 carbon atoms is preferred and a linear alkyl group having 1 to 14 carbon atoms including a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group, and an n-tetradecyl group is further preferred.

Examples of the alkenyl group having 2 to 20 carbon atoms in R²⁵ to R³², R³⁴ to R³⁷, R⁶⁹, R⁷⁰, R^{33b}, and R^{33c} include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonel group, a 2-ethylheptenyl group, an n-decenyl group, an n-dodecenyl group, a cyclopentenyl-1-group, a cyclohexenyl-1-group, a cycloheptenyl-1-group, etc.

Examples of the alkynyl group having 2 to 20 carbon atoms in R²⁵ to R³², R³⁴ to R³⁷, R⁶⁹, R⁷⁰, R^{33b}, and R^{33c} include an ethynyl group, a propynyl group, a butynyl group, an n-pentynyl group, an n-hexynyl group, an n-heptynyl group, an n-octynyl group, an n-nonynyl group, an n-decynyl group, an n-dodecynyl group, etc.

Examples of the alkadienyl group having 4 to 22 carbon atoms in R²⁵ to R³², R³⁴ to R³⁷, R⁶⁹, R⁷⁰, R^{33b}, and R^{33c} include a butadienyl group, a pentadienyl group, a hexadienyl group, an n-heptadienyl group, an n-octadienyl group, an n-nonadienyl group, an n-decadienyl group, an n-dodecadienyl group, an n-tridecadienyl group, etc., with an alka-1,3-dienyl group having 4 to 22 carbon atoms being preferred and with a hexa-1,3-dienyl group, an n-hepta-1,3-dienyl group, an n-octa-1,3-dienyl group, an n-nona-1,3-dienyl group, or an n-deca-1,3-dienyl group being further preferred.

Examples of the alkadiynyl group having 4 to 22 carbon atoms in R²⁵ to R³², R³⁴ to R³⁷, R⁶⁹, R⁷⁰, R^{33b}, and R^{33c} include a butadiynyl group, a pentadiynyl group, a hexadiynyl group, an n-heptadiynyl group, an n-octadiynyl group, an n-nonadiynyl group, an n-decadiynyl group, an n-dodecadiynyl group, an n-tridecadiynyl group, etc., with a 1,3-alkadiynyl group having 4 to 22 carbon atoms being preferred and with a hexa-1,3-diynyl group, an n-hepta-1,3-diynyl group, an n-octa-1,3-diynyl group, an n-nona-1,3-diynyl group, or an n-deca-1,3-diynyl group being further preferred.

The aryl group having 4 to 26 carbon atoms in R²⁵ to R³², R³⁴ to R³⁷, R⁶⁹, R⁷⁰, R^{33b}, and R^{33c} includes a heteroaryl group having 4 to 24 carbon atoms.

Examples of the aryl group having 4 to 26 carbon atoms include a phenyl group; an alkyl-substituted phenyl group such as a p-tolyl group, a p-(n-hexyl)phenyl group, a p-(n-octyl)phenyl group, a p-(2-ethylhexyl)phenyl group, etc.; a 2-furyl group, a 2-thienyl group; and an alkyl-substituted heteroaryl group such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group, a 5-(2-ethylhexyl)-2-thienyl group, etc.

R²⁵ to R³², R⁶⁹, and R⁷⁰ are preferably one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an aryl group having 4 to 26 carbon atoms, or a group represented by Formula (2) from the viewpoint of stability, more preferably a hydrogen atom, an alkyl group having 1 to 20 carbon atoms from the viewpoint of high solubility, and further preferably selected from one of the group consisting of a hydrogen atom and a methyl group.

R³⁴ to R³⁷ are preferably one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an aryl group having 4 to 26 carbon atoms, or a group represented by Formula (2) from the viewpoint of stability, more preferably a hydrogen atom, an alkyl group having 1 to 20 carbon atoms, or a group represented by Formula (2) from the viewpoint of high carrier mobility, and R³⁴ to R³⁷ are further more preferably a hydrogen atom.

In Formulae (6) and (6-2) to (6-4), X⁷ denotes an oxygen atom, a sulfur atom, a selenium atom, CR³⁴=CR³⁵, or NR³⁶, with a sulfur atom, an oxygen atom, a sulfur atom, or a selenium atom being preferred from the viewpoint of high solubility and with a sulfur atom being further preferred from the viewpoint of high carrier mobility.

In Formulae (6) and (6-2) to (6-4), Y⁴ denotes CR³⁷ or a nitrogen atom, with CR³⁷ being preferred from the viewpoint of stability.

The aromatic compounds represented by Formulae (5) and (5-2) preferably have point-symmetric or axial-symmetric structure and more preferably point-symmetric structure from the viewpoint of high carrier mobility.

The aromatic compounds represented by Formulae (5) and (5-2) are preferably represented by Formula (5) from the viewpoint of high solubility.

Furthermore, the aromatic compound represented by Formula (5) or (5-2) is preferably a compound of one of the group consisting of Formulae (7-1) to (7-5) below due to easy synthesis: 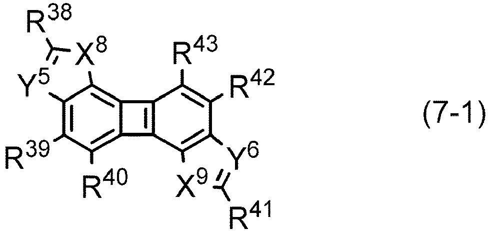 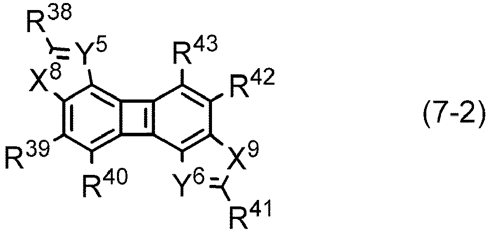 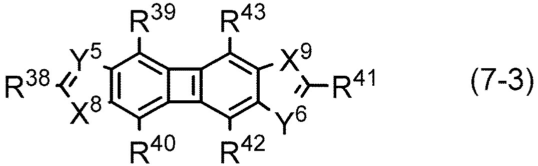 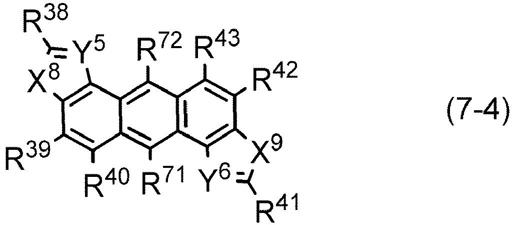 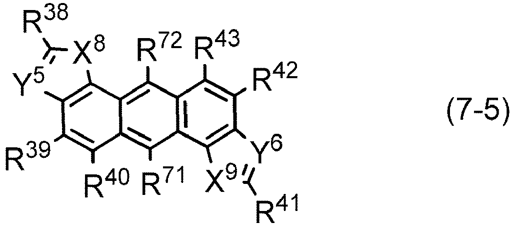
wherein X⁸ and X⁹ each independently denote one of the group consisting of an oxygen atom, a sulfur atom, a selenium atom, or NR⁴⁴;
Y⁵ and Y⁶ each independently denote CR⁴⁵ or a nitrogen atom;
R³⁸ to R⁴⁵, R⁷¹, and R⁷² each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by Formula (2); at least one of R³⁸ to R⁴⁵ is a group represented by Formula (2); and at least one of R³⁸ and R⁴¹ is a group represented by Formula (2).

In Formulae (7-1) to (7-5), X⁸ and X⁹ each independently denote one of the group consisting of an oxygen atom, a sulfur atom, a selenium atom, and NR⁴⁴, with a sulfur atom, an oxygen atom, a sulfur atom, or a selenium atom being preferred from the viewpoint of stability and with a sulfur atom being further preferred from the viewpoint of high carrier mobility. In Formulae (7-1) to (7-5), Y⁵ and Y⁶ each independently denote CR⁴⁵ or a nitrogen atom, with CR⁴⁵ being preferred from the viewpoint of stability.

In Formulae (7-1) to (7-5), R³⁸ to R⁴⁵, R⁷¹, and R⁷² each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by Formula (2) above; and at least one of R³⁸ to R⁴⁵ is a group represented by Formula (2) above.

In Formulae (7-1) to (7-5), only one or both of R³⁸ and R⁴¹ are a group represented by Formula (2).

Note that, the group represented by Formula (2) in Formulae (7-1) to (7-5) is defined in the same way as for Formula (2) in Formulae (1-I) and (1-II) mentioned above.

The halogen atom in R³⁸ to R⁴⁵, R⁷¹, and R⁷² denotes, for example, a fluorine atom, a chlorine atom, a bromine atom, or an iodine atom, with a fluorine atom or a chlorine atom being preferred due to stability.

Examples of the alkyl group having 1 to 20 carbon atoms in R³⁸ to R⁴⁵, R⁷¹, and R⁷² include a linear, branched, or cyclic alkyl group such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, 3-ethyldecyl, a 2-hexyldecyl group, a cyclopentyl group, a cyclohexyl group, a cycloheptyl group, etc.

Among them, since an aromatic compound having high carrier mobility and high solubility is obtained, an alkyl group having 1 to 14 carbon atoms is preferred and a linear alkyl group having 1 to 14 carbon atoms including a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group, and an n-tetradecyl group is further preferred.

Examples of the alkenyl group having 2 to 20 carbon atoms in R³⁸ to R⁴⁵, R⁷¹, and R⁷² include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonel group, a 2-ethylheptenyl group, an n-decenyl group, an n-dodecenyl group, a cyclopentenyl-1-group, a cyclohexenyl-1-group, a cycloheptenyl-1-group, etc.

Examples of the alkynyl group having 2 to 20 carbon atoms in R³⁸ to R⁴⁵, R⁷¹, and R⁷² include an ethynyl group, a propynyl group, a butynyl group, an n-pentynyl group, an n-hexynyl group, an n-heptynyl group, an n-octynyl group, an n-nonynyl group, an n-decynyl group, an n-dodecynyl group, etc.

Examples of the alkadienyl group having 4 to 22 carbon atoms in R³⁸ to R⁴⁵, R⁷¹, and R⁷² include a butadienyl group, a pentadienyl group, a hexadienyl group, an n-heptadienyl group, an n-octadienyl group, an n-nonadienyl group, an n-decadienyl group, an n-dodecadienyl group, an n-tridecadienyl group, etc., with an alka-1,3-dienyl group having 4 to 22 carbon atoms being preferred and with a hexa-1,3-dienyl group, an n-hepta-1,3-dienyl group, an n-octa-1,3-dienyl group, an n-nona-1,3-dienyl group, or an n-deca-1,3-dienyl group being further preferred.

Examples of the alkadiynyl group having 4 to 22 carbon atoms in R³⁸ to R⁴⁵, R⁷¹, and R⁷² include a butadiynyl group, a pentadiynyl group, a hexadiynyl group, an n-heptadiynyl group, an n-octadiynyl group, an n-nonadiynyl group, an n-decadiynyl group, an n-dodecadiynyl group, an n-tridecadiynyl group, etc., with a 1,3-alkadiynyl group having 4 to 22 carbon atoms being preferred and with a hexa-1,3-diynyl group, an n-hepta-1,3-diynyl group, an n-octa-1,3-diynyl group, an n-nona-1,3-diynyl group, or an n-deca-1,3-diynyl group being further preferred.

The aryl group having 4 to 26 carbon atoms in R³⁸ to R⁴⁵, R⁷¹, and R⁷² includes a heteroaryl group having 4 to 24 carbon atoms.

Examples of the aryl group having 4 to 26 carbon atoms include a phenyl group; an alkyl-substituted phenyl group such as a p-tolyl group, a p-(n-hexyl)phenyl group, a p-(n-octyl)phenyl group, a p-(2-ethylhexyl)phenyl group, etc.; a 2-furyl group, a 2-thienyl group; and an alkyl-substituted heteroaryl group such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group, a 5-(2-ethylhexyl)-2-thienyl group, etc.

R³⁸ to R⁴⁵, R⁷¹, and R⁷² are preferably one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an aryl group having 4 to 26 carbon atoms, or a group represented by Formula (2) from the viewpoint of stability and more preferably a hydrogen atom, an alkyl group having 1 to 20 carbon atoms, or a group represented by Formula (2) from the viewpoint of high solubility.

From the viewpoint of high carrier mobility, R³⁸ and R⁴¹ are preferably one of the group consisting of a group represented by Formula (2) above, a hydrogen atom, and a fluorine atom and more preferably one of the group consisting of a group represented by Formula (2) and a hydrogen atom. Further preferably, both of R³⁸ and R⁴¹ are a group represented by Formula (2) above.

R³⁹, R⁴⁰, R⁴² to R⁴⁵, R⁷¹, and R⁷² are further more preferably selected from one of the group consisting of a hydrogen atom and a methyl group and yet more preferably a hydrogen atom.

The aromatic compounds represented by Formulae (7-1) to (7-5) above preferably have point-symmetric or axial-symmetric structure and more preferably point-symmetric structure from the viewpoint of high carrier mobility.

Among Formulae (7-1) to (7-5), Formula (7-1), (7-2), or (7-5) is preferred from the viewpoint of high solubility and Formula (7-1) is more preferred from the viewpoint of high carrier mobility.

A more preferred compound structure of the compound of the present invention is represented by one of the group consisting of Formulae (7-1) to (7-5) above and Formulae (8-1) to (8-11) below.

Among them, an aromatic compound represented by one of the group consisting of Formulae (7-1) to (7-5) and (8-2) to (8-9) which is a 4 to 5 fused ring is preferred from the viewpoints of high heat resistance and high solubility and an aromatic compound represented by one of the group consisting of Formulae (7-1) to (7-5), (8-2) to (8-3), and (8-5) to (8-8) which has point-symmetric structure is further preferred from the viewpoint of high carrier mobility: 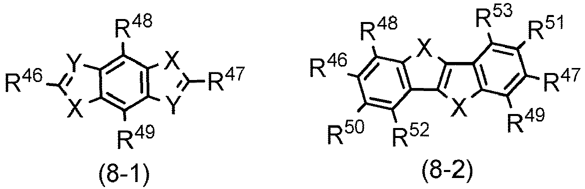 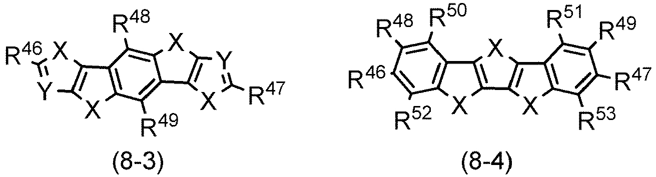 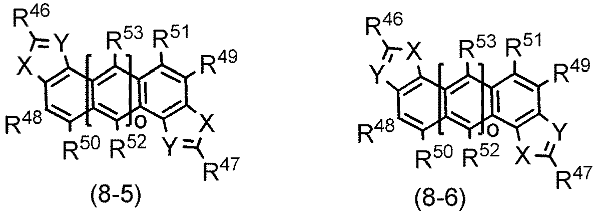 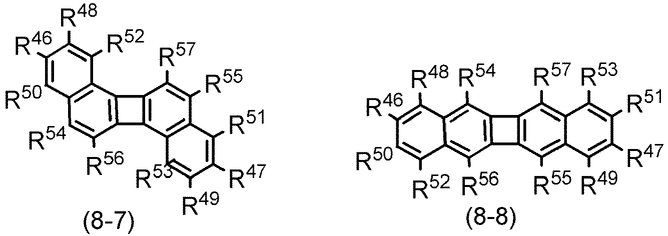 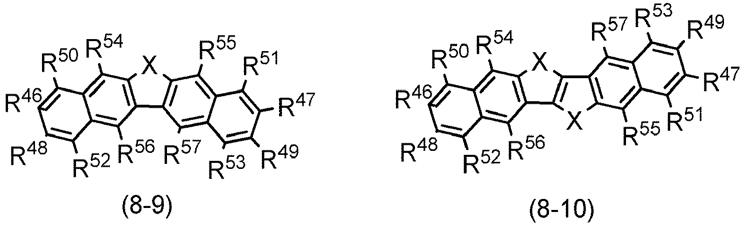 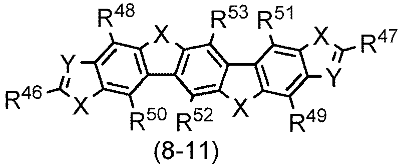
wherein X denotes one of the group consisting of an oxygen atom, a sulfur atom, a selenium atom, NR⁵⁸, or CR⁵⁹=CR⁶⁰;
Y denotes CR⁶¹ or a nitrogen atom;
R⁴⁶ to R⁶¹ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by Formula (2); at least one of R⁴⁶ to R⁶¹ is a group represented by Formula (2); at least one of R⁴⁶ and R⁴⁷ is a group represented by Formula (2); and o denotes 0 or 1.

In Formulae (8-1) to (8-11), X denotes one of the group consisting of an oxygen atom, a sulfur atom, a selenium atom, NR⁵⁸, or CR⁵⁹=CR⁶⁰; with one of the group consisting of an oxygen atom, a sulfur atom, and a selenium atom being preferred from the viewpoint of stability and with a sulfur atom being further preferred from the viewpoint of high carrier mobility.

In Formulae (8-1) to (8-11), Y denotes CR⁶¹ or a nitrogen atom, with CR⁶¹ being preferred from the viewpoint of stability.

In Formulae (8-1) to (8-11), R⁴⁶ to R⁶¹ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by Formula (2); and at least one of R⁴⁶ to R⁶¹ is a group represented by Formula (2).

Note that, the group represented by Formula (2) in Formulae (8-1) to (8-11) is defined in the same way as for Formulae (1-I) and (1-II) mentioned above.

The halogen atom in R⁴⁶ to R⁶¹ denotes, for example, a fluorine atom, a chlorine atom, a bromine atom, or an iodine atom, with a fluorine atom or a chlorine atom being preferred due to stability.

Examples of the alkyl group having 1 to 20 carbon atoms in R⁴⁶ to R⁶¹ include a linear, branched, or cyclic alkyl group such as a methyl group, an ethyl group, an n-propyl group, an isopropyl group, an n-butyl group, an isobutyl group, an n-pentyl group, an isovaleryl group, an n-hexyl group, an isohexyl group, an n-heptyl group, an n-octyl group, an n-nonyl group, an n-decyl group, an n-undecyl group, an n-dodecyl group, an n-tridecyl group, an n-tetradecyl group, an n-octadecyl group, a 2-ethylhexyl group, a 3-ethylheptyl group, 3-ethyldecyl, a 2-hexyldecyl group, a cyclopentyl group, a cyclohexyl group, a cycloheptyl group, etc.

Among them, since an aromatic compound having high carrier mobility and high solubility is obtained, an alkyl group having 1 to 14 carbon atoms is preferred and a linear alkyl group having 1 to 14 carbon atoms including a methyl group, an ethyl group, an n-propyl group, an n-butyl group, an n-pentyl group, an n-hexyl group, an n-heptyl group, an n-octyl group, an n-nonyl group, an n-decyl group, an n-dodecyl group, an n-tridecyl group, and an n-tetradecyl group is further preferred.

Examples of the alkenyl group having 2 to 20 carbon atoms in R⁴⁶ to R⁶¹ include an ethenyl group, a propenyl group, a butenyl group, a 2-methylpropenyl group, an n-pentenyl group, a 2-methylbutenyl group, an n-hexenyl group, a 2-methylpentenyl group, an n-heptenyl group, an n-octenyl group, a 2-ethylhexenyl group, an n-nonel group, a 2-ethylheptenyl group, an n-decenyl group, an n-dodecenyl group, a cyclopentenyl-1-group, a cyclohexenyl-1-group, a cycloheptenyl-1-group, etc. Examples of the alkynyl group having 2 to 20 carbon atoms in R⁴⁶ to R⁶¹ include an ethynyl group, a propynyl group, a butynyl group, an n-pentynyl group, an n-hexynyl group, an n-heptynyl group, an n-octynyl group, an n-nonynyl group, an n-decynyl group, an n-dodecynyl group, etc.

Examples of the alkadienyl group having 4 to 22 carbon atoms in R⁴⁶ to R⁶¹ include a butadienyl group, a pentadienyl group, a hexadienyl group, an n-heptadienyl group, an n-octadienyl group, an n-nonadienyl group, an n-decadienyl group, an n-dodecadienyl group, an n-tridecadienyl group, etc., with an alka-1,3-dienyl group having 4 to 22 carbon atoms being preferred and with a hexa-1,3-dienyl group, an n-hepta-1,3-dienyl group, an n-octa-1,3-dienyl group, an n-nona-1,3-dienyl group, or an n-deca-1,3-dienyl group being further preferred.

Examples of the alkadiynyl group having 4 to 22 carbon atoms in R⁴⁶ to R⁶¹ include a butadiynyl group, a pentadiynyl group, a hexadiynyl group, an n-heptadiynyl group, an n-octadiynyl group, an n-nonadiynyl group, an n-decadiynyl group, an n-dodecadiynyl group, an n-tridecadiynyl group, etc., with a 1,3-alkadiynyl group having 4 to 22 carbon atoms being preferred and with a hexa-1,3-diynyl group, an n-hepta-1,3-diynyl group, an n-octa-1,3-diynyl group, an n-nona-1,3-diynyl group, or an n-deca-1,3-diynyl group being further preferred. The aryl group having 4 to 26 carbon atoms in R⁴⁶ to R⁶¹ includes a heteroaryl group having 4 to 24 carbon atoms. Examples of the aryl group having 4 to 26 carbon atoms include a phenyl group; an alkyl-substituted phenyl group such as a p-tolyl group, a p-(n-hexyl)phenyl group, a p-(n-octyl)phenyl group, a p-(2-ethylhexyl)phenyl group, etc.; a 2-furyl group, a 2-thienyl group; and an alkyl-substituted heteroaryl group such as a 5-fluoro-2-furyl group, a 5-methyl-2-furyl group, a 5-ethyl-2-furyl group, a 5-(n-propyl)-2-furyl group, a 5-(n-butyl)-2-furyl group, a 5-(n-pentyl)-2-furyl group, a 5-(n-hexyl)-2-furyl group, a 5-(n-octyl)-2-furyl group, a 5-(2-ethylhexyl)-2-furyl group, a 5-fluoro-2-thienyl group, a 5-methyl-2-thienyl group, a 5-ethyl-2-thienyl group, a 5-(n-propyl)-2-thienyl group, a 5-(n-butyl)-2-thienyl group, a 5-(n-pentyl)-2-thienyl group, a 5-(n-hexyl)-2-thienyl group, a 5-(n-octyl)-2-thienyl group, a 5-(2-ethylhexyl)-2-thienyl group, etc.

R⁴⁶ to R⁶¹ are preferably one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an aryl group having 4 to 26 carbon atoms, or the group represented by Formula (2) from the viewpoint of stability.

From the viewpoint of high carrier mobility, R⁴⁶ and R⁴⁷ are preferably one of the group consisting of a group represented by Formula (2), a hydrogen atom, and a fluorine atom and more preferably one of the group consisting of a group represented by Formula (2) and a hydrogen atom. Further preferably, both of R⁴⁶ and R⁴⁷ are a group represented by Formula (2) .

R⁴⁸ to R⁶¹ are preferably selected from one of the group consisting of a hydrogen atom and a methyl group and further preferably a hydrogen atom.

In Formulae (8-5) and (8-6), o denotes 0 or 1, with 1 being preferred from the viewpoint of high carrier mobility.

Specific examples of the compound of the present invention may include the following compounds.

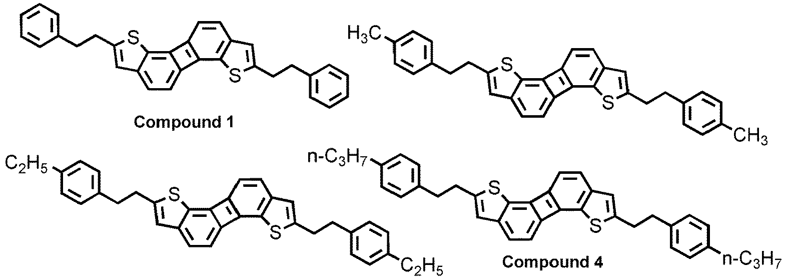

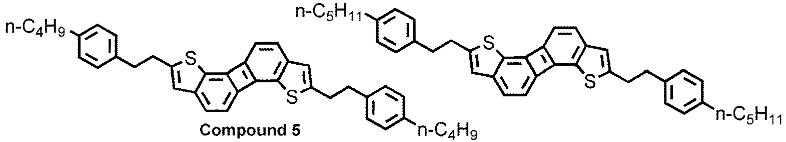

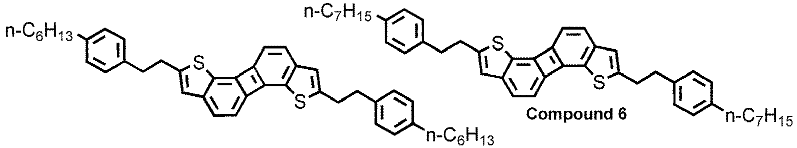

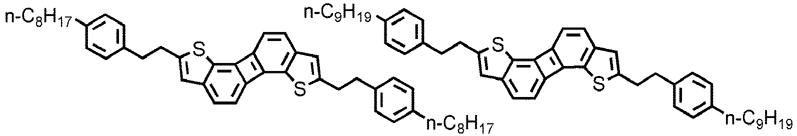

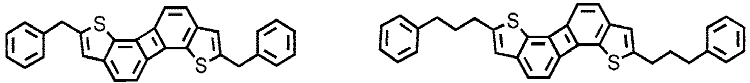

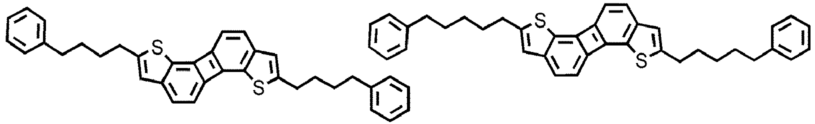

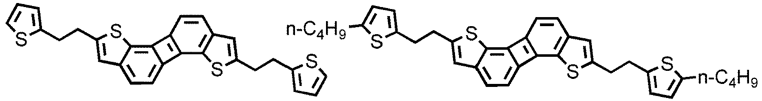

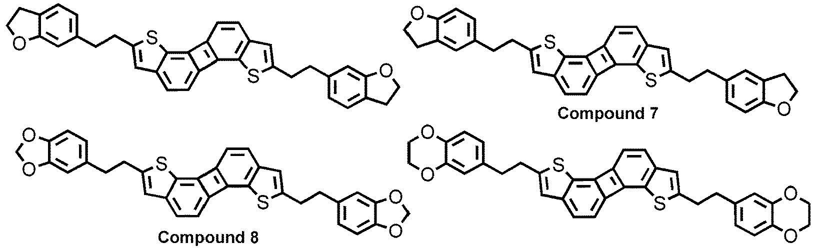

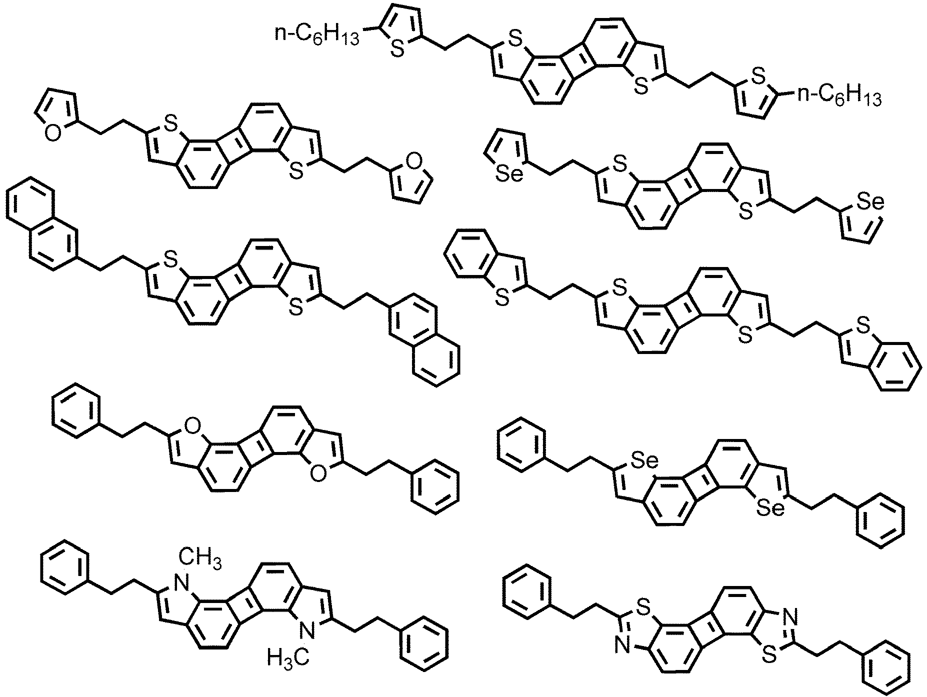

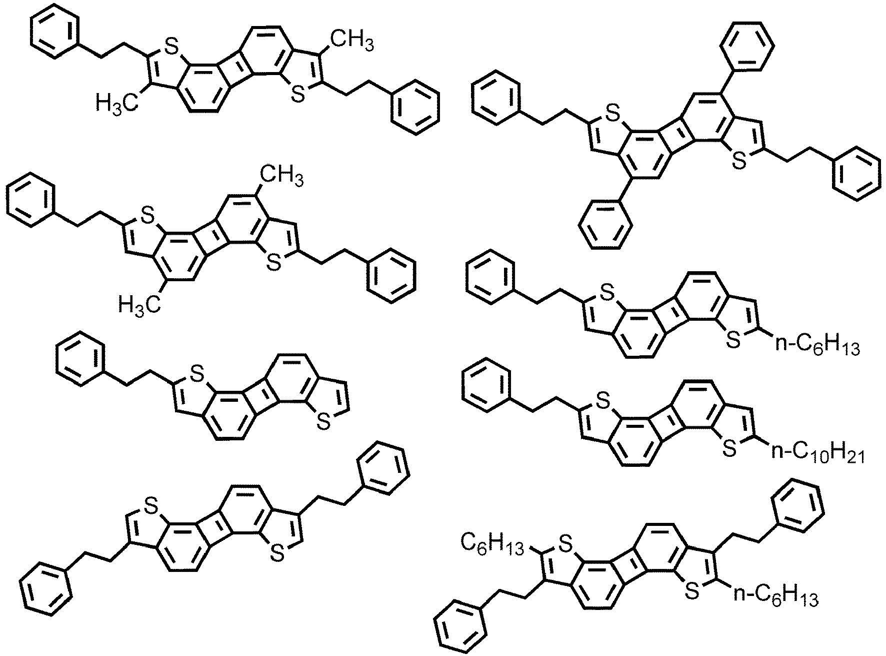

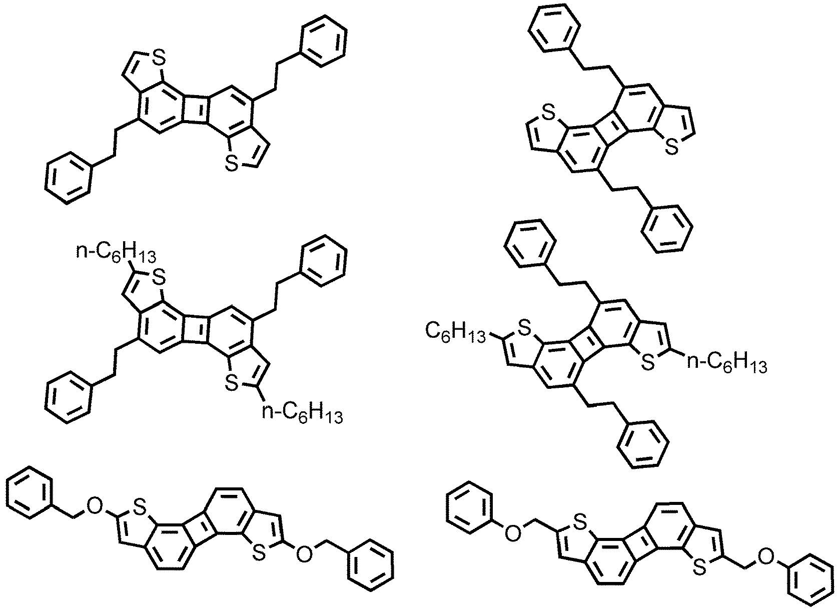

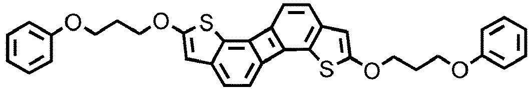

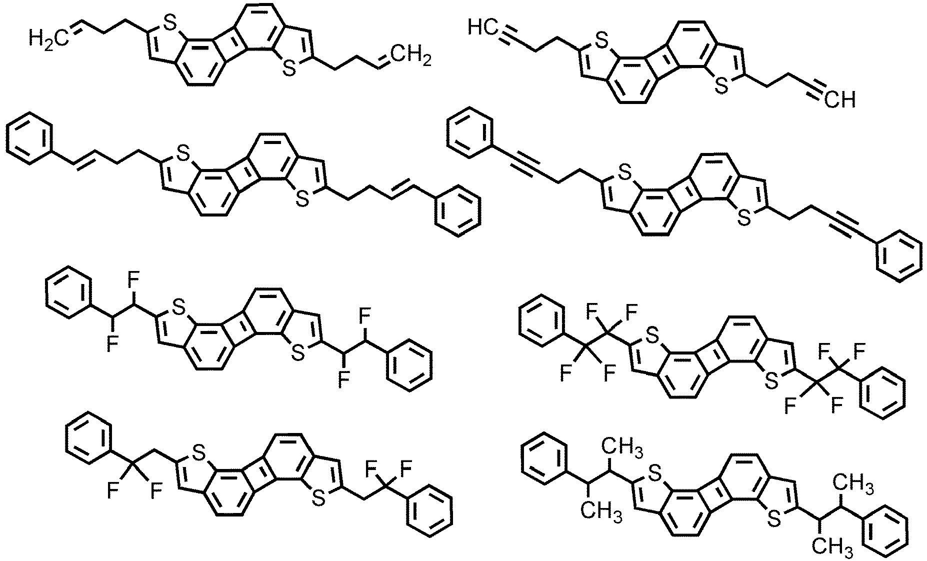

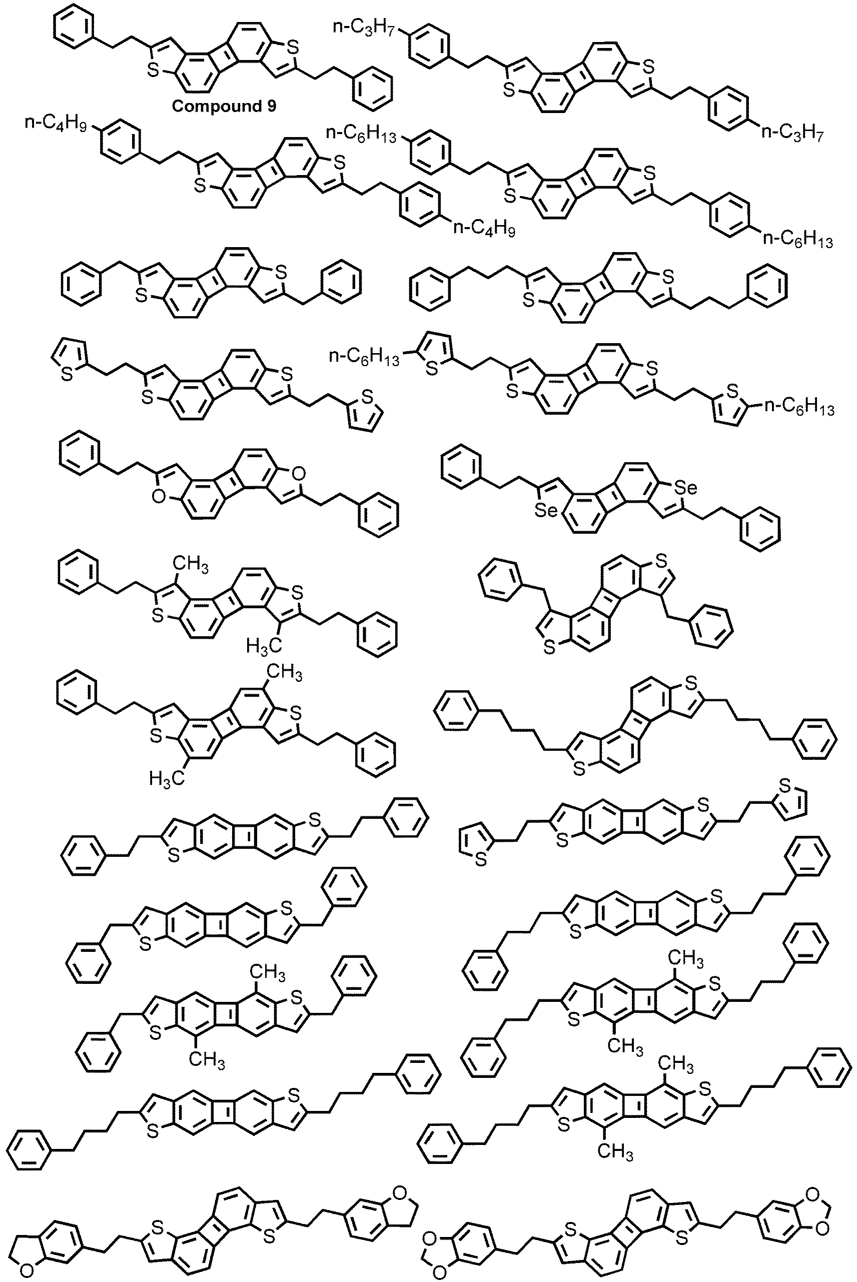

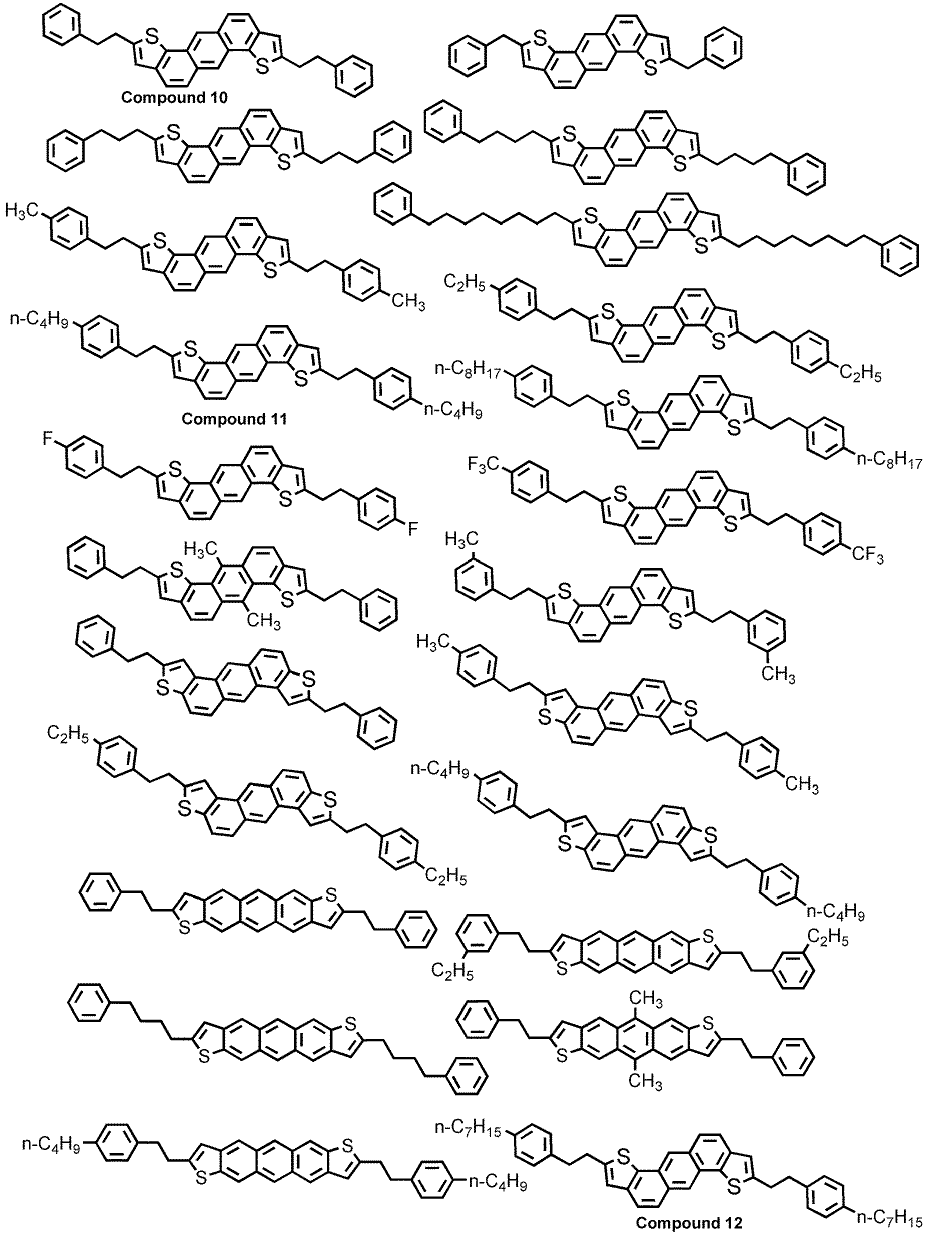

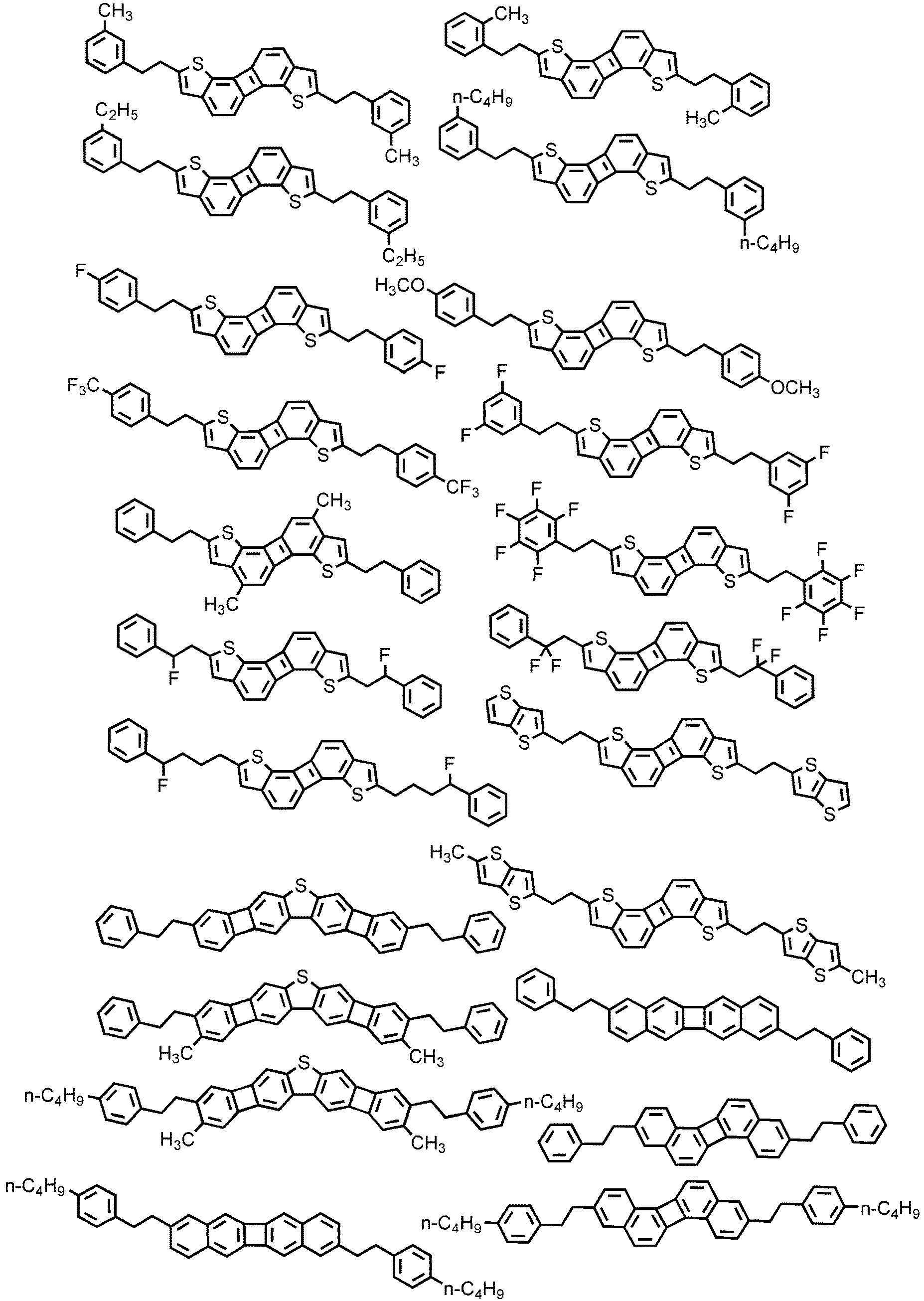

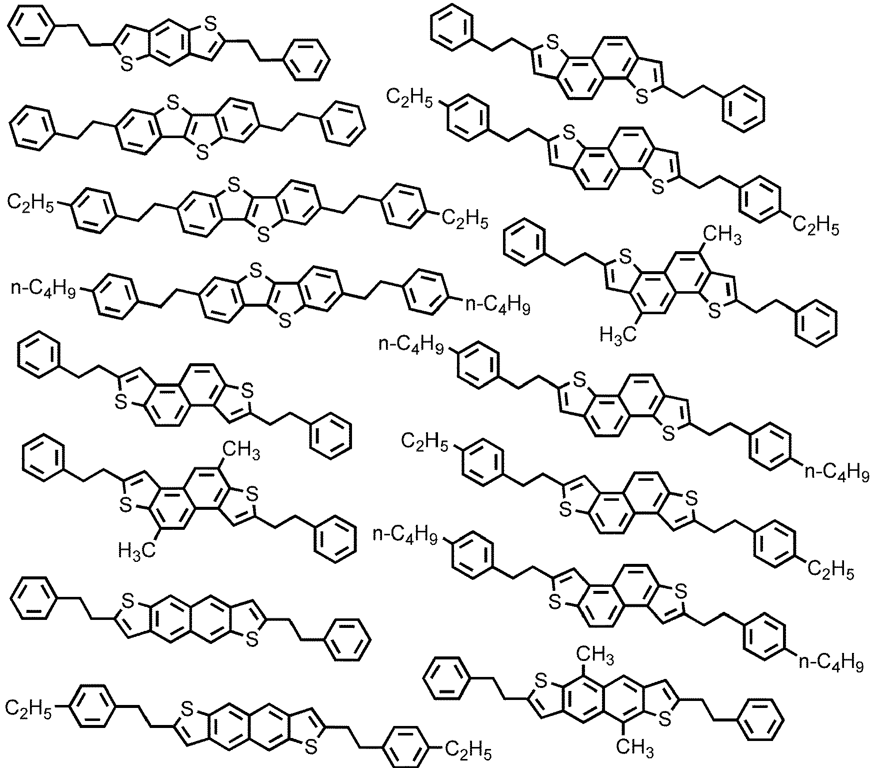

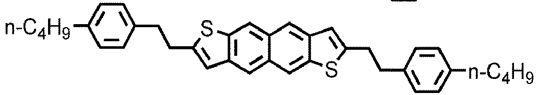

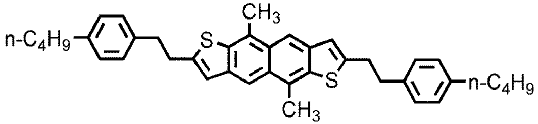

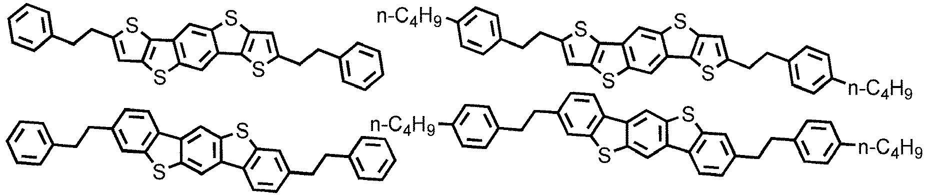

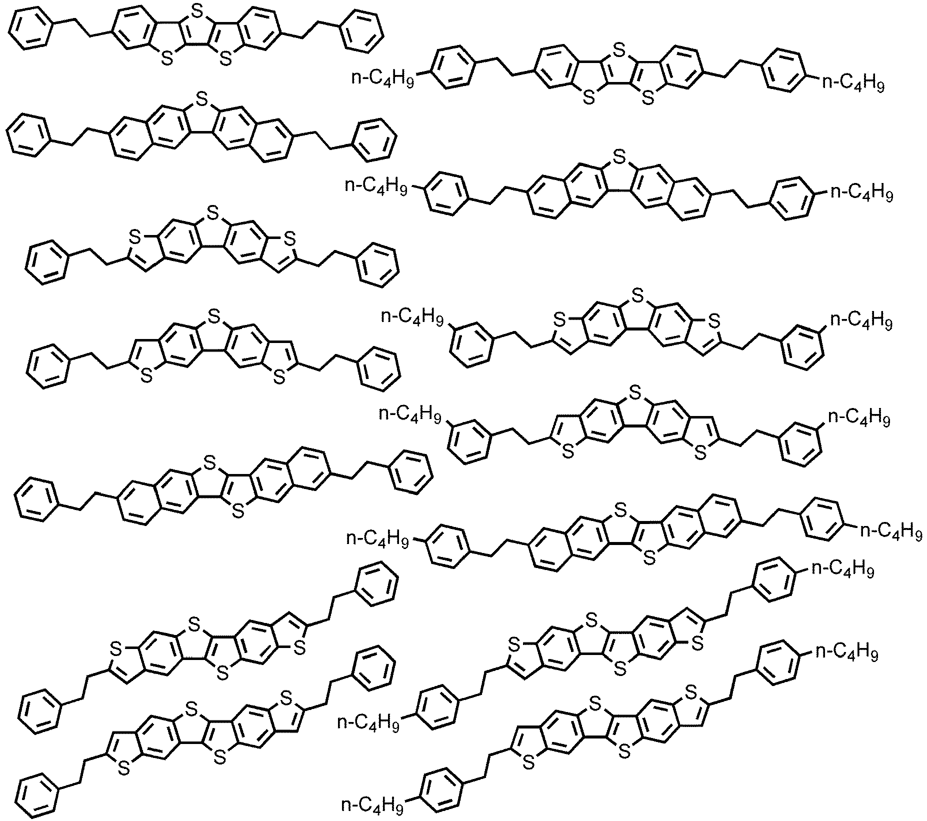

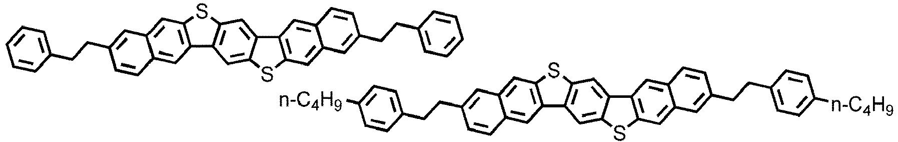

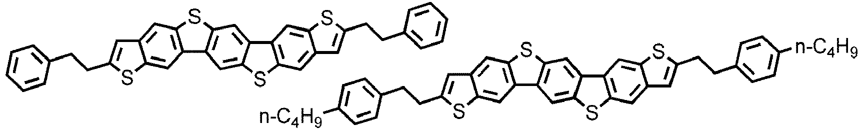

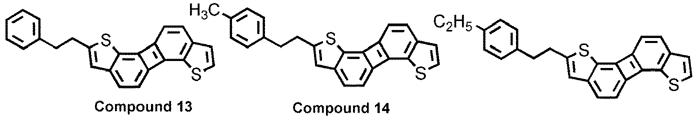

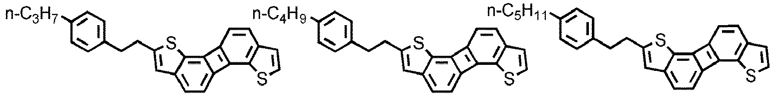

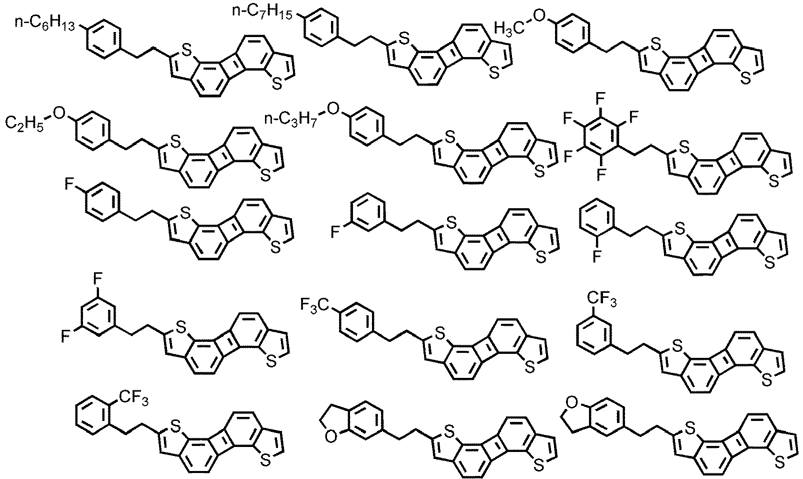

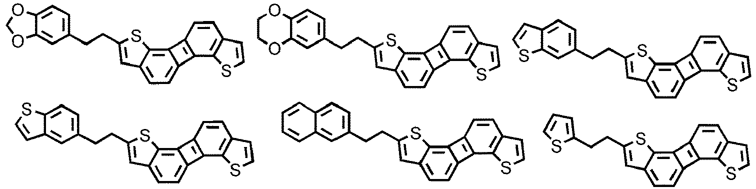

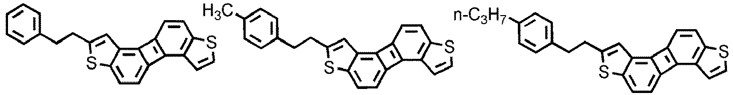

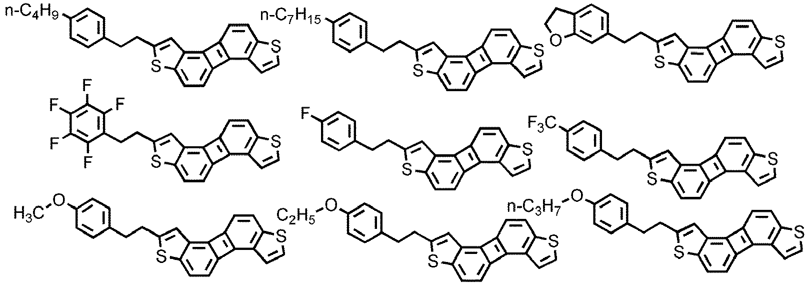

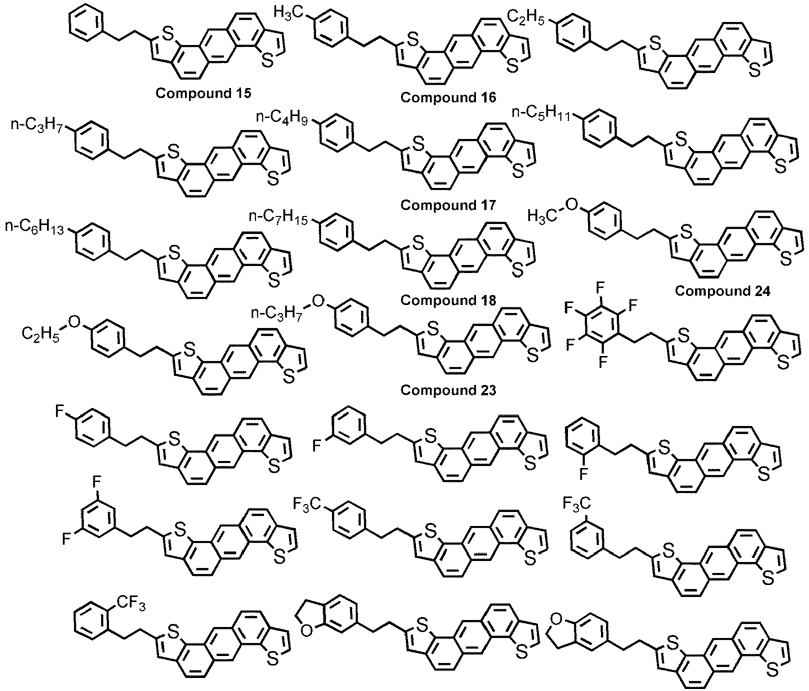

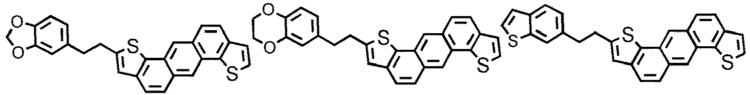

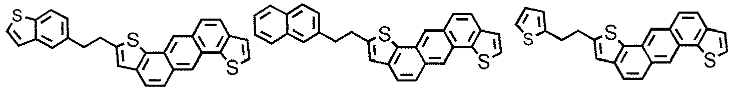

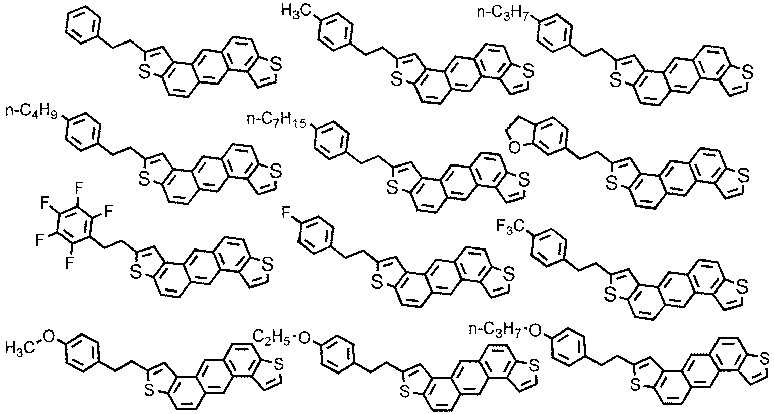

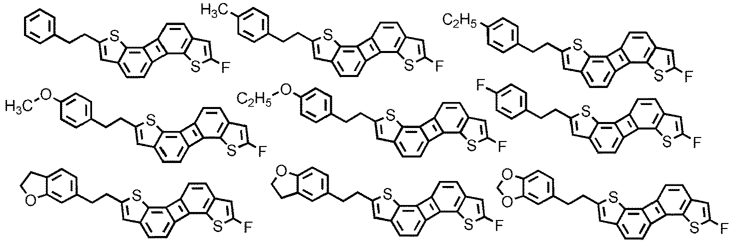

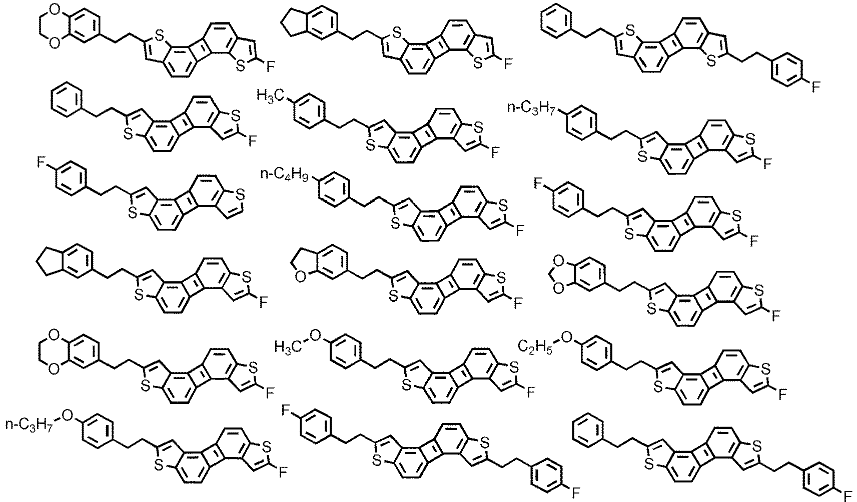

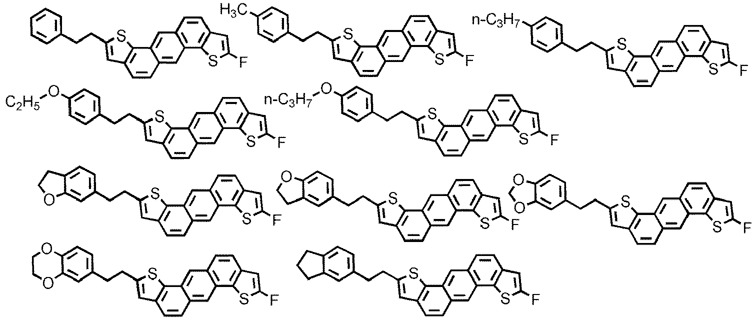

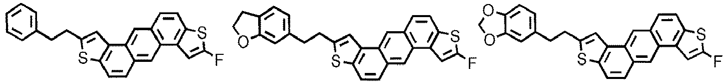

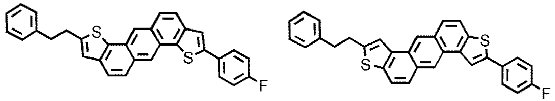

A method for producing the compound of the present invention may be any method as long as the compound can be produced.

As for the method for producing the compound of the present invention, for example, an aromatic compound (7-1a) in which, in Formula (7-1), X⁸ and X⁹ are a sulfur atom; Y⁵ and Y⁶ are CH; R³⁸ and R⁴¹ are a group represented by Formula (2); R³⁹, R⁴⁰, R⁴², and R⁴³ are a hydrogen atom; and, in Formula (2), each of 1 and n is 0 and Z¹ and Z² are a hydrogen atom may be produced by a method including Steps A1 to C1 as described below.

(Step A1); Method for synthesizing a dibromodithienobiphenylene derivative (Compound 3) by converting dithienobiphenylene derivative (Compound 2) into dilithium salt with butyl lithium and reacting with brominating agent.

(Step B1); Method for producing alkylmagnesium bromide by reacting alkyl bromide derivative with magnesium.

(Step C1); Method for producing aromatic compound (7-1a) by reacting alkylzinc chloride derived from alkylmagnesium bromide obtained in Step B1 with dibromodithienobiphenylene derivative (Compound 3) synthesized in Step A1 in presence of palladium catalyst.

Each step will be described in detail.

### (Step A1)

The Step A1 is a method for producing a dibromo form of a dithienobiphenylene derivative by converting the dithienobiphenylene derivative into a dilithium salt with 2 equivalents or more of butyl lithium and reacting it with a brominating agent.

For example, the dilithium salt may be prepared using 2 to 3 equivalents of n-butyl lithium or tert-butyl lithium in a solvent such as THF or diethyl ether at a temperature ranging from -80°C to 20°C.

As the brominating agent, tetrabromomethane, 1,2-dibromotetrachloroethane, N-bromosuccinimide (hereinafter abbreviated as "NBS") may be used.

### (Step B1)

The Step B1 is a method for producing alkylmagnesium bromide by reacting an alkyl bromide derivative with magnesium.

For example, the magnesium salt may be prepared using 1 to 2 equivalents of magnesium in a solvent such as THF or diethyl ether at a temperature ranging from 25°C to 60°C.

Examples of the alkyl bromide derivative in Step B1 include benzyl bromide, 2-phenylethyl bromide, 3-phenylpropyl bromide, 4-phenylbutyl bromide, 4-methylphenetyl bromide, 1-(2-bromoethyl)-4-ethylbenzene, 1-(2-bromoethyl)-4-propylbenzene, 1-(2-bromoethyl)-4-butylbenzene, 1-(2-bromoethyl)-4-pentylbenzene, 1-(2-bromoethyl)-4-hexylbenzene, 1-(2-bromoethyl)-4-heptylbenzene, 1-(2-bromoethyl)-4-octylbenzene, 5-(2-bromoethyl)-2,3-dihydrobenzofuran, etc.

### (Step C1)

The Step C1 is a method for producing an aromatic compound (7-1a) by reacting alkylzinc chloride derived from the alkylmagnesium bromide obtained in Step B1 with the dibromodithienobiphenylene derivative (Compound 3) synthesized in Step A1 in the presence of a palladium catalyst.

For example, the alkylzinc chloride may be prepared using zinc chloride in a solvent such as THF or diethyl ether at a temperature ranging from 0°C to 25°C.

In Step C1, the palladium catalyst may be [1,1'-bis(diphenylphosphino)ferrocene]dichloropalladium (II), tetrakis(triphenylphosphine)palladium, dichlorobis(triphenylphosphine)palladium, etc. and the reaction temperature may range from 20°C to 60°C.

The method, which is preferred due to fewer reaction steps, will be more specifically shown in the following reaction scheme: 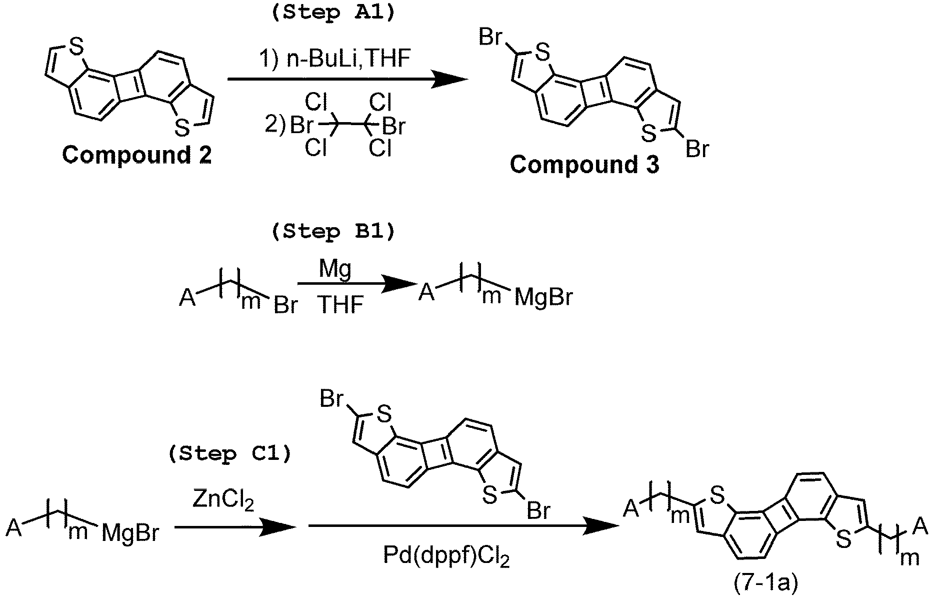 wherein A and m have the same meaning as for A and m in Formula (2).

Furthermore, an aromatic compound (7-2a) in which, in Formula (7-2), X⁸ and X⁹ are a sulfur atom; Y⁵ and Y⁶ are CH; R³⁸ and R⁴¹ are a group represented by Formula (2); R³⁹, R⁴⁰, R⁴², and R⁴³ are a hydrogen atom; and, in Formula (2), each of 1 and n is 0 and Z¹ and Z² are a hydrogen atom may be produced by a method including Steps D1 to G1 as described below.

(Step D1); Step of producing 1,5-bis(trimethylsilylethynyl)-2,6-difluorobiphenylene through Sonogashira coupling of 1,5-difluoro-2,6-diiodohalobiphenylene and trimethylsilylacetylene in presence of palladium/copper catalyst.

(Step E1); Step of producing biphenyleno[2,1-b:6,5-b']dithiophene by reacting 1,5-bis(trimethylsilylethynyl)-2,6-difluorobiphenylene obtained in Step D1 with sodium sulfide.

(Step F1); Step of producing 2,7-dibromobiphenyleno[2,1-b:6,5-b']dithiophene by converting biphenyleno[2,1-b:6,5-b']dithiophene obtained in Step E1 into dilithium salt with butyl lithium and reacting with brominating agent.

(Step G1); Step of producing aromatic compound (7-2a) by reacting alkylzinc chloride derived from alkylmagnesium bromide obtained in Step B1 mentioned above with 2,7-dibromobiphenyleno[2,1-b:6,5-b']dithiophene synthesized in Step F1 in presence of palladium catalyst.

Each step will be described in detail.

### (Step D1)

The Step D1 is a step of producing 1,5-bis(trimethylsilylethynyl)-2,6-difluorobiphenylene through Sonogashira coupling of 2,6-difluoro-1,5-diiodohalobiphenylene and trimethylsilylacetylene in the presence of a palladium catalyst and a copper catalyst.

The palladium catalyst in this step may be, for example, tetrakis(triphenylphosphine)palladium, dichlorobis(triphenylphosphine)palladium, etc. and the copper catalyst may be, for example, copper iodide (I), copper bromide (I), copper chloride (I), etc.

Furthermore, the Sonogashira coupling may take place in a solvent such as triethylamine, diisopropylamine, diisopropylethylamine, piperidine, pyridine, etc. at a temperature ranging from 20°C to 80°C.

Note that, toluene, THF, or the like may be added as the solvent.

### (Step E1)

The Step E1 is a step of producing biphenyleno[2,1-b:6,5-b']dithiophene by reacting 1,5-bis(trimethylsilylethynyl)-2,6-difluorobiphenylene obtained in Step D1 with sodium sulfide. For example, the reaction may be performed in a solvent such as dimethyl sulfoxide (hereinafter abbreviated as DMSO), N,N-dimethylformamide (hereinafter abbreviated as DMF), and N-methylpyrrolidone (hereinafter abbreviated as NMP) at a temperature ranging from 20 to 200°C.

Note that, the step may also be performed using a known reaction condition for synthesizing a benzothiophene ring from 2-haloalkynylbenzene (e.g., Organic Letters, 2009, vol. 11, pp. 2473-2475).

### (Step F1)

The Step F1 is a method for producing a dibromo form of biphenyleno[2,1-b:6,5-b']dithiophene obtained in Step E1 into a dilithium salt with 2 equivalents or more of butyl lithium and reacting it with a brominating agent.

For example, the dilithium salt may be prepared using 2 to 3 equivalents of n-butyl lithium or tert-butyl lithium in a solvent such as THF or diethyl ether at a temperature ranging from -80°C to 20°C.

As the brominating agent, tetrabromomethane, 1,2-dibromotetrachloroethane, NBS, etc. may be used.

### (Step G1)

The Step G1 is a method for producing an aromatic compound (7-2a) by reacting alkylzinc chloride derived from alkylmagnesium bromide obtained in Step B1 with 2,7-dibromobiphenyleno[2,1-b:6,5-b']dithiophene synthesized in Step F1 in the presence of a palladium catalyst.

The alkylzinc chloride may be prepared using zinc chloride in a solvent such as THF or diethyl ether at a temperature ranging from 0°C to 25°C.

In Step G1, the palladium catalyst may be [1,1'-bis(diphenylphosphino)ferrocene]dichloropalladium (II), tetrakis(triphenylphosphine)palladium, dichlorobis(triphenylphosphine)palladium, etc. and the reaction temperature may range from 20°C to 60°C.

The method, which is preferred due to fewer reaction steps, will be more specifically shown in the following reaction scheme: 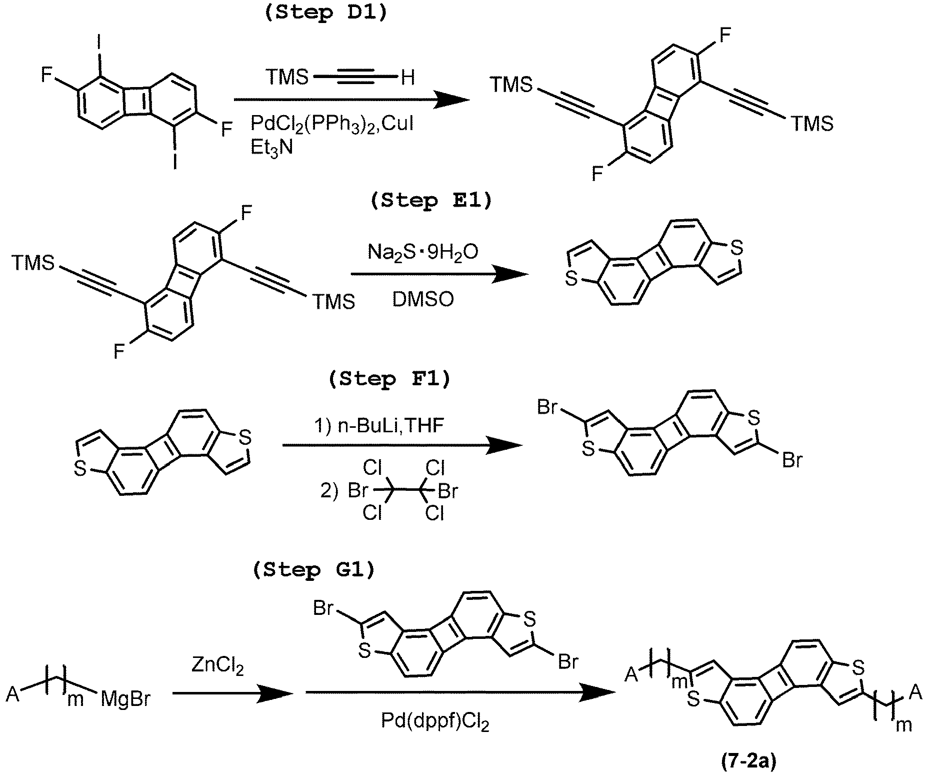 wherein A and m have the same meaning as for A and m in Formula (2).

An aromatic compound (7-5a) in which, in Formula (7-5), X⁸ and X⁹ are a sulfur atom; Y⁵ and Y⁶ are CH; R³⁸ and R⁴¹ are the group represented by Formula (2); R³⁹, R⁴⁰, R⁴², R⁴³, R⁷¹, and R⁷² are a hydrogen atom; and, in Formula (2), each of 1 and n is 0 and Z¹ and Z² are a hydrogen atom, may be produced by a method including Steps A2 below, B1 above, and C2 below.

(Step A2); Method for synthesizing a dibromoanthradithiophene derivative (Compound 20) by converting an anthradithiophene derivative (Compound 19) into a dilithium salt with butyl lithium and reacting it with a brominating agent.

(Step C2); Method for producing aromatic compound (7-5a) by reacting alkylzinc bromide derived from alkylmagnesium bromide obtained in Step B1 with dibromoanthradithiophene derivative (Compound 20) synthesized in Step A2 in the presence of palladium catalyst.

Each step will be described in detail.

### (Step A2)

The Step A2 is a method for producing a dibromo form of an anthradithiophene derivative into a dilithium salt with 2 equivalents or more of butyl lithium and reacting it with a brominating agent.

For example, the dilithium salt may be prepared using 2 to 3 equivalents of n-butyl lithium or tert-butyl lithium in a solvent such as THF or diethyl ether at a temperature ranging from -80°C to 20°C.

As the brominating agent, tetrabromomethane, 1,2-dibromotetrachloroethane, etc. may be used.

### (Step C2)

The Step C2 is a method for producing an aromatic compound (7-5a) by reacting alkylzinc chloride derived from alkylmagnesium bromide obtained in Step B1 with the dibromoanthradithiophene derivative (Compound 20) synthesized in Step A2 in the presence of a palladium catalyst.

For example, the alkylzinc bromide may be prepared using zinc chloride in a solvent such as THF or diethyl ether at a temperature ranging from 0°C to 25°C.

In Step C2, the palladium catalyst may be [1,1'-bis(diphenylphosphino)ferrocene]dichloropalladium (II), tetrakis(triphenylphosphine)palladium, dichlorobis(triphenylphosphine)palladium, etc. and the reaction temperature may range from 20°C to 60°C.

The method, which is preferred due to fewer reaction steps, will be more specifically shown in the following reaction scheme: 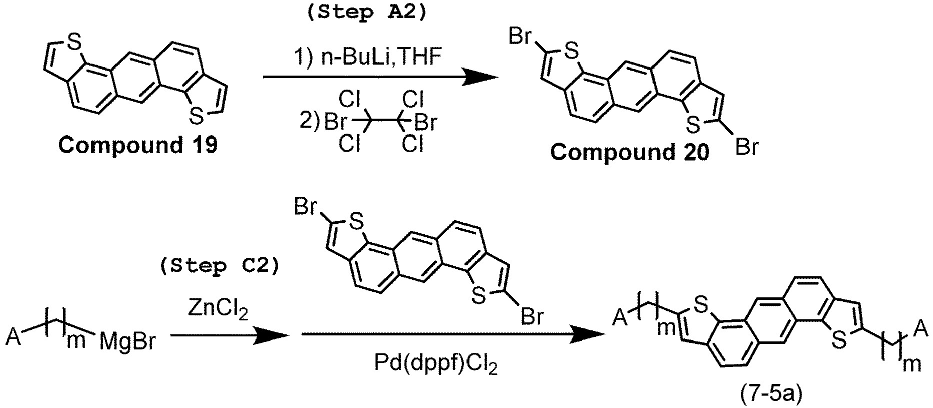 wherein A and m have the same meaning as for A and m in Formula (2).

An aromatic compound (7-1b) in which, in Formula (7-1), X⁸ and X⁹ are a sulfur atom; Y⁵ and Y⁶ are CH; R³⁸ is a group represented by Formula (2); R³⁹ to R⁴³ are a hydrogen atom; and, in Formula (2), each of 1 and n is 0 and Z¹ and Z² are a hydrogen atom, may be produced by a method including Steps A3 below, B1 above, and C3 below.

(Step A3); Method for synthesizing a monobromodithienobiphenylene derivative (Compound 21) by converting a dithienobiphenylene derivative (Compound 2) into a monolithium salt with butyl lithium and reacting it with a brominating agent.

(Step C3); Method for producing aromatic compound (7-1b) by reacting alkylzinc bromide derived from alkylmagnesium bromide obtained in Step B1 with monobromodithienobiphenylene derivative (Compound 21) synthesized in Step A3 in presence of palladium catalyst.

Each step will be described in detail.

### (Step A3)

The Step A3 is a method for producing a monobromo form of a dithienobiphenylene derivative into a monolithium salt with 1.0 equivalent of butyl lithium and reacting it with a brominating agent.

For example, the monolithium salt may be prepared using 0.5 to 1.5 equivalents of n-butyl lithium or tert-butyl lithium in a solvent such as THF or diethyl ether at a temperature ranging from -80°C to 20°C.

As the brominating agent, tetrabromomethane, 1,2-dibromotetrachloroethane, etc. may be used.

### (Step C3)

The Step C3 is a method for producing an aromatic compound (7-1b) by reacting alkylzinc chloride derived from the alkylmagnesium bromide obtained in Step B1 with the monobromodithienobiphenylene derivative (Compound 21) synthesized in Step A3 in the presence of a palladium catalyst.

For example, the alkylzinc bromide may be prepared using zinc chloride in a solvent such as THF or diethyl ether at a temperature ranging from 0°C to 25°C.

In Step C3, the palladium catalyst may be [1,1'-bis(diphenylphosphino)ferrocene]dichloropalladium (II), tetrakis(triphenylphosphine)palladium, dichlorobis(triphenylphosphine)palladium, etc. and a reaction temperature may range from 20°C to 60°C.

The method, which is preferred due to fewer reaction steps, will be more specifically shown in the following reaction scheme: 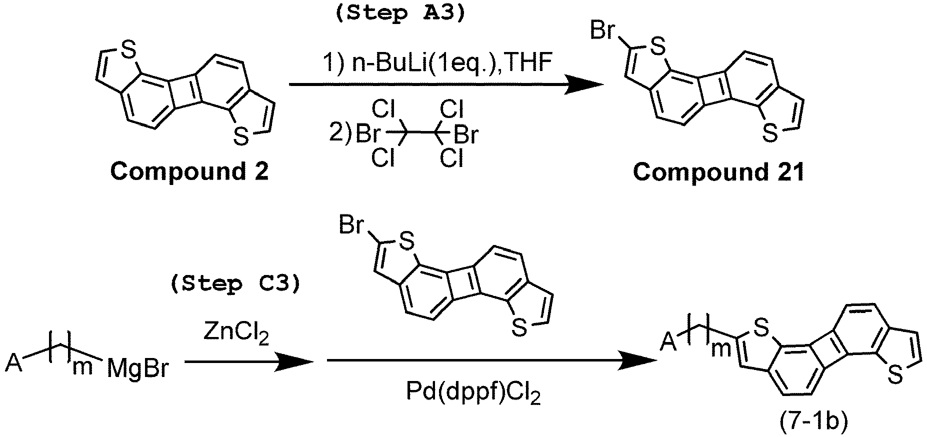 wherein A and m have the same meaning as for A and m in Formula (2).

An aromatic compound (7-5b) in which, in Formula (7-5), X⁸ and X⁹ are a sulfur atom; Y⁵ and Y⁶ are CH; R³⁸ is a group represented by Formula (2); R³⁹ to R⁴¹, R⁴², R⁴³, R⁷¹, and R⁷² are a hydrogen atom; and, in Formula (2), each of 1 and n is 0 and Z¹ and Z² are a hydrogen atom, may be isolated as a by-product in Step C2 above or produced by a method including Steps A4 below, B1 above, and C4 below.

(Step A4); Method for synthesizing an monobromoanthradithiophene derivative (Compound 22) by converting an anthradithiophene derivative (Compound 19) into a monolithium salt with butyl lithium and reacting it with a brominating agent.

(Step C4); Method for producing aromatic compound (7-5b) by reacting alkylzinc bromide derived from alkylmagnesium bromide obtained in Step B1 with monobromoanthradithiophene derivative (Compound 22) synthesized in Step A4 in presence of palladium catalyst.

Each step will be described in detail.

### (Step A4)

The Step A4 is a method for producing a monobromo form of an anthradithiophene derivative into a monolithium salt with 1 equivalent of butyl lithium and reacting it with a brominating agent.

For example, the monolithium salt may be prepared using 0.5 to 1.5 equivalents of n-butyl lithium or tert-butyl lithium in a solvent such as THF or diethyl ether at a temperature ranging from -80°C to 20°C.

As the brominating agent, tetrabromomethane, 1,2-dibromotetrachloroethane, etc. may be used.

### (Step C4)

The Step C4 is a method for producing an aromatic compound (7-5b) by reacting alkylzinc chloride derived from alkylmagnesium bromide obtained in Step B1 with the monobromoanthradithiophene derivative (Compound 22) synthesized in Step A4 in the presence of a palladium catalyst.

For example, the alkylzinc bromide may be prepared using zinc chloride in a solvent such as THF or diethyl ether at a temperature ranging from 0°C to 25°C.

In Step C4, the palladium catalyst may be [1,1'-bis(diphenylphosphino)ferrocene]dichloropalladium (II), tetrakis(triphenylphosphine)palladium, dichlorobis(triphenylphosphine)palladium, etc. and the reaction temperature may range from 20°C to 60°C.

The method, which is preferred due to fewer reaction steps, will be more specifically shown in the following reaction scheme: 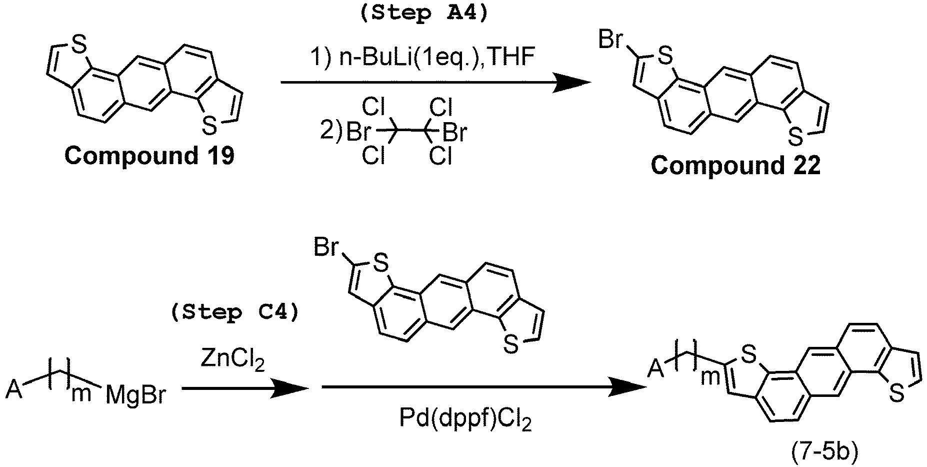 wherein A and m have the same meaning as for A and m in Formula (2).

The compound of the present invention may be dissolved in an appropriate solvent to thereby form a solution for forming an organic semiconductor layer containing the compound.

The solvent may be any solvent as long as the aromatic compound represented by Formula (1-I) or Formula (1-II) can be dissolved therein and is preferably an organic solvent having a boiling point at normal pressure of 100°C or more, which enables a suitable drying rate of the solvent upon formation of the organic semiconductor layer.

The solvent which may be used in the present invention is not particularly limited and examples thereof may include aromatic hydrocarbons such as toluene, mesitylene, o-xylene, isopropylbenzene, pentylbenzene, cyclohexylbenzene, 1,2,4-trimethylbenzene, tetralin, and indane; aromatic ethers such as anisole, 2-methyl anisole, 3-methyl anisole, 2,3-dimethyl anisole, 3,4-dimethyl anisole, 2,6-dimethyl anisole, ethyl phenyl ether, butyl phenyl ether, 1,2-methylenedioxybenzene, and 1,2-ethylenedioxybenzene; aromatic halogen compounds such as chlorobenzene, 1,2-dichlorobenzene, 1,3-dichlorobenzene, 1,4-dichlorobenzene, 1,2-difluorobenzene, 1,3-difluorobenzene, and 1,4-difluorobenzene; heteroaromatics such as thiophene, 3-chlorothiophene, 2-chlorothiophene, 3-methylthiophene, 2-methylthiophene, benzothiophene, 2-methylbenzothiophene, 2,3-dihydrobenzothiophene, furan, 3-methylfuran, 2-methylfuran, 2,5-dimethylfuran, benzofuran, 2-methylbenzofuran, 2,3-dihydrobenzofuran, thiazole, oxazole, benzothiazole, benzooxazole, and pyridine; saturated hydrocarbons such as hexane, cyclohexane, heptane, octane, nonane, decane, undecane, dodecane, and decalin; glycols such as dipropylene glycol dimethylether, dipropylene glycol diacetate, dipropylene glycol methyl-n-propyl ether, dipropylene glycol methyl ether acetate, 1,4-butanediol diacetate, 1,3-butylene glycol diacetate, 1,3-butylene glycol diacetate, 1,6-hexanediol diacetate, ethylene glycol monomethylether acetate, propylene glycol monomethyl ether acetate, ethylene glycol monobutyl ether acetate, diethylene glycol monoethyl ether acetate, and diethylene glycol monobutyl ether acetate; esters such as dimethyl phthalate, diethyl phthalate, dimethyl terephthalate, phenyl acetate, cyclohexanol acetate, 3-methoxy butyl acetate, tetrahydrofurfuryl acetate, tetrahydrofurfuryl propionate, and γ-butyrolactone; and cyclic ethers such as THF and 2-methoxymethyl tetrahydrofuran. Among them, due to an appropriate drying rate, the solvent is preferably toluene, o-xylene, mesitylene, 1,2,4-trimethylbenzene, tetralin, indane, octane, nonane, decane, anisole, 2-methyl anisole, 3-methyl anisole, 2,3-dimethyl anisole, 3,4-dimethyl anisole, 2,6-dimethyl anisole, ethyl phenyl ether, butyl phenyl ether, 1,2-methylenedioxybenzene, 1,2-ethylenedioxybenzene, chlorobenzene, 1,2-dichlorobenzene, 1,3-dichlorobenzene, 1,4-dichlorobenzene, 3-methylthiophene, or benzothiazole and further preferably toluene, o-xylene, mesitylene, tetralin, indane, octane, nonane, decane, anisole, 2-methyl anisole, 3-methyl anisole, 2,3-dimethyl anisole, 3,4-dimethyl anisole, or 2,6-dimethyl anisole.

Note that, as for the solvent for use in the present invention, one type of solvent may be used alone or two or more types of solvents differing in properties such as a boiling point, polarity, or solubility parameter may be mixed.

A temperature at which the aromatic compound represented by Formula (1-I) or Formula (I-II) is mixed and dissolved into the solvent is preferably a temperature ranging from 0 to 80°C and further preferably a temperature ranging from 10 to 60°C for the purpose of promoting dissolution.

Furthermore, a period of time for which the aromatic compound represented by Formula (1-I) or Formula (I-II) is mixed and dissolved in the organic solvent is preferably 1 min to 1 hour in order to obtain a homogeneous solution.

In the present invention, a solution for forming an organic semiconductor layer of the present invention containing the aromatic compound represented by Formula (1-I) or Formula (I-II) at a concentration of 0.1 to 10.0% by weight is easily handled and more efficiently forms an organic semiconductor layer.

Furthermore, the solution for forming an organic semiconductor layer having a viscosity of 0.3 to 10 mPa·s exhibits more suitable coatability.

Note that, the solution can be prepared at a relatively low temperature since the aromatic compound itself has a proper aggregability and can be suitably applied to production of an organic thin film by a coating method since the solution has oxidation resistance.

In other words, a step of coating can be simplified since there is no need to remove air from the atmosphere. Additionally, the solution may include polystyrene, poly(α-methylstyrene), poly(4-methylstyrene), poly(1-vinylnaphthalene), poly(2-vinylnaphthalene), poly(styrene-block-butadiene-block-styrene), poly(styrene-block-isoprene-block-styrene), poly(vinyltoluene), poly(styrene-co-2,4-dimethylstyrene), poly(chlorostyrene), poly(styrene-co-α-methylstyrene), poly(styrene-co-butadiene), poly(ethylene-co-norbornene), polyphenylene ether, polycarbonate, polycarbazole, polytriarylamine, poly(9,9-dioctylfluorene-co-dimethyltriarylamine), poly(N-vinylcarbazole), polymethyl methacrylate, poly(styrene-co-methyl methacrylate), polyethyl methacrylate, polyn-propyl methacrylate, polyisopropyl methacrylate, polyn-butyl methacrylate, polyphenyl methacrylate, polymethyl acrylate, polyethyl acrylate, polyn-propyl acrylate, etc. Preferably, a polymer such as polystyrene, poly(α-methylstyrene), poly(ethylene-co-norbornene), polymethyl methacrylate may be included as a binder.

The polymer binder is preferably included at a concentration of 0.001 to 10.0% by weight in order to provide a proper viscosity to the solution.

The polymer binder preferably has a glass transition temperature (Tg) of 105°C or more, further preferably 120°C or more, and particularly preferably 150°C or more due to suitability for a process temperature upon fabrication of an electronic device.

Furthermore, the polymer preferably has a molecular weight of 5,000 to 1,000,000, further preferably 10,000 to 500,000, and particularly preferably 20,000 to 100,000 due to suitability for obtaining an organic thin film transistor having higher carrier mobility.

Note that, in the present invention, the molecular weight of the polymer refers to a weight average molecular weight (Mw) in terms of polystyrene.

The polymer has an effect as a common polymer binder, that is, improves a film-forming property of the resulting organic semiconductor layer. The polymer may be an insulating polymer or a semiconducting polymer.

Specific examples of a polymer that may be used as the polymer binder in the present invention include, in addition to the above-mentioned polymers, polar cyclic polyolefins, polysulfones, acrylonitrile-styrene copolymers, and methyl methacrylate-styrene copolymers.

More specifically, the polar cyclic polyolefins are further preferably a polymer represented by Formula (9) below: 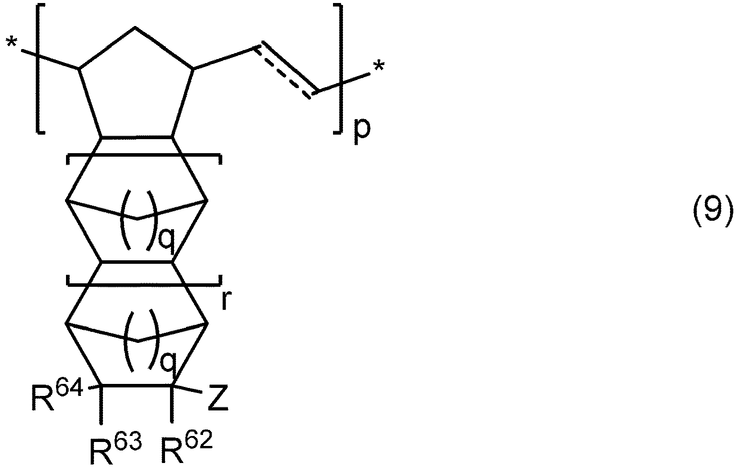
wherein R⁶² to R⁶⁴ each independently denote one of the group consisting of a hydrogen atom, a halogen atom, an alkyl group having 1 to 20 carbon atoms, an aryl group having 6 to 20 carbon atoms, an alkyloxycarbonyl group having 2 to 20 carbon atoms, an aryloxycarbonyl group having 7 to 20 carbon atoms, a cyano group, a nitro group, an alkoxy group having 1 to 20 carbon atoms, an aryloxy group having 6 to 20 carbon atoms, a hydroxyl group, an amino group, or an alkylamino group having 1 to 20 carbon atoms;
Z denotes one of the group consisting of a halogen atom, an alkyloxycarbonyl group having 2 to 20 carbon atoms, an aryloxycarbonyl group having 7 to 20 carbon atoms, a cyano group, a nitro group, an alkoxy group having 1 to 20 carbon atoms, an aryloxy group having 6 to 20 carbon atoms, a hydroxyl group, an amino group, or an alkylamino group having 1 to 20 carbon atoms;
p denotes an integer of 20 to 5,000; q and r each independently denote an integer of 0 to 2; and a bond represented by a solid line and a dotted line denotes a single bond or a double bond.

In Formula (9), R⁶² to R⁶⁴ each independently denote one of the group consisting of a hydrogen atom, a halogen atom, an alkyl group having 1 to 20 carbon atoms, an aryl group having 6 to 20 carbon atoms, an alkyloxycarbonyl group having 2 to 20 carbon atoms, an aryloxycarbonyl group having 7 to 20 carbon atoms, a cyano group, a nitro group, an alkoxy group having 1 to 20 carbon atoms, an aryloxy group having 6 to 20 carbon atoms, a hydroxyl group, an amino group, or an alkylamino group having 1 to 20 carbon atoms, with a hydrogen atom or an alkyl group having 1 to 20 carbon atoms being preferred from the viewpoint of high heat resistance.

The alkyl group having 1 to 20 carbon atoms in R⁶² to R⁶⁴ may be, for example, a linear or branched alkyl group such as a methyl group, an ethyl group, an n-propyl group, an n-butyl group, an isobutyl group, a sec-butyl group, an n-pentyl group, etc.

The aryl group having 6 to 20 carbon atoms may be, for example, a phenyl group, a p-tolyl group, a p-(n-hexyl)phenyl group, a p-(n-octyl)phenyl group, a p-(2-ethylhexyl)phenyl group, etc.

The alkyloxycarbonyl group having 2 to 20 carbon atoms may be, for example, a methyloxycarbonyl group, an ethyloxycarbonyl group, an n-propyloxycarbonyl group, etc.

The aryloxycarbonyl group having 7 to 20 carbon atoms may be, for example, a phenoxycarbonyl group, 4-methylphenoxycarbonyl group, etc.

The alkoxy group having 1 to 20 carbon atoms may be, for example, a methoxy group, an ethoxy group, an n-propoxy group, etc.

The aryloxy group having 6 to 20 carbon atoms may be, for example, a phenoxy group, 4-methylphenoxy, etc.

The alkylamino group having 1 to 20 carbon atoms may be, for example, a methylamino group, an ethylamino group, an n-propylamino group, etc.

Among them, it is preferable that the substituent R⁶⁰ is a methyl group, an ethyl group, or an n-propyl group and the substituents R⁶³ and R⁶⁴ are a hydrogen atom from the viewpoint of high heat resistance.

In Formula (9), Z denotes one of the group consisting of a halogen atom, an alkyloxycarbonyl group having 2 to 20 carbon atoms, an aryloxycarbonyl group having 7 to 20 carbon atoms, a cyano group, a nitro group, an alkoxy group having 1 to 20 carbon atoms, an aryloxy group having 6 to 20 carbon atoms, a hydroxyl group, an amino group, or an alkylamino group having 1 to 20 carbon atoms;

The alkyloxycarbonyl group having 2 to 20 carbon atoms in the substituent Z may be, for example, a methoxycarbonyl group, an ethoxycarbonyl group, an n-propoxycarbonyl group, an n-butoxycarbonyl group, an n-hexyloxycarbonyl group, a cyclohexyloxycarbonyl group, etc. and the aryloxycarbonyl group having 7 to 20 carbon atoms may be, for example, a phenoxycarbonyl group, a 4-methylphenoxycarbonyl group, a 2,4-dimethylphenyxycarbonyl group, a 4-ethylphenoxycarbonyl group, etc.

The alkoxy group having 1 to 20 carbon atoms may be, for example, a methoxy group, an ethoxy group, etc.

The aryloxy group having 6 to 20 carbon atoms may be, for example, a phenoxy group, 4-methylphenoxy, etc.

The alkylamino group having 1 to 20 carbon atoms may be, for example, a methylamino group, an ethylamino group, an n-propylamino group, etc.

From the viewpoint of high solubility and high heat resistance, an alkyloxycarbonyl group having 2 to 20 carbon atoms is preferred.

p denotes an integer of 20 to 5,000 and preferably 40 to 2,000 due to suitability for obtaining an organic thin film transistor having higher carrier mobility.

q denotes an integer of 0 to 2, preferably 1.

r denotes an integer of 0 to 2, preferably 0 or 1,
and further preferably 0.

A bond represented by a solid line and a dotted line denotes a single bond or a double bond and preferably a single bond from the viewpoint of thermal stability.

The polysulfones for use in the present invention as the polymer binder are not particularly limited as long as they have a polysulfone structure. More specific examples of the polysulfones include polysulfones represented by Polysulfones 1 to 5 below: 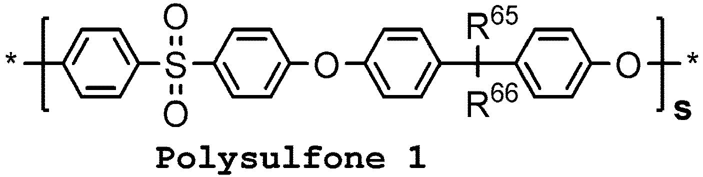 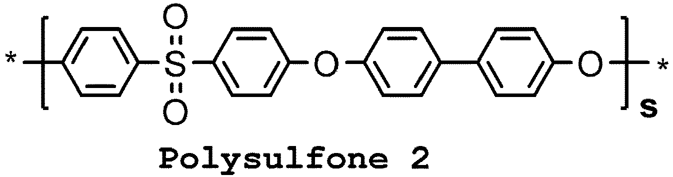 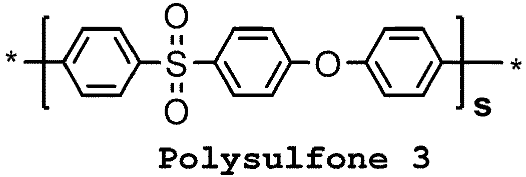 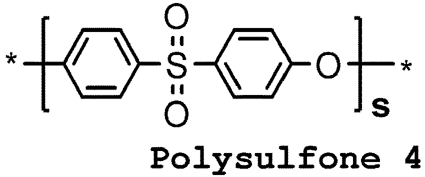 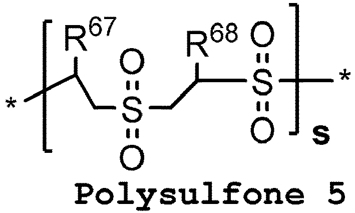 wherein substituents R⁶⁵ to R⁶⁸ each independently denotes an alkyl group having 1 to 20 carbon atoms; and s denotes an integer of 10 to 20,000.

The alkyl group having 1 to 20 carbon atoms in the substituents R⁶⁵ to R⁶⁸, may be, for example, a linear or branched alkyl group such as a methyl group, an ethyl group, an n-propyl group, an n-butyl group, an isobutyl group, an n-pentyl group, an n-hexyl group, an isohexyl group, an n-heptyl group, an n-octyl group, an n-nonyl group, an n-decyl group, an n-dodecyl group, an n-tetradecyl group, an n-octadecyl group, a 2-ethylhexyl group, a 3-ethylheptyl group, a 3-ethyldecyl group, a 2-hexyldecyl group, etc.
s denotes an integer of 10 to 20,000, preferably an integer of 10 to 10,000.

The acrylonitrile-styrene copolymer for use in the present invention as the polymer binder is a copolymer of acrylonitrile and styrene at any ratio and preferably has a weight ratio of acrylonitrile to styrene of 10:90 to 50:50 and further preferably 20:80 to 40:60 due to showing a satisfactory electric property and improved reliability, for example, a smaller change in threshold voltage upon application of bias stress.

The methyl methacrylate-styrene copolymer for use in the present invention as the polymer binder is a copolymer of methyl methacrylate and styrene at any ratio and preferably has a molar ratio of methyl methacrylate to styrene of 1:99 to 90:10 and further preferably 1:99 to 70:30 due to showing a satisfactory electric property and improved reliability, for example, a smaller change in threshold voltage upon application of bias stress

The polymer for use in the present invention as the polymer binder may be those of which surface energy is adjusted with a surface treatment agent.

The surface treatment agent may be a silane coupling agent and specific examples thereof may include 1,1,1,3,3,3-hexamethyldisilazane, phenyltrimethoxysilane, octyltrichlorosilane, β-phenetyltrichlorosilane, β-phenetyltrimethoxysilane, etc.

Note that, as for the polymer for use in the present invention one type of polymer may be used alone or a mixture of two or more types of polymers may be used.

Additionally, a mixture of polymers differing in molecular weight may also be used.

A coating method for forming an organic semiconductor layer using a solution for forming an organic semiconductor layer of the present invention is not particularly limited as long as the organic semiconductor layer can be formed. Examples thereof may include a simple coating method such as spin coating, drop casting, dip coating, and cast coating; and a printing method such as dispenser, inkjet, slit coating, blade coating, flexographic printing, screen printing, gravure printing, and offset printing, with spin coating, drop casting, or inkjet being preferred since the organic semiconductor layer can be easily and efficiently formed.

An organic semiconductor layer can be formed using the solution for forming an organic semiconductor layer by applying the solution for forming an organic semiconductor layer of the present invention and then removing a solvent through drying.

When the solvent is removed through drying from the applied organic semiconductor layer, a drying condition is not particularly limited, for example, the solvent can be removed through drying under normal pressure or reduced pressure.

A temperature at which an organic solvent is removed through drying from the applied organic semiconductor layer is not particularly limited, but is preferably a temperature ranging from 10 to 150°C since the organic solvent can be efficiently removed through drying from the applied organic semiconductor layer to thereby form an organic semiconductor layer.

When the organic solvent is removed through drying from the applied organic semiconductor layer, crystal growth of the aromatic compound represented by Formula (1-I) or Formula (1-II) can be controlled by adjusting a vaporization rate of the organic solvent to be removed.

A film thickness of the organic semiconductor layer formed using the solution for forming an organic semiconductor layer of the present invention is not particularly limited and is preferably a range of 1 nm to 1 µm and further preferably a range of 10 nm to 300 nm due to satisfactory carrier transfer. Furthermore, the resulting organic semiconductor layer may be annealed at 40 to 180°C after the organic semiconductor layer is formed.

The organic semiconductor layer formed using the solution for forming an organic semiconductor layer of the present invention can be used as an organic semiconductor device including the organic semiconductor layer, in particular, an organic thin film transistor including the organic semiconductor layer.

The organic thin film transistor may be obtained by laminating an organic semiconductor layer onto which a source electrode and a drain electrode are attached and a gate electrode via an insulating layer on a substrate. The organic semiconductor layer formed using the solution for forming an organic semiconductor layer of the present invention can be used as the organic semiconductor layer to produce an organic thin film transistor exhibiting an excellent semiconducting and electric property.

FIGS. 1A to 1D show cross-sectional configurations of a common organic thin film transistor.

In these drawings, FIG.1A shows a bottom gate-top contact organic thin film transistor, FIG.1B shows a bottom gate-bottom contact organic thin film transistor, FIG.1C shows a top gate-top contact organic thin film transistor, and FIG.1D shows a top gate-bottom contact organic thin film transistor; 1 denotes an organic semiconductor layer, 2 denotes a substrate, 3 denotes a gate electrode, 4 denotes a gate insulating layer, 5 denotes a source electrode, and 6 denotes a drain electrode; and the organic semiconductor layer formed using the solution for forming an organic semiconductor layer of the present invention can be applied to any organic thin film transistor.

The substrate according to the present invention is not particularly limited and examples thereof may include a plastic substrate such as polyethylene terephthalate, polyethylene naphthalate, polymethyl methacrylate, polymethyl acrylate, polyethylene, polypropylene, polystyrene, cyclic polyolefin, fluorinated cyclic polyolefin, polyimide, polycarbonate, polyvinylphenol, polyvinyl alcohol, poly(diisopropyl fumarate), poly(diethyl fumarate), poly(diisopropyl maleate), polyethersulfone, polyphenylene sulfide, cellulose triacetate, etc.; an inorganic material substrate such as glass, quartz, aluminum oxide, silicon, highly doped silicon, silicon oxide, tantalum dioxide, tantalum pentoxide, indium tin oxide, etc.; and a metal substrate such as gold, copper, chromium, titanium, aluminum, etc.

Note that, when highly doped silicon is used as the substrate, the substrate can also serve as a gate electrode.

The gate electrode according to the present invention is not particularly limited and examples thereof may include an inorganic material such as aluminum, gold, silver, copper, highly doped silicon, tin oxide, indium oxide, indium tin oxide, chromium, titanium, tantalum, graphene, and carbon nanotube; and an organic material such as a doped conductive polymer (e.g., PEDOT-PSS).

Furthermore, the above-mentioned inorganic material may also be sufficiently used as metal nanoparticle ink.

In this case, the solvent is preferably a polar solvent such as water, methanol, ethanol, 2-propanol, 1-butanol, 2-butanol, etc.; an aliphatic hydrocarbon solvent having 6 to 14 carbon atoms such as hexane, heptane, octane, decane, dodecane, tetradecane, etc.; or an aromatic hydrocarbon solvent having 7 to 14 carbon atoms such as toluene, xylene, mesitylene, ethylbenzene, pentylbenzene, hexylbenzene, octylbenzene, cyclohexylbenzene, tetralin, indane, anisole, 1,2-dimethoxybenzene, 1,3-dimethoxybenzene, 1,2-dimethyl anisole, 2,3-dimethyl anisole, 3,4-dimethyl anisole, etc. from the viewpoint of a proper dispersibility.

After the nanoparticle ink is applied, an annealing treatment is preferably performed at a temperature ranging from 80°C to 200°C in order to improve conductivity.

The gate insulating layer according to the present invention is not particularly limited and examples thereof may include an inorganic material such as silicon oxide, silicon nitride, aluminum oxide, aluminum nitride, titanium oxide, tantalum dioxide, tantalum pentoxide, indium tin oxide, tin oxide, vanadium oxide, barium titanate, bismuth titanate, etc.; and a polymer insulating material such as polymethyl methacrylate, polymethyl acrylate, polyimide, polyamic acid polycarbonate, polyvinylphenol, polyvinyl alcohol, poly(diisopropyl fumarate), poly(diethyl fumarate), polyethylene terephthalate, polyethylene naphthalate, polyethyl cinnamate, polymethyl cinnamate, polyethyl crotonate, polyether sulfone, polypropylene-co-1-butene, polyisobutylene, polypropylene, polycyclopentane, polycyclohexane, polycyclohexane-ethylene copolymer, polyfluorinated cyclopentane, polyfluorinated cyclohexane, a polyfluorinated cyclohexane-ethylene copolymer, BCB resin (product name: CYCLOTENE, manufactured by The Dow Chemical Company), Cytop (trademark), Teflon (trademark), PARYLENE (trademark) such as PARYLENE C, etc., with a polymer insulating material (polymer gate insulating layer) to which a coating method can be applied being preferred due to simple process.

A solvent into which the polymer material is dissolved is not particularly limited and examples thereof include an aliphatic hydrocarbon solvent having 6 to 14 carbon atoms such as hexane, heptane, octane, decane, dodecane, tetradecane; an ether solvent such as THF, 1,2-dimethoxyethane, dioxane; an alcoholic solvent such as ethanol, isopropyl alcohol, 1-butanol, 2-butanol, 2-ethylhexanol, tetrahydrofurfuryl alcohol, etc.; a ketone solvent such as acetone, methyl ethyl ketone, diethyl ketone, diisopropyl ketone, acetophenone, etc.; an ester solvent such as ethyl acetate, γ-butyrolactone, cyclohexanol acetate, 3-methoxybutyl acetate, tetrahydrofurfuryl acetate, tetrahydrofurfuryl propionate, etc.; an amide solvent such as DMF, NMP, etc.; a glycol solvent such as dipropylene glycol dimethyl ether, dipropylene glycol diacetate, dipropylene glycol methyl-n-propyl ether, dipropylene glycol methyl ether acetate, 1,4-butanediol diacetate, 1,3-butylene glycol diacetate, 1,6-hexanediol diacetate, ethylene glycol monomethyl ether acetate, propylene glycol monomethyl ether acetate, ethylene glycol monobutyl ether acetate, diethylene glycol monoethyl ether acetate, diethylene glycol monobutyl ether acetate, etc.; and a fluorinated solvent such as perfluorohexane, perfluorooctane, 2-(pentafluoroethyl)hexane, 3-(pentafluoroethyl)heptane, etc. For example, the polymer insulating material has a concentration of 0.1 to 10.0% by weight at a temperature of 20 to 40°C.

A film thickness of the insulating layer that is formed at the concentration is not particularly limited, but is preferably 100 nm to 1 µm and further preferably 150 nm to 900 nm from the viewpoint of insulation resistance.

The gate insulating layer may have a surface which has been modified with, for example, silanes such as octadecyltrichlorosilane, decyltrichlorosilane, decyltrimethoxysilane, octyltrichlorosilane, octadecyltrimethoxysilane, β-phenetyltrichlorosilane, β-phenetyltrimethoxysilane, phenyltrichlorosilane, phenyltrimethoxysilane, etc.; phosphonic acids such as octadecyl phosphonic acid, decyl phosphonic acid, octyl phosphonic acid, etc.; or silyl amines such as hexamethyldisilazane.

Generally, such a surface treatment of the gate insulating layer increases grain size and improves molecular orientation in the organic semiconductor material, which provides beneficial results such as improvement of carrier mobility and a current ON/OFF ratio and a lower threshold voltage. Materials of the source electrode and the drain electrode of the organic thin film transistor of the present invention are not particularly limited. The materials as described above for the gate electrode may be used. The materials of the source electrode and the drain electrode may be the same as or different. A different type of material may also be laminated thereon.

Furthermore, in order to increase efficiency of carrier injection, these electrode materials may be subjected to a surface treatment.

A surface treatment agent for use in the surface treatment may be, for example, benzenethiol, pentafluorobenzenethiol, 4-fluorobenzenethiol, 4-methoxybenzenethiol, etc.

The organic thin film transistor of the present invention preferably has carrier mobility of 1.00 cm²/V·sec or more from the viewpoint of fast operability.

Furthermore, the current ON/OFF ratio is preferably 1.0 × 10⁶ or more from the viewpoint of a high switching property.

The organic thin film transistor of the present invention can be used for an organic semiconductor layer of a transistor such as electronic paper, an organic light emitting display, a liquid crystal display, an IC tag (RFID tag), a pressure sensor, a biosensor, etc.; an organic light emitting display material; an organic semiconductor laser material; an organic thin film solar cell material; a photonic crystal material; or a semiconductor material for an image sensor, and is preferably used for a semiconductor layer of an organic thin film transistor since the aromatic compound represented by Formula (1-I) or Formula (I-II) forms a crystalline thin film.

### EXAMPLES

Hereinafter, the present invention will be described with reference to Examples in more detail, but the present invention is not limited to these Examples.

For identification of a product, ¹H NMR spectrometry and liquid chromatography-mass spectrometry (LCMS) were used.

### <¹H NMR spectrometry>

Device; Delta V5 (product name) (400 MHz), manufactured by JEOL Ltd.
Measurement temperature: 23°C (unless otherwise indicated)

### <Liquid chromatography-mass spectrometry (LCMS)>

Device; microTOF focus (product name), Bruker Daltonics MS ionization; atmospheric pressure chemical ionization (APCI) method
LC conditions; conditions described in the section "Liquid chromatography" below

Thin-layer chromatography, gas chromatography (GC), or liquid chromatography (LC) was used to monitor progress of a reaction.

The liquid chromatography (LC) was also used to measure purity of an aromatic compound.

### <Thin-layer chromatography>

PLC silica gel 60F254, 0.5 mm for thin-layer chromatography manufactured by Merck KGaA was used and hexane or/and toluene were used as a developing solvent.

### <Gas chromatography>

Device; GC2014 (product name), manufactured by SHIMADZU CORPORATION
Column; Rxi-1HT (product name), 30m, manufactured by Restek Corporation

### <Liquid chromatography>

Device; controller; PX-8020, pump; CCPM-II, and degasser; SD-8022, manufactured by Tosoh Corporation
Column; ODS-100V (product name), 5 µm, 4.6 mm × 250 mm, manufactured by Tosoh Corporation
Column temperature; 33°C
Eluent; dichloromethane:acetonitrile = 2:8 (volume ratio)
Flow rate; 1.0 mL/min
Detector; UV (UV-8020 (product name), manufactured by Tosoh Corporation, wavelength; 254 nm).

A melting point of an aromatic compound was measured using DSC (differential scanning calorimeter).

### <DSC measurement>

Device; model; DSC 6220, manufactured by SII NanoTechnology Inc.
Rate of raising and falling temperature; 10°C/min
Scan range; -10°C to 300°C

Synthesis example 1: Synthesis of dibromodithienobiphenylene derivative (Compound 3) (Step A1) Under a nitrogen atmosphere, in a 100 mL Schlenk reaction vessel, 89.0 mg (0.336 mmol) of a dithienobiphenylene derivative synthesized according to the method described in Japanese Unexamined Patent Application, Publication No. 2018-174322 (Compound 2 in the above-mentioned patent publication) and 9 mL of THF (FUJIFILM Wako Pure Chemical Corporation, dehydrated grade) was added.

The resulting mixture was cooled to -80°C, added with 0.75 mL (1.20 mmol) of 1.6 M n-butyl lithium (Tokyo Chemical Industry Co., Ltd.), stirred for 5 min, and then stirred at room temperature for 25 min.

The resultant was cooled to -78°C, added with 5 mL of a solution of 436 mg (1.34 mmol) of 1,2-dibromotetrachloroethane in THF (FUJIFILM Wako Pure Chemical Corporation, dehydrated grade), and stirred while heating to room temperature.

Water was added thereto, and a solid was filtered and washed with water, methanol, and hexane to thereby obtain 122 mg of a dibromodithienobiphenylene derivative as a yellow solid (yield: 87%).

MS (APCI⁺) m/z: 423 (M⁺+H).

¹H NMR (CDCl₃, 58°C): δ = 7.07 (d, J = 7.4 Hz, 2H), 7.05 (s, 2H), δ = 6.65 (d, J = 7.4 Hz, 2H).

### Synthesis example 2: Synthesis of 2-phenylethylmagnesium bromide (Step B1)

Under a nitrogen atmosphere, in a 100 mL two-neck flask, 738 mg (30.4 mmol) of magnesium (FUJIFILM Wako Pure Chemical Corporation, turnings) was added and the resultant was stirred under vacuum for 3 hours.

Under a nitrogen atmosphere, the resultant was added with 50 mL of a solution of 3.70 g (20.0 mmol) of (2-bromoethyl)benzene (Tokyo Chemical Industry Co., Ltd.) in THF (FUJIFILM Wako Pure Chemical Corporation, dehydrated grade) and stirred at room temperature for 1 hour and at 45°C for 1.5 hours.

A solid was filtered off to thereby obtain a 0.4 M solution of 2-phenylethylmagnesium bromide in THF.

### Example 1: Synthesis of 2,7-di(2-phenylethyl)dithienobiphenylene (Compound 1) (Step C1)

Under a nitrogen atmosphere, in a 50 mL Schlenk tube, 136 mg (0.997 mmol) of zinc chloride (FUJIFILM Wako Pure Chemical Corporation) and 3 mL of THF (FUJIFILM Wako Pure Chemical Corporation, dehydrated grade) was added and the resultant was stirred while cooling on ice.

While cooling on ice, the resultant was added with 1.90 mL (0.760 mmol) of the 0.4 M solution of 2-phenylethylmagnesium bromide in THF synthesized in Synthesis example 2 and stirred at room temperature for 14 hours to thereby prepare a zinc reagent solution.

On the other hand, in a 100 mL Schlenk tube, 41.2 mg (0.0976 mmol) of the dibromodithienobiphenylene derivative synthesized in Synthesis example 1, 5.20 mg (0.00711 mmol) of [1,1'-bis(diphenylphosphino)ferrocene]dichloropalladium (II) (Sigma-Aldrich Co. LLC), and 4 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 5 hours and at 55°C for 1.5 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 5/1).

The resulting solid was rinsed with methanol and purified by recrystallization from hexane/toluene = 12/7 to thereby obtain 16.0 mg of an aromatic compound (Compound 1) as a yellow solid (yield: 35%).

MS (APCI⁺) m/z: 473 (M⁺+H).

¹H NMR (CDCl₃) : δ = 7.32 - 7.22 (m, 10H), 7.00 (d, J = 7.4 Hz, 2H), 6.69 (s, 2H), δ = 6.64 (d, J = 7.4 Hz, 2H), δ = 3.11 (t, J = 7.4 Hz, 4H), δ = 3.03 (t, J = 7.4 Hz, 4H).

Melting point: 195°C

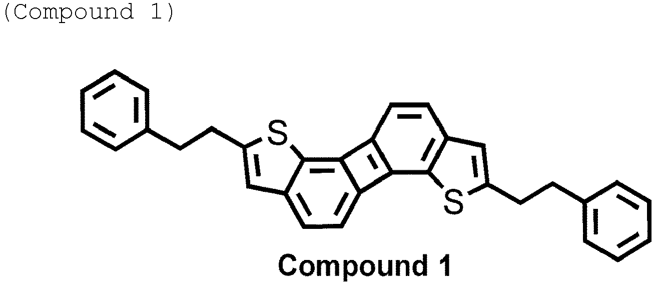

### Synthesis example 3: 1,4-dibromo-2,5-bis(trimethylsilylethynyl)benzene

Under a nitrogen atmosphere, in a 100 mL Schlenk reaction vessel, 2.62 g (5.36 mmol) of 1,4-dibromo-2,5-diiodebenzene (Tokyo Chemical Industry Co., Ltd.), 55.8 mg (0.0794 mmol) of bis(triphenylphosphine)dichloropalladium (FUJIFILM Wako Pure Chemical Corporation), 20.9 mg (0.110 mmol) of copper iodide (I) (FUJIFILM Wako Pure Chemical Corporation), 10 mL of toluene (FUJIFILM Wako Pure Chemical Corporation, dehydrated grade), and 5 mL of triethylamine (FUJIFILM Wako Pure Chemical Corporation) was added.

The resulting mixture was added with 1.67 g (17.0 mmol) of trimethylsilylacetylene (FUJIFILM Wako Pure Chemical Corporation) and stirred at room temperature (25°C) for 50 hours.

The resulting reaction mixture was cooled on ice and quenched with the addition of 1 M hydrochloric acid.

The resultant was extracted with toluene, and the resulting organic phase was washed with water, dried over anhydrous sodium sulfate, and concentrated under reduced pressure.

The resulting residue was purified by silica gel column chromatography (solvent; hexane) to thereby obtain 1.14 g of a solid of interest (yield: 50%).

¹H NMR (CDCl₃): δ = 7.67 (s, 2H), 0.27 (s, 18H).

### Synthesis example 4: 1,4-bis(5-octylthiophene-2-yl)-2,5-bis(trimethylsilylethynyl)benzene

Under a nitrogen atmosphere, in a 50 mL Schlenk reaction vessel, 833 mg (3.02 mmol) of 2-bromo-5-octylthiophene (Tokyo Chemical Industry Co., Ltd.) and 6 mL of THF (FUJIFILM Wako Pure Chemical Corporation, dehydrated grade) was added.

The resulting solution was cooled on ice and added dropwise with 1.6 mL (3.2 mmol) of a solution of ethylmagnesium chloride (Sigma-Aldrich Co. LLC, 2.0 M) in THF.

This mixture was aged at 0°C for 220 min to thereby prepare a solution of 5-octylthiophene-2-ylmagnesium chloride.

On the other hand, under a nitrogen atmosphere, in a 100 mL Schlenk reaction vessel, 527 mg (3.86 mmol) of zinc chloride (FUJIFILM Wako Pure Chemical Corporation) and 7 mL of THF (dehydrated grade) was added and the resultant was cooled to 0°C.

The resulting white fine slurry solution was added dropwise with the above-prepared 5-octylthiophene-2-ylmagnesium chloride solution via a TEFLON (registered trademark) cannular and further added with 2 mL of THF (dehydrated grade) while washing the 100 mL Schlenk reaction vessel and the TEFLON (registered trademark) cannular.

The resulting mixture was stirred while gradually heating to room temperature.

The resulting slurry solution of 5-octylthiophene-2-yl zinc chloride was added with 400 mg (0.933 mmol) of 1,4-dibromo-2,5-bis(trimethylsilylethynyl)benzene synthesized in Synthesis example 3 and 24.0 mg (0.0207 mmol, 2.2% by mole relative to 1,4-dibromo-2,5-bis(trimethylsilylethynyl)benzene) of tetrakis(triphenylphosphine)palladium (Tokyo Chemical Industry Co., Ltd.) as a catalyst.

After reaction at 60°C for 11 hours, the vessel was cooled with water and the reaction was stopped by adding 1M hydrochloric acid.

Toluene was added thereto to thereby separate an organic phase, which was washed with water and dried over anhydrous sodium sulfate.

The resultant was concentrated under reduced pressure and the resulting residue was purified by silica gel column chromatography (solvent: hexane:toluene = 1/0 to 20/1).

As a result, 541 mg of 1,4-bis(5-octylthiophene-2-yl)-2,5-bis(trimethylsilylethynyl)benzene was obtained as a yellow solid (yield: 87%).

¹H NMR (CDCl₃) : δ = 7.68 (s, 2H), 7.52 (d, J = 3.7 Hz, 2H), 6.74 (d, J = 3.7 Hz, 2H), 2.83 (t, J = 7.8 Hz, 4H), 1.71 (m, 4H), 1.43 to 1.28 (m, 20H), 0.90 (t, J = 7.3 Hz, 6H), 0.27 (s. 18H) .

### Synthesis example 5: 1,4-bis(5-octylthiophene-2-yl)-2,5-diethynylbenzene

Under a nitrogen atmosphere, in a 100 mL Schlenk reaction vessel, 540 mg (0.819 mmol) of 1,4-bis(5-octylthiophene-2-yl)-2,5-bis(trimethylsilylethynyl)benzene synthesized in Synthesis example 4, 7 mL of THF, 3.5 mL of methanol, and 45.8 mg (0.331 mmol) of potassium carbonate was added.

This mixture was stirred at room temperature for 3 hours.

The resulting reaction mixture was cooled on ice and the reaction was stopped by adding 1M hydrochloric acid.

Toluene was added thereto to thereby separate an organic phase, which was washed with water twice and dried over anhydrous sodium sulfate.

The resultant was concentrated under reduced pressure to thereby obtain 422 mg of 1,4-bis(5-octylthiophene-2-yl)-2,5-diethynylbenzene as a yellow solid (quantitative).

¹H NMR (CDCl₃): δ = 7.71 (s, 2H), 7.50 (d, J = 3.6 Hz, 2H), 6.76 (d, J = 3.5 Hz, 2H), 3.37 (s, 2H), 2.83 (t, J = 7.8 Hz, 4H), 1.71 (m, 4H), 1.43 to 1.22 (m, 20H), 0.89 (t, J = 7.0 Hz, 6H) .

### Synthesis example 6: 2,8-dioctylanthra[1,2-b:5,6-b']dithiophene

Under a nitrogen atmosphere, in a 50 mL Schlenk reaction vessel, 190 mg (0.370 mmol) of 1,4-bis(5-octylthiophene-2-yl)-2,5-diethynylbenzene synthesized in Synthesis example 5, 6 mL of N,N-dimethylformamide, and 20.4 mg of platinum chloride (FUJIFILM Wako Pure Chemical Corporation) was added.

The resulting mixture was stirred at 80°C for 4 hours and then the solvent was distilled off under reduced pressure.

The resulting residue was purified by silica gel column chromatography (solvent: hexane:toluene = 10/1).

Furthermore, the resultant was purified by recrystallization from hexane/toluene = 4/1 twice to thereby obtain 82 mg of 2,8-dioctylanthra[1,2-b:5,6-b']dithiophene as a red-yellow solid (yield: 43%).

¹H NMR (CDCl₃): δ = 8.58 (s, 2H), 7.83 (d, J = 8.7 Hz, 2H), 6.69 (d, J = 8.7 Hz, 2H), 7.14 (s, 2H), 3.00 (t, J = 7.6 Hz, 4H), 1.81 (m, 4H), 1.45 to 1.22 (m, 20H), 0.90 (t, J = 7.0 Hz, 6H) .

Melting point: 124°C

### (2,8-dioctylanthra[1,2-b:5,6-b']dithiophene)

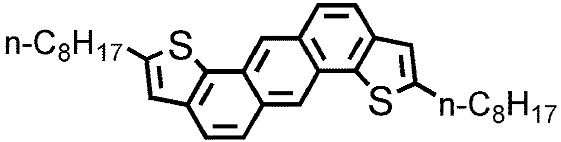

### Example 2 (Production of solution for forming organic semiconductor layer)

In the air, in a 10 mL sample tube, 0.87 mg of 2,7-di(2-phenylethyl)dithienobiphenylene (Compound 1) synthesized in Example 1 and 434 mg of toluene (FUJIFILM Wako Pure Chemical Corporation, pure grade) was added and the resultant was heated to 50°C to dissolve, and then allowed to cool at room temperature (25°C) to thereby prepare a solution for forming an organic semiconductor layer.

The solution for forming an organic semiconductor layer was kept in a solution state after 10 hours at 25°C (concentration of Compound 1: 0.20% by weight), confirming that this was a compound suitable for film formation by drop casting and inkjet.

### Example 3 (Production of organic semiconductor layer and organic thin film transistor)

The solution for forming an organic semiconductor layer obtained in Example 2 was used to produce a top gate-bottom contact p-type organic thin film transistor.

Materials and film formation methods for components are shown in Table 1.

**[Table 1]**

| Component | Component substrate | Method for film formation |
|---|---|---|
| Substrate | Glass | - |
| Gate electrode | Aluminum | Vacuum deposition |
| Gate insulating layer | PARYLENE C | CVD |
| Source/Drain electrode | Gold | Vacuum deposition |
| Surface treatment agent | Pentafluorobenzenethiol | Immersion |
| Organic semiconductor | Compound 1 | Drop casting |

As a result of evaluating the transfer characteristics of the transistor element, carrier mobility for holes was 1.45 cm²/V· sec and a current ON/OFF ratio was 1.6 × 10⁶.

Furthermore, this organic thin film transistor was annealed at 130°C for 10 min and then measured for an electric physical property.

Carrier mobility for holes was 1.40 cm²/V·sec and a current ON/OFF ratio was 1.2 x 10⁶, and there was almost no degradation in performance due to heat treatment.

### Example 4 (Production of organic semiconductor layer and organic thin film transistor)

The solution for forming an organic semiconductor layer obtained in Example 2 and the materials and the film formation methods for components shown in Example 3 were used to produce a bottom gate-bottom contact p-type organic thin film transistor.

As a result of evaluating the transfer characteristics of the transistor element, carrier mobility for holes was 2.29 cm²/V· sec and a current ON/OFF ratio was 1.5 x 10⁶.

Furthermore, this organic thin film transistor was annealed at 130°C for 10 min and then measured for an electric physical property.

Carrier mobility for holes was 2.25 cm²/V·sec and a current ON/OFF ratio was 1.0 x 10⁶, and there was almost no degradation in performance due to heat treatment.

Synthesis example 7: Synthesis of 4-propylbenzene ethanol Under a nitrogen atmosphere, in a 100 mL Schlenk reaction vessel, 5.03 g (25.3 mmol) of 1-bromo-4-propylbenzene (Tokyo Chemical Industry Co., Ltd.) and 80 mL of THF (FUJIFILM Wako Pure Chemical Corporation, dehydrated grade) was added.

The resulting mixture was cooled to -78°C, added with 19.0 mL (30.4 mmol) of 1.6 M n-butyl lithium (Tokyo Chemical Industry Co., Ltd.), and stirred at -78°C for 90 min.

The resultant was added with 25.0 mL (30.0 mmol) of a 1.2 M solution of ethylene oxide in THF (Tokyo Chemical Industry Co., Ltd.) at -78°C and stirred while heating to room temperature.

After 1 M hydrochloric acid was added, diethyl ether was added to separate phases.

The resulting organic phase was washed with water, dried over anhydrous sodium sulfate, and concentrated under reduced pressure.

The resulting residue was purified by silica gel column chromatography (solvent; dichloromethane) to thereby obtain 2.55 g of 4-propylbenzene ethanol as colorless liquid (yield: 58%) .

¹H NMR (CDCl₃): δ = 7.11 (s, 4H), 3.85 (m, 2H), 2.85 (t, J = 6.5 Hz, 2H), 2.57 (t, J = 7.5 Hz, 2H), 1.69 - 1.59 (m, 2H), 1.51 -1.47 (m, 1H), 0.95 (t, J = 7.3 Hz, 3H).

### Synthesis example 8: Synthesis of 1-(2-bromoethyl)-4-propylbenzene

Under a nitrogen atmosphere, in a 100 mL two-neck flask, 1.07 g (6.54 mmol) of 4-propylbenzene ethanol synthesized in Synthesis example 7 and 30 mL of dichloromethane (FUJIFILM Wako Pure Chemical Corporation, dehydrated grade) was added. This solution was cooled on ice, added with 0.60 mL (6.32 mmol) of phosphorus tribromide, stirred for 20 min, and then stirred at room temperature for 22 hours.

The resulting reaction solution was poured into ice, neutralized with a saturated sodium hydrogen carbonate aqueous solution, and then added with dichloromethane to separate phases.

The resulting organic phase was washed with a saturated sodium hydrogen carbonate aqueous solution and water, dried over anhydrous sodium sulfate, and concentrated under reduced pressure.

The resulting residue was purified by silica gel column chromatography (solvent; dichloromethane) to thereby obtain 489 mg of 1-(2-bromoethyl)-4-propylbenzene as colorless liquid (yield: 33%).

¹H NMR (CDCl₃): δ = 7.13 (s, 4H), 3.56 (t, J = 7.7 Hz, 2H), 3.14 (t, J = 7.7 Hz, 2H), 2.57 (t, J = 7.6 Hz, 2H), 1.64 (dt, J = 7.6 Hz, J = 7.3 Hz, 2H), 0.95 (t, J = 7.3 Hz, 3H).

### Synthesis example 9: Synthesis of 2-(4-propylphenyl)ethylmagnesium bromide (Step B1)

Under a nitrogen atmosphere, in a 100 mL two-neck flask, 42.1 mg (1.73 mmol) of magnesium (FUJIFILM Wako Pure Chemical Corporation, turnings) was added and the resultant was stirred under vacuum for 2 hours.

Under a nitrogen atmosphere, the resultant was added with 5 mL of a solution of 231 mg (1.02 mmol) of 1-(2-bromoethyl)-4-propylbenzene synthesized in Synthesis example 8 in THF (FUJIFILM Wako Pure Chemical Corporation, dehydrated grade) and stirred at room temperature for 90 min and at 40°C for 1 hour.

A solid was filtered off to thereby obtain a 0.2 M solution of 2-(4-propylphenyl)ethylmagnesium bromide in THF.

### Example 5: Synthesis of 2,7-di(2-(4-propylphenyl)ethyl)dithienobiphenylene (Compound 4) (Step C1)

Under a nitrogen atmosphere, in a 50 mL Schlenk tube, 169 mg (1.24 mmol) of zinc chloride (FUJIFILM Wako Pure Chemical Corporation) and 2 mL of THF (FUJIFILM Wako Pure Chemical Corporation, dehydrated grade) was added and the resultant was stirred while cooling on ice.

While cooling on ice, the resultant was added with 5.00 mL (1.00 mmol) of the 0.2 M solution of 2-(4-propylphenyl)ethylmagnesium bromide in THF synthesized in Synthesis example 9 and stirred while cooling on ice for 30 min and at room temperature for 14 hours to thereby prepare a zinc reagent solution.

On the other hand, in a 100 mL Schlenk tube, 51.0 mg (0.121 mmol) of the dibromodithienobiphenylene derivative synthesized in Synthesis example 1, 8.60 mg (0.0131 mmol) of [1,1'-bis(diphenylphosphino)ferrocene]dichloropalladium (II) (Sigma-Aldrich Co. LLC), and 6 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 8 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 2/1).

The resulting solid was rinsed with methanol and purified by recrystallization from toluene to thereby obtain 43.6 mg of 2,7-di(2-(4-propylphenyl)ethyl)dithienobiphenylene (Compound 4) as a yellow solid (yield: 65%).

¹H NMR (CDCl₃): δ = 7.13 (m, 8H), 6.99 (d, J = 7.6 Hz, 2H), 6.69 (s, 2H), 6.64 (d, J = 7.6 Hz, 2H), 3.09 (t, J = 6.7 Hz, 4H), 2.99 (t, J = 6.7 Hz, 4H), 2.57 (t, J = 7.4 Hz, 4H), 1.64 (dt, J = 7.4 Hz, J = 7.2 Hz, 4H), 0.95 (t, J = 7.2 Hz, 6H). Melting point: 230°C

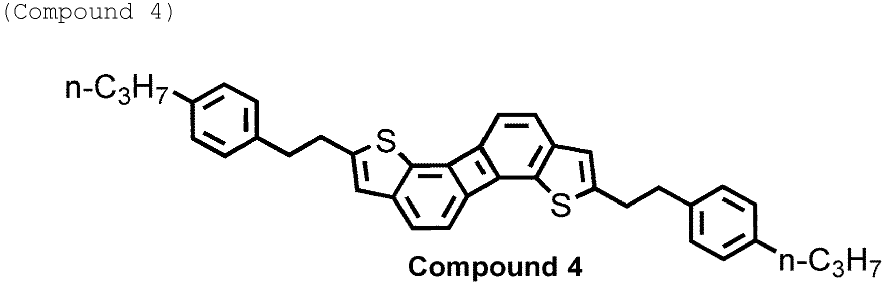

### Synthesis example 10: Synthesis of 4-butylbenzeneethanol

Under a nitrogen atmosphere, in a 300 mL Schlenk reaction vessel, 4.34 g (20.4 mmol) of 1-bromo-4-butylbenzene (Tokyo Chemical Industry Co., Ltd.) and 80 mL of THF (FUJIFILM Wako Pure Chemical Corporation, dehydrated grade) was added.

The resulting mixture was cooled to -78°C, added with 26.0 mL (41.6 mmol) of 1.6 M n-butyl lithium (Tokyo Chemical Industry Co., Ltd.), and stirred at -78°C for 2 hours.

The resultant was added with 25.0 mL (30.0 mmol) of a 1.2 M solution of ethylene oxide in THF (Tokyo Chemical Industry Co., Ltd.) at -78°C and stirred while heating to room temperature.

After 1 M hydrochloric acid was added, diethyl ether was added to separate phases.

The resulting organic phase was washed with water, dried over anhydrous sodium sulfate, and concentrated under reduced pressure.

The resulting residue was purified by silica gel column chromatography (solvent; hexane:dichloromethane = 1/1 to 0/1, ethyl acetate) to thereby obtain 2.20 g of 4-butylbenzeneethanol as colorless liquid (yield: 60%).

¹H NMR (CDCl₃): δ = 7.14 (s, 4H), 3.85 (m, 2H), 2.84 (t, J = 6.5 Hz, 2H), 2.59 (t, J = 7.8 Hz, 2H), 1.63 - 1.56 (m, 2H), 1.41 -1.32 (m, 1H), 0.93 (t, J = 7.3 Hz, 3H).

### Synthesis example 11: Synthesis of 1-(2-bromoethyl)-4-butylbenzene

Under a nitrogen atmosphere, in a 100 mL two-neck flask, 2.20 g (12.3 mmol) of 4-butylbenzeneethanol synthesized in Synthesis example 10 and 30 mL of dichloromethane (FUJIFILM Wako Pure Chemical Corporation, dehydrated grade) was added. This solution was cooled on ice, added with 2.40 mL (25.2 mmol) of phosphorus tribromide, stirred for 10 min, and then stirred at room temperature for 25 hours.

The resulting reaction solution was poured into ice, neutralized with a saturated sodium hydrogen carbonate aqueous solution, and then added with dichloromethane to separate phases.

The resulting organic phase was washed with a saturated sodium hydrogen carbonate aqueous solution and water, dried over anhydrous sodium sulfate, and concentrated under reduced pressure.

The resulting residue was purified by silica gel column chromatography (solvent; dichloromethane) to thereby obtain 922 mg of 1-(2-bromoethyl)-4-butylbenzene as colorless liquid (yield: 31%).

¹H NMR (CDCl₃): δ = 7.13 (m, 4H), 3.56 (t, J = 7.7 Hz, 2H), 3.14 (t, J = 7.7 Hz, 2H), 2.59 (t, J = 7.7 Hz, 2H), 1.61 - 1.57 (m, 2H), 1.41 - 1.31 (m, 2H), 0.93 (t, J = 7.3 Hz, 3H).

### Synthesis example 12: Synthesis of 2-(4-butylphenyl)ethylmagnesium bromide (Step B1)

Under a nitrogen atmosphere, in a 100 mL two-neck flask, 41.2 mg (1.69 mmol) of magnesium (FUJIFILM Wako Pure Chemical Corporation, turnings) was added and the resultant was stirred under vacuum for 2 hours.

Under a nitrogen atmosphere, the resultant was added with 5 mL of a solution of 248 mg (1.03 mmol) of 1-(2-bromoethyl)-4-butylbenzene synthesized in Synthesis example 11 in THF (FUJIFILM Wako Pure Chemical Corporation, dehydrated grade) and stirred at room temperature for 30 min and at 40°C for 1 hour.

A solid was filtered off to thereby obtain a 0.2 M solution of 2-(4-butylphenyl)ethylmagnesium bromide in THF .

### Example 6: Synthesis of 2,7-di(2-(4-butylphenyl)ethyl)dithienobiphenylene (Compound 5) (Step C1)

Under a nitrogen atmosphere, in a 50 mL Schlenk tube, 189 mg (1.39 mmol) of zinc chloride (FUJIFILM Wako Pure Chemical Corporation) and 4 mL of THF (FUJIFILM Wako Pure Chemical Corporation, dehydrated grade) was added and the resultant was stirred while cooling on ice.

While cooling on ice, the resultant was added with 5.00 mL (1.00 mmol) of the 0.2 M solution of 2-(4-butylphenyl)ethylmagnesium bromide in THF synthesized in Synthesis example 12 and stirred while cooling on ice for 30 min and at room temperature for 17 hours to thereby prepare a zinc reagent solution.

On the other hand, in a 100 mL Schlenk tube, 50.6 mg (0.120 mmol) of the dibromodithienobiphenylene derivative synthesized in Synthesis example 1, 9.40 mg (0.0128 mmol) of [1,1'-bis(diphenylphosphino)ferrocene]dichloropalladium (II) (Sigma-Aldrich Co. LLC), and 6 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 4 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 2/1).

The resulting solid was rinsed with methanol and purified by recrystallization from hexane/toluene = 1/1 to thereby obtain 37.4 mg of 2,7-di(2-(4-butylphenyl)ethyl)dithienobiphenylene (Compound 5) as a yellow solid (yield: 53%).

¹H NMR (CDCl₃): δ = 7.12 (m, 8H), 6.99 (d, J = 7.4 Hz, 2H), 6.69 (s, 2H), 6.64 (d, J = 7.4 Hz, 2H), 3.09 (t, J = 6.6 Hz, 4H), 2.99 (t, J = 6.6 Hz, 4H), 2.59 (t, J = 7.7 Hz, 4H), 1.61 - 1.57 (m, 4H), 1.40 - 1.31 (m, 4H), 0.93 (t, J = 7.3 Hz, 6H). Melting point: 211°C

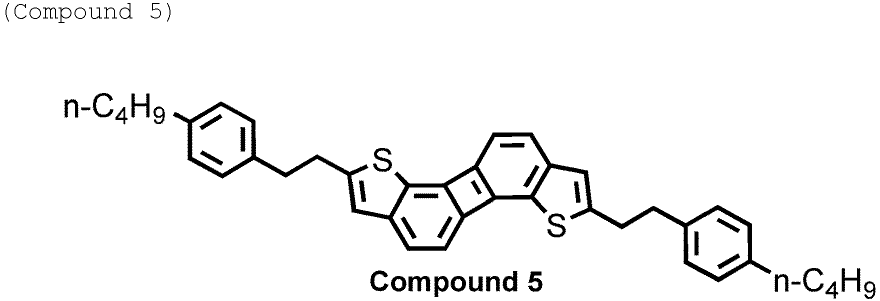

### Synthesis example 13: Synthesis of 4-heptylbenzeneethanol

Under a nitrogen atmosphere, in a 300 mL Schlenk reaction vessel, 5.18 g (20.3 mmol) of 1-bromo-4-heptylbenzene (Tokyo Chemical Industry Co., Ltd.) and 80 mL of THF (FUJIFILM Wako Pure Chemical Corporation, dehydrated grade) was added.

The resulting mixture was cooled to -78°C, added with 26.0 mL (41.6 mmol) of 1.6 M n-butyl lithium (Tokyo Chemical Industry Co., Ltd.), and stirred at -78°C for 4 hours.

The resultant was added with 25.0 mL (30.0 mmol) of a 1.2 M solution of ethylene oxide in THF (Tokyo Chemical Industry Co., Ltd.) at -78°C and stirred while heating to room temperature.

After 1 M hydrochloric acid was added, diethyl ether was added to separate phases.

The resulting organic phase was washed with water, dried over anhydrous sodium sulfate, and concentrated under reduced pressure.

The resulting residue was purified by silica gel column chromatography (solvent; dichloromethane, ethyl acetate) to thereby obtain 3.30 g of 4-heptylbenzeneethanol as colorless liquid (yield: 74%).

¹H NMR (CDCl₃): δ = 7.14 (s, 4H), 3.85 (m, 2H), 2.85 (t, J = 6.6 Hz, 2H), 2.58 (t, J = 7.6 Hz, 2H), 1.60 (m, 2H), 1.46 (bs, 1H), 1.30 (m, 8H), 0.89 (t, J = 6.8 Hz, 3H).

### Synthesis example 14: Synthesis of 1-(2-bromoethyl)-4-heptylbenzene

Under a nitrogen atmosphere, in a 100 mL two-neck flask, 3.15 g (14.3 mmol) of 4-heptylbenzeneethanol synthesized in Synthesis example 13 and 30 mL of dichloromethane (FUJIFILM Wako Pure Chemical Corporation, dehydrated grade) was added. This solution was cooled on ice, added with 1.40 mL (14.7 mmol) of phosphorus tribromide, stirred for 30 min, and then stirred at room temperature for 19 hours.

The resulting reaction solution was poured into ice, neutralized with a saturated sodium hydrogen carbonate aqueous solution, and then added with dichloromethane to separate phases.

The resulting organic phase was washed with a saturated sodium hydrogen carbonate aqueous solution and water, dried over anhydrous sodium sulfate, and concentrated under reduced pressure.

The resulting residue was purified by silica gel column chromatography (solvent; dichloromethane) to thereby obtain 1.74 g of 1-(2-bromoethyl)-4-heptylbenzene as colorless liquid (yield: 43%).

¹H NMR (CDCl₃): δ = 7.12 (m, 4H), 3.56 (t, J = 7.4 Hz, 2H), 3.13 (t, J = 7.4 Hz, 2H), 2.58 (t, J = 7.6 Hz, 2H), 1.60 (m, 2H), 1.30 (m, 8H), 0.88 (t, J = 7.0 Hz, 3H).

### Synthesis example 15: Synthesis of 2-(4-heptylphenyl)ethylmagnesium bromide (Step B1)

Under a nitrogen atmosphere, in a 100 mL two-neck flask, 42.1 mg (1.73 mmol) of magnesium (FUJIFILM Wako Pure Chemical Corporation, turnings) was added and the resultant was stirred under vacuum for 2 hours.

Under a nitrogen atmosphere, the resultant was added with 5 mL of a solution of 288 mg (1.02 mmol) of 1-(2-bromoethyl)-4-heptylbenzene synthesized in Synthesis example 14 in THF (FUJIFILM Wako Pure Chemical Corporation, dehydrated grade) and stirred at room temperature for 40 min and at 40°C for 1 hour.

A solid was filtered off to thereby obtain a 0.2 M solution of 2-(4-heptylphenyl)ethylmagnesium bromide in THF.

Example 7: Synthesis of 2,7-di(2-(4-heptylphenyl)ethyl)dithienobiphenylene (Compound 6) (Step C1) Under a nitrogen atmosphere, a in 50 mL Schlenk tube, 190 mg (1.40 mmol) of zinc chloride (FUJIFILM Wako Pure Chemical Corporation) and 4 mL of THF (FUJIFILM Wako Pure Chemical Corporation, dehydrated grade) was added and the resultant was stirred while cooling on ice.

While cooling on ice, the resultant was added with 5.00 mL (1.00 mmol) of the 0.2 M solution of 2-(4-heptylphenyl)ethylmagnesium bromide in THF synthesized in Synthesis example 15 and stirred while cooling on ice for 1 hour and at room temperature for 16 hours to thereby prepare a zinc reagent solution.

On the other hand, in a 50 mL Schlenk tube, 49.8 mg (0.118 mmol) of the dibromodithienobiphenylene derivative synthesized in Synthesis example 1, 9.60 mg (0.0131 mmol) of [1,1'-bis(diphenylphosphino)ferrocene]dichloropalladium (II) (Sigma-Aldrich Co. LLC), and 6 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 23 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 2/1).

The resulting solid was rinsed with methanol and purified by recrystallization from hexane/toluene = 1/1 to thereby obtain 36.1 mg of 2,7-di(2-(4-heptylphenyl)ethyl)dithienobiphenylene (Compound 6) as a yellow solid (yield: 46%).

¹H NMR (CDCl₃): δ = 7.12 (m, 8H), 6.99 (d, J = 7.5 Hz, 2H), 6.69 (s, 2H), 6.64 (d, J = 7.5 Hz, 2H), 3.09 (t, J = 6.8 Hz, 4H), 2.99 (t, J = 6.8 Hz, 4H), 2.58 (t, J = 7.6 Hz, 4H), 1.60 (m, 4H), 1.31 (m, 16H), 0.88 (t, J = 7.1 Hz, 6H).

Melting point: 198°C

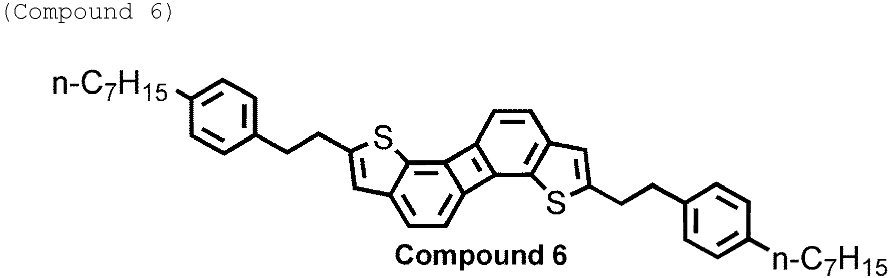

### Synthesis example 16: Synthesis of 2-(2,3-dihydrobenzofuran-5-yl)ethylmagnesium bromide (Step B1)

Under a nitrogen atmosphere, in a 100 mL two-neck flask, 369 mg (15.2 mmol) of magnesium (FUJIFILM Wako Pure Chemical Corporation, turnings) was added and the resultant was stirred under vacuum for 2 hours.

Under a nitrogen atmosphere, the resultant was added with 25 mL of a solution of 2.27 g (10.0 mmol) of 5-(2-bromoethyl)-2,3-dihydrobenzofuran (Tokyo Chemical Industry Co., Ltd.) in THF (FUJIFILM Wako Pure Chemical Corporation, dehydrated grade) and stirred at room temperature for 1 hour and at 40°C for 1 hour.

A solid was filtered off to thereby obtain a 0.4 M solution of 2-(2,3-dihydrobenzofuran-5-yl)ethylmagnesium bromide in THF.

### Example 8: Synthesis of aromatic compound (Compound 7) (Step C1)

Under a nitrogen atmosphere, in a 50 mL Schlenk tube, 173 mg (1.30 mmol) of zinc chloride (FUJIFILM Wako Pure Chemical Corporation) and 5 mL of THF (FUJIFILM Wako Pure Chemical Corporation, dehydrated grade) was added and the resultant was stirred while cooling on ice.

While cooling on ice, the resultant was added with 2.50 mL (1.00 mmol) of the 0.4 M solution of 2-(2,3-dihydrobenzofuran-5-yl)ethylmagnesium bromide in THF synthesized in Synthesis example 16 and stirred while cooling on ice for 30 min and at room temperature for 16 hours to thereby prepare a zinc reagent solution.

On the other hand, in a 50 mL Schlenk tube, 50.8 mg (0.120 mmol) of the dibromodithienobiphenylene derivative synthesized in Synthesis example 1 (Compound 3), 10.4 mg (0.0142 mmol) of [1,1'-bis(diphenylphosphino)ferrocene]dichloropalladium (II) (Sigma-Aldrich Co. LLC), and 8 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 6 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/2 to 0/1).

The resulting solid was rinsed with methanol and purified by recrystallization from toluene to thereby obtain 4.0 mg of an aromatic compound (Compound 7) as a yellow solid (yield: 6%).

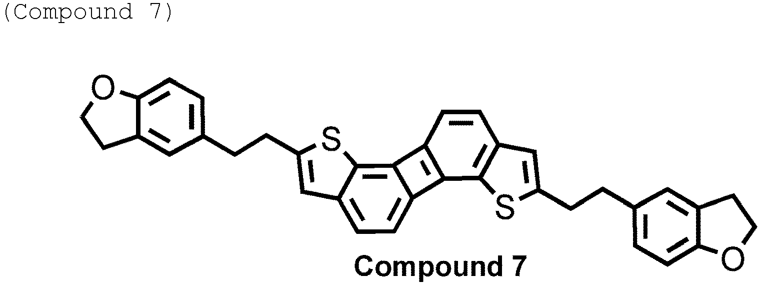

### Synthesis example 17: Synthesis of 2-(1,2-methylenedioxybenzene-4-yl) ethanol

Under a nitrogen atmosphere, in a 300 mL Schlenk reaction vessel, 4.03 g (20.1 mmol) of 4-bromo-1,2-methylenedioxybenzene (Tokyo Chemical Industry Co., Ltd.) and 80 mL of THF (FUJIFILM Wako Pure Chemical Corporation, dehydrated grade) was added.

The resulting mixture was cooled to -78°C, added with 26.0 mL (41.6 mmol) of 1.6 M n-butyl lithium (Tokyo Chemical Industry Co., Ltd.), and stirred at -78°C for 4 hours.

The resultant was added with 25.0 mL (30.0 mmol) of a 1.2 M solution of ethylene oxide in THF (Tokyo Chemical Industry Co., Ltd.) at -78°C and stirred while heating to room temperature.

After 1 M hydrochloric acid was added, diethyl ether was added to separate phases.

The resulting organic phase was washed with water, dried over anhydrous sodium sulfate, and concentrated under reduced pressure.

The resulting residue was purified by silica gel column chromatography (solvent; dichloromethane, ethyl acetate) to thereby obtain 2.91 g of 2-((1,2-methylenedioxybenzene-4-yl)ethanol as colorless liquid (yield: 72%).

¹H NMR (CDCl₃): δ = 6.76 (d, J = 7.8 Hz, 1H), 6.72 (s, 1H), 6.68 (d, J = 7.8 Hz, 1H), 5.94 (s, 2H), 3.82 (m, 2H), 2.79 (t, J = 6.4 Hz, 2H), 1.43 (m, 1H).

### Synthesis example 18: Synthesis of 4-(2-bromoethyl)-1,2-methylenedioxybenzene

Under a nitrogen atmosphere, in a 100 mL two-neck flask, 2.78 g (16.7 mmol) of 2-(1,2-methylenedioxybenzene-4-yl)ethanol synthesized in Synthesis example 17 and 20 mL of dichloromethane (FUJIFILM Wako Pure Chemical Corporation, dehydrated grade) was added.

This solution was cooled on ice, added with 1.60 mL (16.8 mmol) of phosphorus tribromide, stirred for 30 min, and then stirred at room temperature for 20 hours.

The resulting reaction solution was poured into ice, neutralized with a saturated sodium hydrogen carbonate aqueous solution, and then added with dichloromethane to separate phases.

The resulting organic phase was washed with a saturated sodium hydrogen carbonate aqueous solution and water, dried over anhydrous sodium sulfate, and concentrated under reduced pressure.

The resulting residue was purified by silica gel column chromatography (solvent; dichloromethane/hexane = 1:5) to thereby obtain 1.03 g of 4-(2-bromoethyl)-1,2-methylenedioxybenzene as colorless liquid (yield: 33%).

¹H NMR (CDCl₃): δ = 6.76 (d, J = 7.9 Hz, 1H), 6.69 (s, 1H), 6.66 (d, J = 7.9 Hz, 1H), 5.95 (s, 2H), 2.52 (t, J = 7.6 Hz, 2H), 3.08 (t, J = 7.6 Hz, 2H).

### Synthesis example 19: Synthesis of 2-(1,2-methylenedioxybenzene-4-yl)ethylmagnesium bromide (Step B1)

Under a nitrogen atmosphere, in a 100 mL two-neck flask, 39.1 mg (1.61 mmol) of magnesium (FUJIFILM Wako Pure Chemical Corporation, turnings) was added and the resultant was stirred under vacuum for 2 hours.

Under a nitrogen atmosphere, the resultant was added with 5 mL of a solution of 229 mg (1.00 mmol) of 4-(2-bromoethyl)-1,2-methylenedioxybenzene synthesized in Synthesis example 18 in THF (FUJIFILM Wako Pure Chemical Corporation, dehydrated grade) and stirred at room temperature for 30 min and at 40°C for 1 hour.

A solid was filtered off to thereby obtain a 0.2 M solution of 2-(1,2-methylenedioxybenzene-4-yl)ethylmagnesium bromide in THF.

### Example 9: Synthesis of aromatic compound (Compound 8) (Step C1)

Under a nitrogen atmosphere, in a 50 mL Schlenk tube, 187 mg (1.38 mmol) of zinc chloride (FUJIFILM Wako Pure Chemical Corporation) and 7 mL of THF (FUJIFILM Wako Pure Chemical Corporation, dehydrated grade) was added and the resultant was stirred while cooling on ice.

While cooling on ice, the resultant was added with 5.0 mL (1.00 mmol) of the 0.2 M solution of 2-(1,2-methylenedioxybenzene-4-yl)ethylmagnesium bromide in THF synthesized in Synthesis example 19 and stirred while cooling on ice for 1 hour and at room temperature for 15 hours to thereby prepare a zinc reagent solution.

On the other hand, in a 50 mL Schlenk tube, 50.6 mg (0.112 mmol) of the dibromodithienobiphenylene derivative synthesized in Synthesis example 1 (Compound 3), 10.8 mg (0.0148 mmol) of [1,1'-bis(diphenylphosphino)ferrocene]dichloropalladium (II) (Sigma-Aldrich Co. LLC), and 8 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 8 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/2 to 0/1).

The resulting solid was rinsed with methanol and purified by recrystallization from toluene to thereby obtain 34.7 mg of an aromatic compound (Compound 8) as a yellow solid (yield: 55%).

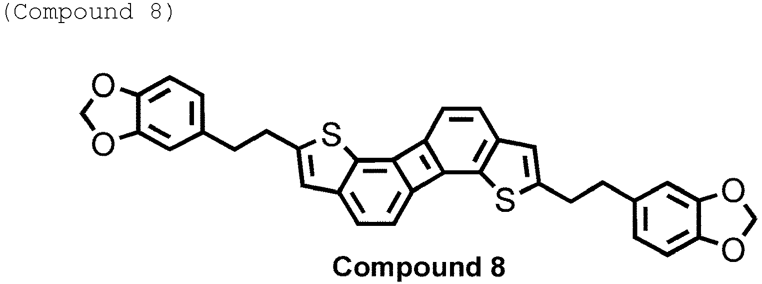

### Synthesis example 20: Synthesis of 1,4-bis(thiophene-2-yl)-2,5-bis(trimethylsilylethynyl)benzene

Under a nitrogen atmosphere, in a 300 mL Schlenk reaction vessel, 8.18 g (50.2 mmol) of 2-bromo-thiophene (Tokyo Chemical Industry Co., Ltd.) and 90 mL of THF (FUJIFILM Wako Pure Chemical Corporation, dehydrated grade) was added.

The resulting solution was cooled on ice and added dropwise with 28.0 mL (56.0 mmol) of a solution of ethylmagnesium chloride (Sigma-Aldrich Co. LLC, 2.0 M) in THF.

This mixture was aged at 0°C for 3 hours to thereby prepare a solution of thiophene-2-ylmagnesium chloride.

On the other hand, under a nitrogen atmosphere, in a 500 mL Schlenk reaction vessel, 9.31 g (68.3 mmol) of zinc chloride (FUJIFILM Wako Pure Chemical Corporation) and 110 mL of THF (dehydrated grade) was added and the resultant was cooled to 0°C.

The resulting white fine slurry solution was added dropwise with the above-prepared thiophene-2-ylmagnesium chloride solution via a TEFLON (registered trademark) cannular and further added with 40 mL of THF (dehydrated grade) while washing the 100 mL Schlenk reaction vessel and the TEFLON (registered trademark) cannular.

The resulting mixture was stirred while gradually heating to room temperature.

The resulting slurry solution of thiophene-2-yl zinc chloride was added with 6.56 g (15.3 mmol) of 1,4-dibromo-2,5-bis(trimethylsilylethynyl)benzene synthesized in Synthesis example 3 and 515 mg (0.445 mmol, 2.9% by mole relative to 1,4-dibromo-2,5-bis(trimethylsilylethynyl)benzene) of tetrakis(triphenylphosphine)palladium (Tokyo Chemical Industry Co., Ltd.) as a catalyst.

After reaction at 60°C for 7 hours, the vessel was cooled with water and quenched with the addition of 1 M hydrochloric acid. Toluene was added thereto to thereby separate an organic phase, which was washed with water and dried over anhydrous sodium sulfate.

The resultant was concentrated under reduced pressure and the resulting residue was purified by silica gel column chromatography (solvent: hexane:toluene = 1/10).

As a result, 5.44 g of 1,4-bis(thiophene-2-yl)-2,5-bis(trimethylsilylethynyl)benzene was obtained as a yellow solid (yield: 82%).

¹H NMR (CDCl₃): δ = 7.74 (s, 2H), 7.70 (dd, J = 1.4 Hz, J = 3.7 Hz, 2H), 7.37 (dd, J = 1.3 Hz, J = 5.1 Hz, 2H), 7.09 (dd, J = 3.7 Hz, J = 5.1 Hz, 2H), 0.26 (s, 18H).

### Synthesis example 21: Synthesis of 1,4-bis(thiophene-2-yl)-2,5-diethynylbenzene

Under a nitrogen atmosphere, in a 300 mL eggplant flask, 5.38 g (12.4 mmol) of 1,4-bis(thiophene-2-yl)-2,5-bis(trimethylsilylethynyl)benzene synthesized in Synthesis example 20, 100 mL of THF, 50 mL of methanol, and 801 mg (5.79 mmol) of potassium carbonate was added.

This mixture was stirred at room temperature for 6 hours.

The resulting reaction mixture was cooled on ice and the reaction was stopped by adding 1M hydrochloric acid.

Toluene was added thereto to thereby separate an organic phase, which was washed with water twice and dried over anhydrous sodium sulfate.

The resultant was concentrated under reduced pressure to thereby obtain 3.53 g of 1,4-bis(thiophene-2-yl)-2,5-diethynylbenzene as a yellow solid (quantitative).

¹H NMR (CDCl₃): δ = 7.78 (s, 2H), 7.67 (dd, J = 1.3 Hz, J = 3.7 Hz, 2H), 7.39 (dd, J = 1.0 Hz, J = 5.1 Hz, 2H), 7.12 (dd, J = 3.7 Hz, J = 5.1 Hz, 2H), 3.37 (s, 2H).

### Synthesis example 22: Synthesis of anthra[1,2-b:5,6-b']dithiophene (Compound 19)

Under a nitrogen atmosphere, in a 200 mL Schlenk reaction vessel, 1.74 g (6.00 mmol) of 1,4-bis(thiophene-2-yl)-2,5-diethynylbenzene synthesized in Synthesis example 21, 60 mL of N,N-dimethylformamide, and 323 mg (1.21 mmol) of platinum chloride (FUJIFILM Wako Pure Chemical Corporation) was added. The resulting mixture was stirred at 80°C for 4 hours and then the solvent was distilled off under reduced pressure.

The resulting residue was purified by silica gel column chromatography (solvent: hexane:toluene = 10/1 to 2/1) to thereby obtain 701 mg of anthra[1,2-b:5,6-b']dithiophene (Compound 19) as a yellow solid (yield: 40%).

¹H NMR (CDCl₃): δ = 8.73 (s, 2H), 7.92 (d, J = 8.9 Hz, 2H), 7.84 (d, J = 8.9 Hz, 2H), 7.56 (d, J = 5.4 Hz, 2H), 7.56 (d, J = 5.4 Hz, 2H).

### Synthesis example 23: Synthesis of 2,8-dibromoanthra[1,2-b:5,6-b']dithiophene (Compound 20) (Step A-2)

Under a nitrogen atmosphere, in a 100 mL Schlenk reaction vessel, 602 mg (2.07 mmol) of anthra[1,2-b:5,6-b']dithiophene (Compound 19) synthesized in Synthesis example 22 and 50 mL of THF (FUJIFILM Wako Pure Chemical Corporation, dehydrated grade) was added.

The resulting mixture was cooled to -78°C, added with 6.00 mL (9.60 mmol) of 1.6 M n-butyl lithium (Tokyo Chemical Industry Co., Ltd.), stirred for 20 min, and then stirred at room temperature for 50 min.

The resultant was cooled to -78°C, added with 25 mL of a solution of 3.45 g (10.6 mmol) of 1,2-dibromotetrachloroethane in THF (FUJIFILM Wako Pure Chemical Corporation, dehydrated grade), and stirred while heating to room temperature.

Water was added thereto and a solid was filtered and washed with water, methanol, and hexane to thereby obtain 761 mg of 2,8-dibromoanthra[1,2-b:5,6-b']dithiophene (Compound 20) as a yellow solid (yield: 82%).

¹H NMR (CDCl₃, 50°C): δ = 8.53 (s, 2H), 7.87 (d, J = 8.9 Hz, 2H), 7.87 (d, J = 8.9 Hz, 2H), 7.47 (s, 2H).

### Example 10: Synthesis of 2,8-di(2-phenylethyl)anthra[1,2-b:5,6-b']dithiophene (Compound 10) (Step C2)

Under a nitrogen atmosphere, in a 50 mL Schlenk tube, 413 mg (3.02 mmol) of zinc chloride (FUJIFILM Wako Pure Chemical Corporation) and 4 mL of THF (FUJIFILM Wako Pure Chemical Corporation, dehydrated grade) was added and the resultant was stirred while cooling on ice.

While cooling on ice, the resultant was added with 5.60 mL (2.34 mmol) of the 0.4 M solution of 2-phenylethylmagnesium bromide in THF synthesized in Synthesis example 2 and stirred at 0°C for 40 min and at room temperature for 16 hours to thereby prepare a zinc reagent solution.

On the other hand, in a 100 mL Schlenk tube, 156 mg (0.348 mmol) of 2,8-dibromoanthra[1,2-b:5,6-b']dithiophene (Compound 20) synthesized in Synthesis example 23, 48.5 mg (0.0662 mmol) of [1,1'-bis(diphenylphosphino)ferrocene]dichloropalladium (II) (Sigma-Aldrich Co. LLC), and 20 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 24 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 5/1).

The resulting solid was rinsed with methanol and purified by recrystallization from toluene to thereby obtain 73.2 mg of 2,8-di(2-phenylethyl)anthra[1,2-b:5,6-b']dithiophene (Compound 10) as a yellow solid (yield: 42%).

¹H NMR (CDCl₃): δ = 8.59 (s, 2H), 7.85 (d, J = 8.8 Hz, 2H), 7.69 (d, J = 8.8 Hz, 2H), 7.28 (m, 10H), 7.14 (s, 2H), 3.34 (t, J = 7.6 Hz, 4H), 3.15 (t, J = 7.6 Hz, 4H). Melting point: 221°C

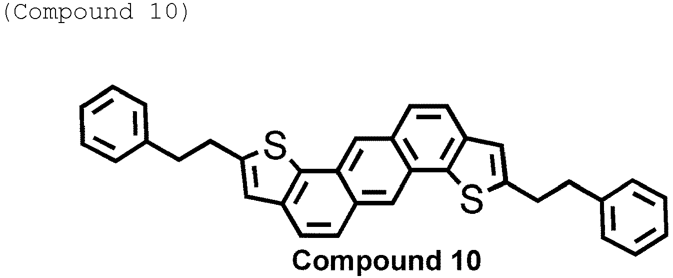

### Example 11: Synthesis of 2-(2-phenylethyl)anthra[1,2-b:5,6-b']dithiophene (Compound 15) (Step C2)

Other components obtained by silica gel column chromatography in Example 10 were purified by recrystallization from hexane to thereby obtain 2.0 mg of 2-(2-phenylethyl)anthra[1,2-b:5,6-b']dithiophene (Compound 15) as a yellow solid (yield: 1.5%). ¹H NMR (CDCl₃): δ = 8.70 (s, 1H), 8.62 (s, 1H), 7.90 (d, J = 8.6 Hz, 1H), 7.88 (d, J = 8.4 Hz, 1H), 7.82 (d, J = 8.7 Hz, 1H), 7.71 (d, J = 8.8 Hz, 1H), 7.54 (d, J = 5.1 Hz, 1H), 7.49 (d, J = 5.4 Hz, 1H), 7.30 (m, 5H), 7.15 (s, 1H), 3.34 (t, J = 7.7 Hz, 4H), 3.16 (t, J = 7.3 Hz, 4H).

Melting point: 174°C

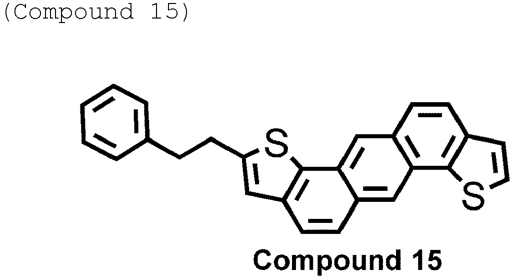

Example 12: Synthesis of 2,8-di(2-(4-n-butylphenyl)ethyl)anthra[1,2-b:5,6-b']dithiophene (Compound 11) (Step C2)

Under a nitrogen atmosphere, in a 50 mL Schlenk tube, 194 mg (1.42 mmol) of zinc chloride (FUJIFILM Wako Pure Chemical Corporation) and 4 mL of THF (FUJIFILM Wako Pure Chemical Corporation, dehydrated grade) was added and the resultant was stirred while cooling on ice.

While cooling on ice, the resultant was added with 4.5 mL (0.90 mmol) of the 0.2 M solution of 2-(4-butylphenyl)ethylmagnesium bromide in THF synthesized in Synthesis example 12 and stirred at 0°C for 30 min and at room temperature for 16 hours to thereby prepare a zinc reagent solution.

On the other hand, in a 100 mL Schlenk tube, 39.7 mg (0.0886 mmol) of 2,8-dibromoanthra[1,2-b:5,6-b']dithiophene (Compound 20) synthesized in Synthesis example 23, 7.96 mg (0.0109 mmol) of [1,1'-bis(diphenylphosphino)ferrocene]dichloropalladium (II) (Sigma-Aldrich Co. LLC), and 3 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 24 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 5/1).

The resulting solid was rinsed with methanol and purified by recrystallization from hexane/toluene = 6/5 to thereby obtain 12.6 mg of 2,8-di(2-(4-n-butylphenyl)ethyl)anthra[1,2-b:5,6-b']dithiophene (Compound 11) as a yellow solid (yield: 23%). ¹H NMR (CDCl₃): δ = 8.59 (s, 2H), 7.85 (d, J = 8.7 Hz, 2H), 7.69 (d, J = 8.9 Hz, 2H), 7.19 (d, J = 8.0 Hz, 2H), 7.15 (s, 2H), 7.13 (d, J = 8.0 Hz, 2H), 3.32 (t, J = 7.5 Hz, 4H), 3.12 (t, J = 8.5 Hz, 4H), 2.61 (t, J = 7.6 Hz, 4H), 1.61 (m, 4H), 1.37 (m, 4H), 0.94 (t, J = 7.3 Hz, 6H).

Melting point: 227°C

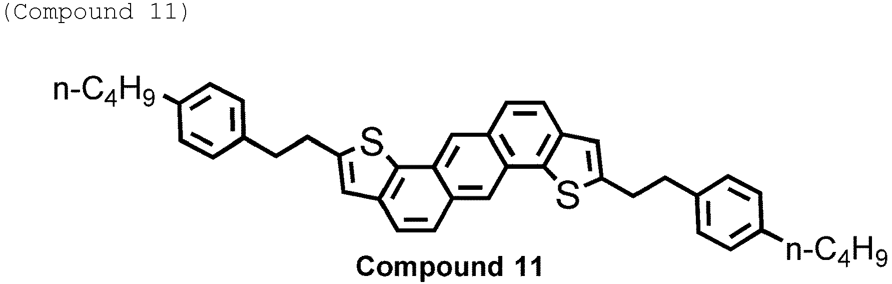

### Example 13: Synthesis of 2-(2-(4-n-butylphenyl)ethyl)anthra[1,2-b:5,6-b']dithiophene (Compound 17) (Step C2)

Other components obtained by silica gel column chromatography in Example 12 were purified by recrystallization from hexane to thereby obtain 5.8 mg of 2-(2-(4-n-butylphenyl)ethyl)anthra[1,2-b:5,6-b']dithiophene (Compound 17) as a yellow solid (yield: 15%).

¹H NMR (CDCl₃): δ = 8.70 (s, 1H), 8.62 (s, 1H), 7.89 (d, J = 8.5 Hz, 1H), 7.87 (d, J = 8.0 Hz, 1H), 7.82 (d, J = 8.8 Hz, 1H), 7.71 (d, J = 8.8 Hz, 1H), 7.54 (d, J = 5.4 Hz, 1H), 7.49 (d, J = 5.1 Hz, 1H), 7.19 (d, J = 8.0 Hz, 2H), 7.16 (s, 1H), 7.13 (d, J = 8.1 Hz, 2H), 3.32 (t, J = 7.4 Hz, 2H), 3.12 (t, J = 8.6 Hz, 2H), 2.61 (t, J = 7.7 Hz, 2H), 1.60 (m, 2H), 1.37 (m, 2H), 0.94 (t, J = 7.3 Hz, 3H).

Melting point: 161°C

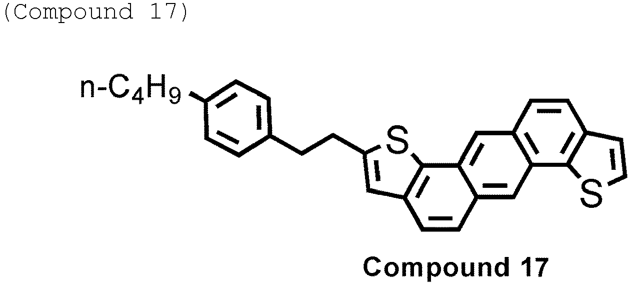

### Example 14: Synthesis of 2,8-di(2-(4-n-heptylphenyl)ethyl)anthra[1,2-b:5,6-b']dithiophene (Compound 12) (Step C2)

Under a nitrogen atmosphere, in a 50 mL Schlenk tube, 124 mg (0.909 mmol) of zinc chloride (FUJIFILM Wako Pure Chemical Corporation) and 2 mL of THF (FUJIFILM Wako Pure Chemical Corporation, dehydrated grade) was added and the resultant was stirred while cooling on ice.

While cooling on ice, the resultant was added with 3.4 mL (0.68 mmol) of the 0.2 M solution of 2-(4-heptylphenyl)ethylmagnesium bromide in THF synthesized in Synthesis example 15 and stirred at 0°C for 20 min and at room temperature for 16 hours to thereby prepare a zinc reagent solution.

On the other hand, in a 100 mL Schlenk tube, 31.4 mg (0.0701 mmol) of 2,8-dibromoanthra[1,2-b:5,6-b']dithiophene (Compound 20) synthesized in Synthesis example 23, 5.20 mg (0.00711 mmol) of [1,1'-bis(diphenylphosphino)ferrocene]dichloropalladium (II) (Sigma-Aldrich Co. LLC), and 3 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 90 min and at 50°C for 1 hour.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure, and the resulting solid was rinsed with methanol. The resulting residue was purified by silica gel column chromatography (solvent; hexane:toluene = 1/0 to 5/1).

The resulting solid was rinsed with methanol and purified by recrystallization from hexane/toluene = 5/4 to thereby obtain 3.5 mg of 2,8-di(2-(4-n-heptylphenyl)ethyl)anthra[1,2-b:5,6-b']dithiophene (Compound 12) as a yellow solid (yield: 7%). ¹H NMR (CDCl₃): δ = 8.59 (s, 2H), 7.85 (d, J = 9.0 Hz, 2H), 7.69 (d, J = 8.8 Hz, 2H), 7.19 (d, J = 7.9 Hz, 2H), 7.15 (s, 2H), 7.13 (d, J = 8.0 Hz, 2H), 3.32 (t, J = 7.6 Hz, 4H), 3.11 (t, J = 8.5 Hz, 4H), 2.59 (t, J = 7.6 Hz, 4H), 1.61 (m, 4H), 1.29 (m, 16H), 0.89 (t, J = 6.5 Hz, 6H).

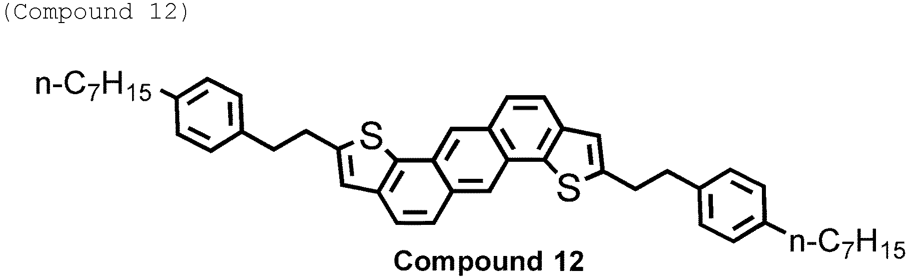

### Example 15: Synthesis of 2-(2-(4-n-heptylphenyl)ethyl)anthra[1,2-b:5,6-b']dithiophene (Compound 18) (Step C2)

Other components obtained by silica gel column chromatography in Example 14 were purified by recrystallization from hexane to thereby obtain 3.5 mg of 2-(2-(4-n-heptylphenyl)ethyl)anthra[1,2-b:5,6-b']dithiophene (Compound 18) as a yellow solid (yield: 10%).

¹H NMR (CDCl₃): δ = 8.70 (s, 1H), 8.62 (s, 1H), 7.90 (d, J = 8.5 Hz, 1H), 7.88 (d, J = 8.0 Hz, 1H), 7.82 (d, J = 8.9 Hz, 1H), 7.71 (d, J = 8.9 Hz, 1H), 7.54 (d, J = 5.4 Hz, 1H), 7.49 (d, J = 5.1 Hz, 1H), 7.19 (d, J = 8.0 Hz, 2H), 7.16 (s, 1H), 7.13 (d, J = 8.2 Hz, 2H), 3.32 (t, J = 7.7 Hz, 2H), 3.12 (t, J = 8.4 Hz, 2H), 2.59 (t, J = 7.5 Hz, 2H), 1.61 (m, 4H), 1.29 (m, 8H), 0.89 (t, J = 6.8 Hz, 3H).

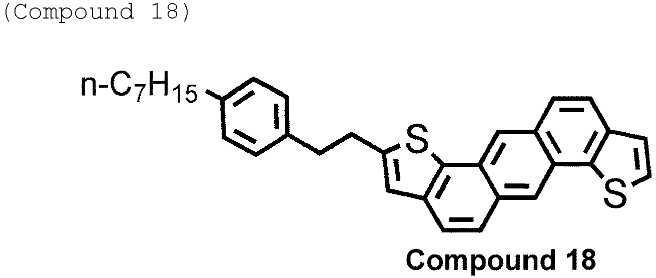

### Synthesis example 24: Synthesis of bromodithienobiphenylene derivative (Compound 21) (Step A3)

Under a nitrogen atmosphere, in a 50 mL Schlenk reaction vessel, 126 mg (0.476 mmol) of a dithienobiphenylene derivative synthesized according to the method described in Japanese Unexamined Patent Application, Publication No. 2018-174322 (Compound 2 in the above-mentioned patent publication) and 15 mL of THF (FUJIFILM Wako Pure Chemical Corporation, dehydrated grade) was added.

The resulting mixture was cooled to -78°C, added with 0.30 mL (0.480 mmol) of 1.6 M n-butyl lithium (Tokyo Chemical Industry Co., Ltd.), stirred at -78°C for 15 min, and then stirred at room temperature for 30 min.

The resultant was cooled to -78°C, added with 6 mL of a solution of 186 mg (0.571 mmol) of 1,2-dibromotetrachloroethane in THF (FUJIFILM Wako Pure Chemical Corporation, dehydrated grade), and stirred while heating to room temperature.

After cooling on ice, water was added to stop the reaction and toluene was added to separate phases.

The resulting organic phase was washed with water, dried over anhydrous sodium sulfate, and concentrated under reduced pressure.

The resulting residue was purified by silica gel column chromatography (solvent; hexane) and purified by recrystallization from hexane to thereby obtain 85.1 mg of a bromodithienobiphenylene derivative (Compound 21) as a yellow solid (yield: 47%).

¹H NMR (CDCl₃): δ = 7.23 (d, J = 7.1 Hz, 1H), 7.21 (d, J = 5.4 Hz, 1H), 7.05 (d, J = 5.8 Hz, 2H), 7.04 (s, 1H), 6.71 (d, J = 7.5 Hz, 1H), 6.67 (d, J = 7.3 Hz, 1H).

### Example 16: Synthesis of 2-(2-phenylethyl)dithienobiphenylene (Compound 13) (Step C3)

Under a nitrogen atmosphere, in a 50 mL Schlenk tube, 118 mg (0.866 mmol) of zinc chloride (FUJIFILM Wako Pure Chemical Corporation) and 2 mL of THF (FUJIFILM Wako Pure Chemical Corporation, dehydrated grade) was added and the resultant was stirred while cooling on ice.

While cooling on ice, the resultant was added with 1.90 mL (0.760 mmol) of the 0.4 M solution of 2-phenylethylmagnesium bromide in THF synthesized in Synthesis example 2 and stirred at 0°C for 30 min and at room temperature for 14 hours to thereby prepare a zinc reagent solution.

On the other hand, in a 50 mL Schlenk tube, 36.2 mg (0.105 mmol) of bromodithienobiphenylene derivative synthesized in Synthesis example 24, 10.1 mg (0.0138 mmol) of [1,1'-bis(diphenylphosphino)ferrocene]dichloropalladium (II) (Sigma-Aldrich Co. LLC), and 5 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 5 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane).

The resulting solid was rinsed with methanol to thereby obtain 15.1 mg of 2-(2-phenylethyl)dithienobiphenylene (Compound 13) as a yellow solid (yield: 39%).

¹H NMR (CDCl₃): δ = 7.30 (m, 2H), 7.21-7.18 (m, 5H), 7.04 (d, J = 5.6 Hz, 1H), 7.01 (d, J = 7.5 Hz, 1H), 6.71 (d, J = 7.3 Hz, 1H), 6.69 (s, 1H), 6.66 (d, J = 7.3 Hz, 1H), 3.12 (t, J = 6.4 Hz, 2H), 3.03 (t, J = 6.4 Hz, 2H).

Melting point: 161°C

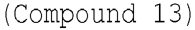

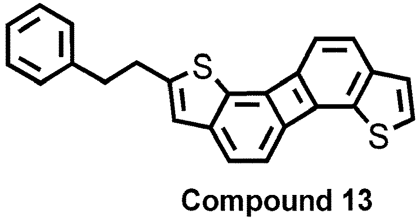

### Synthesis example 25: Synthesis of 1-(2-bromoethyl)-4-methylbenzene

Under a nitrogen atmosphere, in a 100 mL two-neck flask, 5.36 g (39.3 mmol) of 2-(p-tolyl)ethanol (Tokyo Chemical Industry Co., Ltd.) and 80 mL of dichloromethane (FUJIFILM Wako Pure Chemical Corporation, dehydrated grade) was added.

This solution was cooled on ice, added with 4.00 mL (42.1 mmol) of phosphorus tribromide, stirred for 30 min, and then stirred at room temperature for 21 hours.

The resulting reaction solution was poured into ice, neutralized with a saturated sodium hydrogen carbonate aqueous solution, and then added with dichloromethane to separate phases.

The resulting organic phase was washed with a saturated sodium hydrogen carbonate aqueous solution and water, dried over anhydrous sodium sulfate, and concentrated under reduced pressure.

The resulting residue was purified by silica gel column chromatography (solvent; dichloromethane) to thereby obtain 4.05 g of 1-(2-bromoethyl)-4-methylbenzene as colorless liquid (yield: 52%).

¹H NMR (CDCl₃): δ = 7.15 (d, J = 8.2 Hz, 2H), 7.11 (d, J = 8.2 Hz, 2H), 3.56 (t, J = 7.7 Hz, 2H), 3.14 (t, J = 7.7 Hz, 2H), 2.35 (s, 3H).

### Synthesis example 26: Synthesis of 2-(4-methylphenyl)ethylmagnesium bromide (Step B1)

Under a nitrogen atmosphere, in a 50 mL two-neck flask, 228 mg (9.36 mmol) of magnesium (FUJIFILM Wako Pure Chemical Corporation, turnings) was added and the resultant was stirred under vacuum for 1 hour.

Under a nitrogen atmosphere, the resultant was added with 20 mL of a solution of 1.60 g (8.02 mmol) of 1-(2-bromoethyl)-4-methylbenzene synthesized in Synthesis example 25 in THF (FUJIFILM Wako Pure Chemical Corporation, dehydrated grade) and stirred at room temperature for 90 min.

A solid was filtered off to thereby obtain a 0.4 M solution of 2-(4-metylphenyl)ethylmagnesium bromide in THF.

### Example 17: Synthesis of 2-(2-(4-methylphenyl)ethyl)dithienobiphenylene (Compound 14) (Step C3)

Under a nitrogen atmosphere, in a 50 mL Schlenk tube, 114 mg (0.836 mmol) of zinc chloride (FUJIFILM Wako Pure Chemical Corporation) and 2 mL of THF (FUJIFILM Wako Pure Chemical Corporation, dehydrated grade) was added and the resultant was stirred while cooling on ice.

While cooling on ice, the resultant was added with 1.80 mL (0.720 mmol) of the 0.4 M solution of 2-(4-methylphenyl)ethylmagnesium bromide in THF synthesized in Synthesis example 26 and stirred at 0°C for 15 min and at room temperature for 14 hours to thereby prepare a zinc reagent solution.

On the other hand, in a 50 mL Schlenk tube, 24.4 mg (0.0711 mmol) of the bromodithienobiphenylene derivative synthesized in Synthesis example 24, 9.00 mg (0.0123 mmol) of [1,1'-bis(diphenylphosphino)ferrocene]dichloropalladium (II) (Sigma-Aldrich Co. LLC), and 7 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 5 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane).

The resulting solid was rinsed with methanol to thereby obtain 4.9 mg of 2-(2-(4-methylphenyl)ethyl)dithienobiphenylene (Compound 14) as a yellow solid (yield: 18%).

¹H NMR (CDCl₃): δ = 7.20 (d, J = 8.3 Hz, 1H), 7.19 (d, J = 5.4 Hz, 1H), 7.04 (d, J = 5.5 Hz, 1H), 7.01 (d, J = 7.4 Hz, 1H), 6.71 (d, J = 7.3 Hz, 1H), 6.69 (s, 1H), 6.66 (d, J = 7.4 Hz, 1H), 3.09 (t, J = 7.0 Hz, 2H), 2.99 (t, J = 7.8 Hz, 2H), 2.33 (s, 3H).

Melting point: 151°C

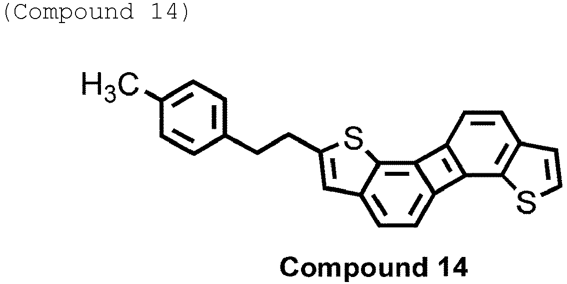

### Synthesis example 27: Synthesis of 2-bromoanthra[1,2-b:5,6-b']dithiophene (Compound 22) (Step A-4)

Under a nitrogen atmosphere, in a 100 mL Schlenk reaction vessel, 481 mg (1.66 mmol) of anthra[1,2-b:5,6-b']dithiophene (Compound 19) synthesized in Synthesis example 22 and 57 mL of THF (FUJIFILM Wako Pure Chemical Corporation, dehydrated grade) was added.

The resulting mixture was cooled to -78°C, added with 1.05 mL (1.68 mmol) of 1.6 M n-butyl lithium (Tokyo Chemical Industry Co., Ltd.), and stirred at -78°C for 2 hours.

The resultant was cooled to -78°C, added with 24 mL of a solution of 634 mg (1.95 mmol) of 1,2-dibromotetrachloroethane in THF (FUJIFILM Wako Pure Chemical Corporation, dehydrated grade), and stirred while heating to room temperature.

After cooling on ice, water was added to stop the reaction and toluene was added to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 2/1) and purified by recrystallization from toluene to thereby obtain 372 mg of 2-bromoanthra[1,2-b:5,6-b']dithiophene (Compound 22) as a yellow solid (yield: 51%).

¹H NMR (CDCl₃): δ = 8.70 (s, 1H), 8.56 (s, 1H), 7.90-7.86 (m, 3H), 7.71 (d, J = 8.7 Hz, 1H), 7.57 (d, J = 5.0 Hz, 1H), 7.50 (d, J = 5.4 Hz, 1H), 7.48 (s, 1H).

### Example 18: Synthesis of 2-(2-(4-methylphenyl)ethyl)anthra[1,2-b:5,6-b']dithiophene (Compound 16) (Step C4)

Under a nitrogen atmosphere, in a 50 mL Schlenk tube, 207 mg (1.51 mmol) of zinc chloride (FUJIFILM Wako Pure Chemical Corporation) and 4 mL of THF (FUJIFILM Wako Pure Chemical Corporation, dehydrated grade) was added and the resultant was stirred while cooling on ice.

While cooling on ice, the resultant was added with 2.50 mL (1.00 mmol) of the 0.4 M solution of 2-(4-methylphenyl)ethylmagnesium bromide in THF synthesized in Synthesis example 26 and stirred at 0°C for 30 min and at room temperature for 14 hours to thereby prepare a zinc reagent solution.

On the other hand, in a 50 mL Schlenk tube, 50.1 mg (0.136 mmol) of 2-bromoanthra[1,2-b:5,6-b']dithiophene (Compound 22) synthesized in Synthesis example 27 and 11.0 mg (0.0150 mmol) of [1,1'-bis(diphenylphosphino)ferrocene]dichloropalladium (II) (Sigma-Aldrich Co. LLC), and 7 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 24 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 10/1).

The resulting solid was purified by recrystallization from hexane/toluene = 5/1 to thereby obtain 13.8 mg of 2-(2-(4-methylphenyl)ethyl)anthra[1,2-b:5,6-b']dithiophene (Compound 16) as a yellow solid (yield: 30%).

¹H NMR (CDCl₃): δ = 8.70 (s, 1H), 8.62 (s, 1H), 7.90 (d, J = 8.8 Hz, 1H), 7.87 (d, J = 8.8 Hz, 1H), 7.82 (d, J = 8.7 Hz, 1H), 7.71 (d, J = 8.8 Hz, 1H), 7.54 (d, J = 5.4 Hz, 1H), 7.49 (d, J = 5.1 Hz, 1H), 7.18-7.12 (m, 5H), 3.32 (t, J = 7.7 Hz, 2H), 3.12 (t, J = 7.4 Hz, 2H), 2.35 (s, 3H).

Melting point: 190°C

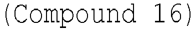

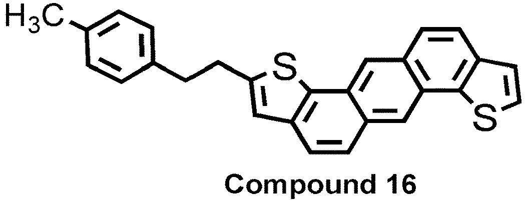

### Synthesis example 28: Synthesis of 1,5-bis(trimethylsilylethynyl)-2,6-difluorobiphenylene (Step D1)

Under a nitrogen atmosphere, in a 50 mL Schlenk reaction vessel, 260 mg (0.591 mmol) of 2,6-difluoro-1,5-diiodebiphenylene synthesized according to the method described in Japanese Unexamined Patent Application, Publication No. 2018-174322, 10.3 mg (0.0146 mmol) of bis(triphenylphosphine)dichloropalladium (FUJIFILM Wako Pure Chemical Corporation), 5.2 mg (0.0273 mmol) of copper iodide (I) (FUJIFILM Wako Pure Chemical Corporation), 6 mL of toluene, and 6 mL of trimethylamine was added.

The resulting mixture was added with 174 mg (1.77 mmol) of trimethylsilylacetylene (Tokyo Chemical Industry Co., Ltd.) and stirred at 30°C for 6 hours.

The resulting reaction mixture was quenched with the addition of water.

The resultant was extracted with toluene and the organic phase was washed with water, dried over anhydrous sodium sulfate, and concentrated under reduced pressure.

The resulting residue was purified by silica gel column chromatography (solvent; hexane:toluene = 1/0 to 20/1) to thereby obtain 188 mg of a light yellow solid of object (yield: 84%).

¹H NMR (CDCl₃): δ = 6.57 (dd, J = 7.3 Hz, 4.0 Hz, 2H), 6.42 (dd, J = 11.0 Hz, 7.4 Hz, 2H), 0.26 (s, 18H).

### Synthesis example 29: Synthesis of biphenyleno[2,1-b:6,5-b']dithiophene (Step E1)

Under a nitrogen atmosphere, in a 100 mL Schlenk reaction vessel, 133 mg (0.349 mmol) of 1,5-bis(trimethylsilylethynyl)-2,6-difluorobiphenylene synthesized in Synthesis example 28, 301 mg (1.25 mmol) of sodium sulfide nonahydrate (Sigma-Aldrich Co. LLC), and 8 mL of DMSO (FUJIFILM Wako Pure Chemical Corporation) was added.

The mixture was heated to 90°C and stirred for 4 hours.

The resulting reaction mixture was cooled to 0°C and then added with water and toluene.

The resultant was extracted with hot toluene to separate phases, and the resultant organic phase was washed with water. The organic phase was concentrated under reduced pressure to thereby obtain 78 mg of biphenyleno[2,1-b:6,5-b']dithiophene as a yellow solid (yield: 84%).

¹H NMR (CDCl₃): δ = 7.31 (d, J = 5.7 Hz, 2H), 7.18 (d, J = 7.6H), 6.94 (d, J = 5.6 Hz, 2H), 6.65 (d, J = 7.4,2H).

### Synthesis example 30: Synthesis of 2,7-dibromobiphenyleno[2,1-b:6,5-b']dithiophene (Step F1)

Under a nitrogen atmosphere, in a 100 mL Schlenk reaction vessel, 105 mg (0.397 mmol) of biphenyleno[2,1-b:6,5-b']dithiophene synthesized in Synthesis example 29 and 9 mL of THF (FUJIFILM Wako Pure Chemical Corporation, dehydrated grade) was added.

The resulting mixture was cooled to -78°C, added with 0.94 mL (1.50 mmol) of 1.6 M n-butyl lithium (Tokyo Chemical Industry Co., Ltd.), and stirred at -78°C for 10 min.

After stirring at 20°C for 25 min, the resultant was cooled to -78°C, added with 3 mL of a solution of 555 mg (1.70 mmol) of 1,2-dibromotetrachloroethane in THF (FUJIFILM Wako Pure Chemical Corporation, dehydrated grade), and stirred while heating to room temperature.

After cooling on ice, water was added to stop the reaction. The deposited solid was added with hexane and filtered through filter paper.

The solid was washed with water, methanol, and hexane, and the resulting residue was dried to thereby obtain 86 mg of 2,7-dibromobiphenyleno[2,1-b:6,5-b']dithiophene as a red-orange solid (yield: 51%).

¹H NMR (CDCl₃, 58°C): δ = 7.02 (d, J = 7.7 Hz, 2H), 6.96 (s, 2H), δ = 6.58 (d, J = 7.7 Hz, 2H).

### Example 19: Synthesis of 2,7-di(2-phenylethyl)dithienobiphenylene (Compound 9) (Step G1)

Under a nitrogen atmosphere, in a 50 mL Schlenk tube, 150 mg (1.01 mmol) of zinc chloride (FUJIFILM Wako Pure Chemical Corporation) and 7 mL of THF (FUJIFILM Wako Pure Chemical Corporation, dehydrated grade) was added and the resultant was stirred while cooling on ice.

While cooling on ice, the resultant was added with 1.4 mL (0.56 mmol) of the 0.4 M solution of 2-phenylethylmagnesium bromide in THF synthesized in Synthesis example 2 and stirred at room temperature for 1 hours to thereby prepare a zinc reagent solution.

The resultant was added with 45.4 mg (0.107 mmol) of 2,7-dibromobiphenyleno[2,1-b:6,5-b']dithiophene synthesized in Synthesis example 30 and 3.30 mg (0.00451 mmol) of [1,1'-bis(diphenylphosphino)ferrocene]dichloropalladium (II) (Sigma-Aldrich Co. LLC).

This mixture was stirred at 40°C for 13 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 3/1).

The resulting solid was purified by recrystallization from heptane/toluene = 1/2 to thereby obtain 26 mg of an aromatic compound (Compound 9) as a yellow solid (yield: 51%).

¹H NMR (CDCl₃): δ = 7.34-7.20 (m, 10H), 7.04 (d, J = 7.6 Hz, 2H), 6.61 (s, 2H), 6.63 (d, J = 7.4 Hz, 2H), δ = 3.11 (t, J = 8.64 Hz, 4H), δ = 3.03 (t, J = 8.4 Hz, 4H).

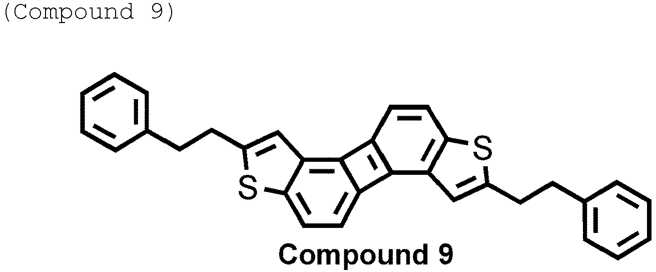

### Example 20 (Production of solution for forming organic semiconductor layer)

A solution for forming an organic semiconductor layer was prepared in the same manner as in Example 2, except that 0.44 mg of 2,7-di(2-(4-propylphenyl)ethyl)dithienobiphenylene (Compound 4) synthesized in Example 5 was used.

The solution for forming an organic semiconductor layer was kept in a solution state after 10 hours at 25°C (concentration of Compound 4: 0.10% by weight), confirming that this was a compound suitable for film formation by drop casting and inkjet.

### Example 21 (Production of organic semiconductor layer and organic thin film transistor)

The solution for forming an organic semiconductor layer obtained in Example 20 and the materials and the film formation methods for components shown in Example 3 were used to produce a bottom gate-bottom contact p-type organic thin film transistor.

As a result of evaluating the transfer characteristics of the transistor element, carrier mobility for holes was 1.86 cm²/V· sec and a current ON/OFF ratio was 1.1 × 10⁶.

Furthermore, this organic thin film transistor was annealed at 130°C for 10 min and then measured for an electric physical property.

Carrier mobility for holes was 1.70 cm²/V·sec and a current ON/OFF ratio was 1.0 x 10⁶, and there was almost no degradation in performance due to heat treatment.

### Example 22 (Production of organic semiconductor layer and organic thin film transistor)

In the air, in a 10 mL sample tube, 0.87 mg of 2,7-di(2-(4-propylphenyl)ethyl)dithienobiphenylene (Compound 4) synthesized in Example 5 and 440 mg of anisole (Sigma-Aldrich Co. LLC) was added and the resultant was heated at 60°C to thereby prepare a 0.20% by weight solution of Compound 4.

The resulting solution and the materials and the film formation methods for components shown in Example 3 were used to produce a bottom gate-bottom contact p-type organic thin film transistor.

As a result of evaluating the transfer characteristics of the transistor element, carrier mobility for holes was 2.48 cm²/V· sec and a current ON/OFF ratio was 1.6 x 10⁶.

Furthermore, this organic thin film transistor was annealed at 130°C for 10 min and then measured for an electric physical property.

Carrier mobility for holes was 2.40 cm²/V·sec and a current ON/OFF ratio was 1.4 × 10⁶, and there was almost no degradation in performance due to heat treatment.

### Example 23 (Production of solution for forming organic semiconductor layer)

A solution for forming an organic semiconductor layer was prepared in the same manner as in Example 2, except that 1.74 mg of 2,7-di(2-(4-butylphenyl)ethyl)dithienobiphenylene (Compound 5) synthesized in Example 6 was used.

The solution for forming an organic semiconductor layer was kept in a solution state after 10 hours at 25°C (concentration of Compound 5: 0.40% by weight), confirming that this was a compound suitable for film formation by drop casting and inkjet.

### Example 24 (Production of solution for forming organic semiconductor layer)

A solution for forming an organic semiconductor layer was prepared in the same manner as in Example 2, except that 0.87 mg of 2,7-di(2-(4-heptylphenyl)ethyl)dithienobiphenylene (Compound 6) synthesized in Example 7 was used.

The solution for forming an organic semiconductor layer was kept in a solution state after 10 hours at 25°C (concentration of Compound 6: 0.20% by weight), confirming that this was a compound suitable for film formation by drop casting and inkjet.

### Example 25 (Production of organic semiconductor layer and organic thin film transistor)

The solution for forming an organic semiconductor layer obtained in Example 24 and the materials and the film formation methods for components shown in Example 3 were used to produce a bottom gate-bottom contact p-type organic thin film transistor.

As a result of evaluating the transfer characteristics of the transistor element, carrier mobility for holes was 1.19 cm²/V· sec and a current ON/OFF ratio was 1.1 × 10⁶.

Furthermore, this organic thin film transistor was annealed at 130°C for 10 min and then measured for an electric physical property.

Carrier mobility for holes was 1.15 cm²/V·sec and a current ON/OFF ratio was 1.0 x 10⁶, and there was almost no degradation in performance due to heat treatment.

### Example 26 (Production of solution for forming organic semiconductor layer)

A solution for forming an organic semiconductor layer was prepared in the same manner as in Example 2, except that 0.87 mg of 2,8-di(2-phenylethyl)anthra[1,2-b:5,6-b']dithiophene (Compound 10) synthesized in Example 10 was used.

The solution for forming an organic semiconductor layer was kept in a solution state after 10 hours at 25°C (concentration of Compound 10: 0.20% by weight), confirming that this was a compound suitable for film formation by drop casting and inkjet.

### Example 27 (Production of solution for forming organic semiconductor layer)

A solution for forming an organic semiconductor layer was prepared in the same manner as in Example 2, except that 4.35 mg of 2-(2-phenylethyl)dithienobiphenylene (Compound 13) synthesized in Example 16 was used.

The solution for forming an organic semiconductor layer was kept in a solution state after 10 hours at 25°C (concentration of Compound 13: 1.00% by weight), confirming that this was a compound suitable for film formation by drop casting and inkjet.

### Example 28 (Production of solution for forming organic semiconductor layer)

A solution for forming an organic semiconductor layer was prepared in the same manner as in Example 2, except that 4.35 mg of 2-(2-(4-methylphenyl)ethyl)dithienobiphenylene (Compound 14) synthesized in Example 17 was used.

The solution for forming an organic semiconductor layer was kept in a solution state after 10 hours at 25°C (concentration of Compound 14: 1.00% by weight), confirming that this was a compound suitable for film formation by drop casting and inkjet.

### Example 29 (Production of solution for forming organic semiconductor layer)

A solution for forming an organic semiconductor layer was prepared in the same manner as in Example 2, except that 1.74 mg of 2-(2-phenylethyl)anthra[1,2-b:5,6-b']dithiophene (Compound 15) synthesized in Example 11 was used.

The solution for forming an organic semiconductor layer was kept in a solution state after 10 hours at 25°C (concentration of Compound 15: 0.40% by weight), confirming that this was a compound suitable for film formation by drop casting and inkjet.

### Example 30 (Production of solution for forming organic semiconductor layer)

A solution for forming an organic semiconductor layer was prepared in the same manner as in Example 2, except that 2.18 mg of 2-(2-(4-methylphenyl)ethyl)anthra[1,2-b:5,6-b']dithiophene (Compound 16) synthesized in Example 18 was used.

The solution for forming an organic semiconductor layer was kept in a solution state after 10 hours at 25°C (concentration of Compound 16: 0.50% by weight), confirming that this was a compound suitable for film formation by drop casting and inkjet.

Example 31 (Production of solution for forming organic semiconductor layer)

A solution for forming an organic semiconductor layer was prepared in the same manner as in Example 2, except that 4.35 mg of 2-(2-(4-n-butylphenyl)ethyl)anthra[1,2-b:5,6-b']dithiophene (Compound 17) synthesized in Example 13 was used.

The solution for forming an organic semiconductor layer was kept in a solution state after 10 hours at 25°C (concentration of Compound 17: 1.00% by weight), confirming that this was a compound suitable for film formation by drop casting and inkjet.

### Example 32 (Production of organic semiconductor layer and organic thin film transistor)

A solution for forming an organic semiconductor layer (concentration of Compound 17: 0.20% by weight) was prepared in the same manner as in Example 2, except that 0.87 mg of 2-(2-(4-n-butylphenyl)ethyl)anthra[1,2-b:5,6-b']dithiophene (Compound 17) synthesized in Example 13 was used.

The resulting solution for forming an organic semiconductor layer and the materials and the film formation methods for components shown in Example 3 were used to produce a bottom gate-bottom contact p-type organic thin film transistor.

As a result of evaluating the transfer characteristics of the transistor element, carrier mobility for holes was 1.13 cm²/V· sec and a current ON/OFF ratio was 8.1 × 10⁷.

Furthermore, this organic thin film transistor was annealed at 130°C for 10 min and then measured for an electric physical property.

Carrier mobility for holes was 1.10 cm²/V·sec and a current ON/OFF ratio was 8.0 × 10⁷, and there was almost no degradation in performance due to heat treatment.

### Example 33 (Production of solution for forming organic semiconductor layer)

A solution for forming an organic semiconductor layer was prepared in the same manner as in Example 2, except that 0.87 mg of 2-(2-(4-n-heptylphenyl)ethyl)anthra[1,2-b:5,6-b']dithiophene (Compound 18) synthesized in Example 15 was used.

The solution for forming an organic semiconductor layer was kept in a solution state after 10 hours at 25°C (concentration of Compound 18: 0.20% by weight), confirming that this was a compound suitable for film formation by drop casting and inkjet.

### Synthesis example 31: Synthesis of 4-propoxybenzeneethanol

Under a nitrogen atmosphere, in a 300 mL Schlenk reaction vessel, 4.51 g (21.0 mmol) of 1-bromo-4-propoxybenzene (Tokyo Chemical Industry Co., Ltd.) and 80 mL of THF (FUJIFILM Wako Pure Chemical Corporation, dehydrated grade) was added.

The resulting mixture was cooled to -78°C, added with 22.0 mL (35.0 mmol) of 1.6 M n-butyl lithium (Tokyo Chemical Industry Co., Ltd.), and stirred at -78°C for 2 hours.

The resultant was added with 25.0 mL (30.0 mmol) of a 1.2 M solution of ethylene oxide in THF (Tokyo Chemical Industry Co., Ltd.) at -78°C and stirred while heating to room temperature.

After 1 M hydrochloric acid was added, diethyl ether was added to separate phases.

The resulting organic phase was washed with water, dried over anhydrous sodium sulfate, and concentrated under reduced pressure.

The resulting residue was purified by silica gel column chromatography (solvent; dichloromethane, ethyl acetate) to thereby obtain 2.83 g of 4-propoxybenzeneethanol as colorless liquid (yield: 64%).

¹H NMR (CDCl₃): δ = 7.13 (dd, J = 1.9 Hz, 6.4 Hz, 2H), 6.85 (dd, J = 2.1 Hz, 6.4 Hz, 2H), 3.91 (t, J = 6.7 Hz, 2H), 3.82 (t, J = 6.7 Hz, 2H), 2.81 (t, J = 6.6 Hz, 2H), 1.81 (tq, J = 6.6 Hz, 7.4 Hz, 2H), 1.04 (t, J = 7.4 Hz, 3H).

### Synthesis example 32: Synthesis of 1-(2-bromoethyl)-4-propoxybenzene

Under a nitrogen atmosphere, in a 100 mL eggplant flask, 2.83 g (15.7 mmol) of 4-propoxybenzeneethanol synthesized in Synthesis example 31 and 20 mL of toluene (FUJIFILM Wako Pure Chemical Corporation, dehydrated grade) was added.

The resultant was added with 0.80 mL (8.4 mmol) of phosphorus tribromide at room temperature, stirred for 20 min, and then stirred at 100°C for 4 hours.

The resulting reaction solution was poured into ice, neutralized with a saturated sodium hydrogen carbonate aqueous solution, and then added with dichloromethane to separate phases.

The resulting organic phase was washed with a saturated sodium hydrogen carbonate aqueous solution and water, dried over anhydrous sodium sulfate, and concentrated under reduced pressure.

The resulting residue was purified by silica gel column chromatography (solvent; dichloromethane) to thereby obtain 3.40 g of 1-(2-bromoethyl)-4-propoxybenzene as colorless liquid (yield: 84%).

¹H NMR (CDCl₃): δ = 7.11 (d, J = 8.4 Hz, 2H), 6.86 (d, J = 8.6 Hz, 2H), 3.91 (t, J = 6.6 Hz, 2H), 3.53 (t, J = 7.4 Hz, 2H), 3.10 (t, J = 7.6 Hz, 2H), 1.82 (tq, J = 6.8 Hz, 7.4 Hz, 2H), 1.04 (t, J = 7.4 Hz, 3H).

### Synthesis example 33: Synthesis of 2-(4-propoxyphenyl)ethylmagnesium bromide (Step B1)

Under a nitrogen atmosphere, in a 20 mL two-neck flask, 172 mg (7.06 mmol) of magnesium (FUJIFILM Wako Pure Chemical Corporation, turnings) was added and the resultant was stirred under vacuum for 1 hour.

Under a nitrogen atmosphere, the resultant was added with 15 mL of a solution of 1.46 g (6.00 mmol) of 1-(2-bromoethyl)-4-propoxybenzene synthesized in Synthesis example 32 in THF (FUJIFILM Wako Pure Chemical Corporation, dehydrated grade) and stirred at room temperature for 1 hour.

A solid was filtered off to thereby obtain a 0.4 M solution of 2-(4-propoxyphenyl)ethylmagnesium bromide in THF.

### Example 34: Synthesis of 2-(2-(4-propoxyphenyl)ethyl)anthra[1,2-b:5,6-b']dithiophene (Compound 23) (Step C4)

Under a nitrogen atmosphere, in a 50 mL Schlenk tube, 149 mg (1.10 mmol) of zinc chloride (FUJIFILM Wako Pure Chemical Corporation) and 2 mL of THF (FUJIFILM Wako Pure Chemical Corporation, dehydrated grade) was added and the resultant was stirred while cooling on ice.

While cooling on ice, the resultant was added with 1.80 mL (0.72 mmol) of the 0.4 M solution of 2-(4-propoxyphenyl)ethylmagnesium bromide in THF synthesized in Synthesis example 33 and stirred at 0°C for 15 min and at room temperature for 15 hours to thereby prepare a zinc reagent solution.

On the other hand, in a 50 mL Schlenk tube, 50.6 mg (0.144 mmol) of 2-bromoanthra[1,2-b:5,6-b']dithiophene (Compound 22) synthesized in Synthesis example 27 and 6.6 mg (0.0090 mmol) of [1,1'-bis(diphenylphosphino)ferrocene]dichloropalladium (II) (Sigma-Aldrich Co. LLC), and 5 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 24 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 2/5).

The resulting solid was purified by recrystallization from hexane/toluene = 10/3 to thereby obtain 8.5 mg of 2-(2-(4-propoxyphenyl)ethyl)anthra[1,2-b:5,6-b']dithiophene (Compound 23) as a yellow solid (yield: 13%).

¹H NMR (CDCl₃): δ = 8.69 (s, 1H), 8.62 (s, 1H), 7.90 (d, J = 9.1 Hz, 1H), 7.87 (d, J = 9.2 Hz, 1H), 7.82 (d, J = 8.9 Hz, 1H), 7.71 (d, J = 8.8 Hz, 1H), 7.54 (d, J = 5.0 Hz, 1H), 7.49 (d, J = 5.1 Hz, 1H), 7.17 (d, J = 8.6 Hz, 1H), 7.14 (s, 1H), 6.86 (d, J = 8.7 Hz, 1H), 3.92 (t, J = 6.4 Hz, 2H), 3.30 (t, J = 7.6 Hz, 2H), 3.10 (t, J = 7.5 Hz, 2H), 1.81 (tq, J = 7.5 Hz, 7.6 Hz, 2H), 1.04 (t, J = 7.5 Hz, 3H).

Melting point: 181°C

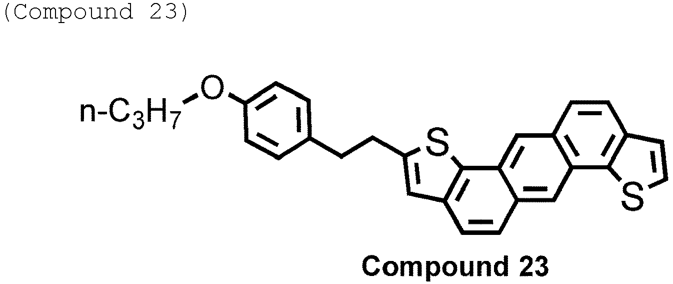

### Synthesis example 34: Synthesis of 2-(4-methoxyphenyl)ethylmagnesium bromide (Step B1)

Under a nitrogen atmosphere, in a 20 mL two-neck flask, 161 mg (6.61 mmol) of magnesium (FUJIFILM Wako Pure Chemical Corporation, turnings) was added and the resultant was stirred under vacuum for 1 hour.

Under a nitrogen atmosphere, the resultant was added with 15 mL of a solution of 1.29 g (6.00 mmol) of 4-methoxyphenetyl bromide (Sigma-Aldrich Co. LLC) in THF (FUJIFILM Wako Pure Chemical Corporation, dehydrated grade) and stirred at room temperature for 1 hour.

A solid was filtered off to thereby obtain a 0.4 M solution of 2-(4-methoxyphenyl)ethylmagnesium bromide in THF.

### Example 35: Synthesis of 2-(2-(4-methoxyphenyl)ethyl)anthra[1,2-b:5,6-b']dithiophene (Compound 24) (Step C4)

Under a nitrogen atmosphere, in a 50 mL Schlenk tube, 125 mg (0.914 mmol) of zinc chloride (FUJIFILM Wako Pure Chemical Corporation) and 2 mL of THF (FUJIFILM Wako Pure Chemical Corporation, dehydrated grade) was added and the resultant was stirred while cooling on ice.

While cooling on ice, the resultant was added with 1.60 mL (0.640 mmol) of the 0.4 M solution of 2-(4-methoxyphenyl)ethylmagnesium bromide in THF synthesized in Synthesis example 34 and stirred at 0°C for 15 min and at room temperature for 15 hours to thereby prepare a zinc reagent solution.

On the other hand, in a 50 mL Schlenk tube, 50.4 mg (0.136 mmol) of 2-bromoanthra[1,2-b:5,6-b']dithiophene (Compound 22) synthesized in Synthesis example 27 and 7.8 mg (0.011 mmol) of [1,1'-bis(diphenylphosphino)ferrocene]dichloropalladium (II) (Sigma-Aldrich Co. LLC), and 5 mL of THF (FUJIFILM Wako Pure Chemical Corporation, dehydrated grade) was added.

The resultant was added dropwise with the above-prepared zinc reagent solution via a TEFLON (registered trademark) cannular and stirred at room temperature for 24 hours.

The resulting reaction mixture was cooled on ice, added with 1 M hydrochloric acid, and then added with toluene to separate phases.

The resulting organic phase was washed with water, dried over anhydrous sodium sulfate, and concentrated under reduced pressure.

The resulting residue was purified by silica gel column chromatography (solvent; hexane:toluene = 1/0 to 2/5).

The resulting solid was purified by recrystallization from hexane/toluene = 3/2 to thereby obtain 17.8 mg of 2-(2-(4-methoxyphenyl)ethyl)anthra[1,2-b:5,6-b']dithiophene (Compound 24) as a yellow solid (yield: 31%).

¹H NMR (CDCl₃): δ = 8.70 (s, 1H), 8.62 (s, 1H), 7.90 (d, J = 9.2 Hz, 1H), 7.87 (d, J = 9.2 Hz, 1H), 7.82 (d, J = 8.7 Hz, 1H), 7.71 (d, J = 8.7 Hz, 1H), 7.54 (d, J = 5.3 Hz, 1H), 7.49 (d, J = 5.1 Hz, 1H), 7.18 (d, J = 8.6 Hz, 1H), 7.14 (s, 1H), 6.86 (d, J = 8.6 Hz, 1H), 3.81 (s, 3H), 3.30 (t, J = 7.5 Hz, 2H), 3.09 (t, J = 7.5 Hz, 2H).

Melting point: 231°C

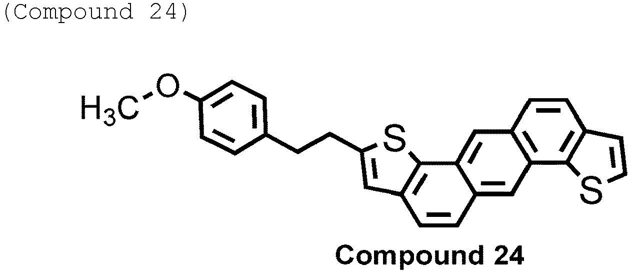

### Example 36 (Production of solution for forming organic semiconductor layer)

A solution for forming an organic semiconductor layer was prepared in the same manner as in Example 2, except that 4.35 mg of 2-(2-(4-propoxyphenyl)ethyl)anthra[1,2-b:5,6-b']dithiophene (Compound 23) synthesized in Example 34 was used.

The solution for forming an organic semiconductor layer was kept in a solution state after 10 hours at 25°C (concentration of Compound 23: 1.00% by weight), confirming that this was a compound suitable for film formation by drop casting and inkjet.

### Example 37 (Production of solution for forming organic semiconductor layer)

A solution for forming an organic semiconductor layer was prepared in the same manner as in Example 2, except that 0.44 mg of 2-(2-(4-methoxyphenyl)ethyl)anthra[1,2-b:5,6-b']dithiophene (Compound 24) synthesized in Example 35 was used.

The solution for forming an organic semiconductor layer was kept in a solution state after 10 hours at 25°C (concentration of Compound 24: 0.10% by weight), confirming that this was a compound suitable for film formation by drop casting and inkjet.

### Comparative Example 1

### (Production of solution for forming organic semiconductor layer)

In the air, in a 10 mL sample tube, .44 mg of 2,7-diphenyl[1]benzothieno[3,2-b][1]benzothiophene (Sigma-Aldrich Co. LLC) and 434 mg of toluene (FUJIFILM Wako Pure Chemical Corporation, pure grade) was added and the resultant was heated to 50°C, and allowed to cool at room temperature (25°C). As a result, it was confirmed that a solid was deposited and was a compound unsuitable for film formation by drop casting or inkjet due to low solubility.

### Comparative Example 2

### (Production of solution for forming organic semiconductor layer)

A solution for forming an organic semiconductor layer was prepared in the same manner as in Example 2 using 2,7-dioctyl[1]benzothieno[3,2-b][1]benzothiophene (Sigma-Aldrich Co. LLC) in a 10 mL sample tube in the air.

The solution for forming an organic semiconductor layer was kept in a solution state after 10 hours at 25°C (0.20% by weight), confirming that this was a compound suitable for film formation by drop casting and inkjet.

### (Production of organic semiconductor layer and organic thin film transistor)

A bottom gate-bottom contact p-type organic thin film transistor was produced by forming a thin film of 2,7-dioctyl[1]benzothieno[3,2-b][1]benzothiophene in the same manner as in Example 4 using the solution for forming an organic semiconductor layer.

As a result of evaluating the transfer characteristics of the transistor element, carrier mobility for holes was 1.02 cm²/V. sec and a current ON/OFF ratio was 3.0 × 10⁵.

Furthermore, this organic thin film transistor was annealed at 130°C for 10 min and then measured for an electric physical property.

As a result, transistor operation was not achieved, and significant degradation in performance due to heat treatment was observed.

Microscopic observation confirmed that the organic semiconductor layer was destroyed by heating.

### Comparative Example 3

### (Production of solution for forming organic semiconductor layer)

A solution for forming an organic semiconductor layer was prepared in the same manner as in Example 2 using 2,7-di(normal octyl)dithienobiphenylene described in Japanese Unexamined Patent Application, Publication No. 2018-174322 (Compound 3 in the above-mentioned patent publication) in a 10 mL sample tube in the air.

The solution for forming an organic semiconductor layer was kept in a solution state after 10 hours at 25°C (0.20% by weight), confirming that this was a compound suitable for film formation by drop casting and inkjet.

### (Production of organic semiconductor layer and organic thin film transistor)

The solution for forming an organic semiconductor layer and the materials and the film formation methods for components shown in Example 3 were used to produce a top gate-bottom contact p-type organic thin film transistor.

As a result of evaluating the transfer characteristics of the transistor element, carrier mobility for holes was 0.60 cm²/V. sec and a current ON/OFF ratio was 3.0 × 10⁶.

### Comparative Example 4

### (Production of solution for forming organic semiconductor layer)

A solution for forming an organic semiconductor layer was prepared in the same manner as in Example 2 using 2,8-dioctylanthra[1,2-b:5,6-b']dithiophene synthesized in Synthesis example 6 in a 10 mL sample tube in the air.

The solution for forming an organic semiconductor layer was kept in a solution state after 10 hours at 25°C (0.20% by weight), confirming that this was a compound suitable for film formation by drop casting and inkjet.

### (Production of organic semiconductor layer and organic thin film transistor)

The solution for forming an organic semiconductor layer and the materials and the film formation methods for components shown in Example 3 were used.

However, a thin film was not formed and a top gate-bottom contact p-type organic thin film transistor was not able to be produced.

### INDUSTRIAL APPLICABILITY

An aromatic compound of the present invention is expected to be applied as a semiconductor device material typified by an organic thin film transistor since it provides high carrier mobility and has excellent heat resistance and solubility.

### EXPLANATION OF REFERENCE NUMERALS

(A): Bottom gate-top contact organic thin film transistor
(B): Bottom gate-bottom contact organic thin film transistor
(C): Top gate-top contact organic thin film transistor
(D): Top gate-bottom contact organic thin film transistor
1: Organic semiconductor layer
2: Substrate
3: Gate electrode
4: Gate insulating layer
5: Source electrode
6: Drain electrode

## Claims

1. An aromatic compound represented by Formula (1-I) or Formula (1-II) below: 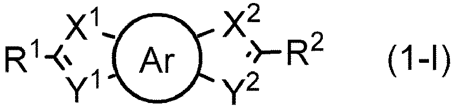 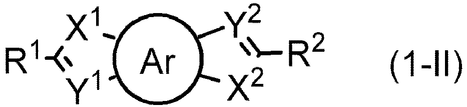
wherein Ar denotes a monocyclic ring or 2 to 6 fused ring;
X¹ and X² each independently denote one of the group consisting of an oxygen atom, a sulfur atom, a selenium atom, NR³, or CR⁴=CR⁵;
Y¹ and Y² each independently denote CR⁶ or a nitrogen atom; and
R¹ to R⁶ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or a group represented by Formula (2) below; and at least one of R¹ to R⁶ is a group represented by Formula (2) below; 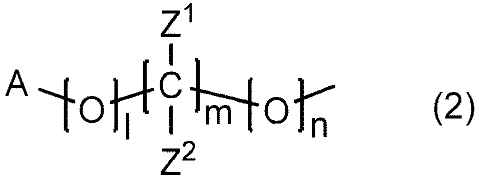
wherein A denotes one of the group consisting of an alkenyl group having 2 to 20 carbon atoms, an alkynyl group having 2 to 20 carbon atoms, or an aryl group having 4 to 26 carbon atoms;
1 and n each independently denote 0 or 1; m denotes an integer of 1 to 20; and
Z¹ and Z² are the same or different at each occurrence and denote one of the group consisting of a hydrogen atom, a halogen atom, and an alkyl group having 1 to 20 carbon atoms.

2. The aromatic compound according to claim 1, wherein, among R¹ to R⁶, only one or both of R¹ and R² are the group represented by the above Formula (2).

3. The aromatic compound according to claim 1 or 2, wherein the aromatic compound represented by Formula (1-I) or Formula (1-II) is an aromatic compound represented by one of the group consisting of Formulae (3-1) to (3-6) below: 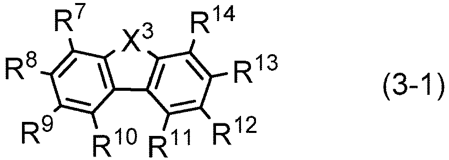 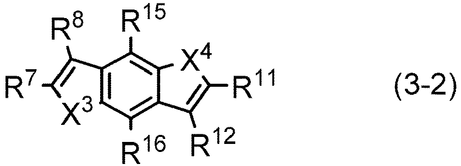 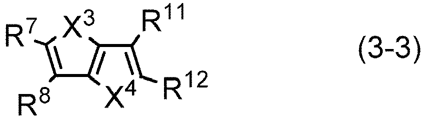 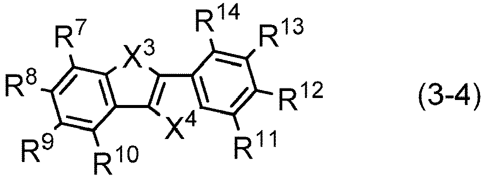 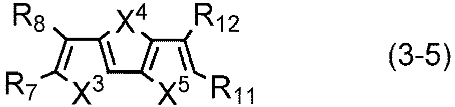 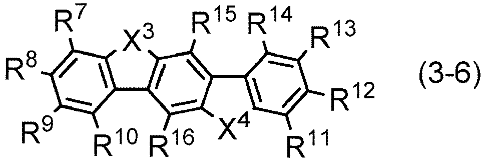
wherein X³, X⁴, and X⁵ each independently denote one of the group consisting of an oxygen atom, a sulfur atom, a selenium atom, a single bond, NR¹⁷, or CR¹⁸=CR¹⁹;
only one of combinations consisting of adjacent two of R⁷ to R¹⁰ constitutes Formula (4) below and only one of combinations consisting of adjacent two of R¹¹ to R¹⁴ constitutes Formula (4-2) below, each of which forms a 5- or 6-membered ring; and
remainders of R⁷ to R¹⁴ that do not constitute Formula (4) or Formula (4-2) below and R¹⁵ to R¹⁹ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2); 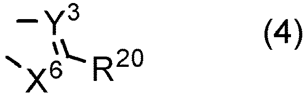
wherein X⁶ denotes one of the group consisting of an oxygen atom, a sulfur atom, a selenium atom, CR²¹=CR²², or NR²³;
Y³ denotes CR²⁴ or a nitrogen atom;
R²¹ to R²⁴ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2); and R²⁰ is the group represented by the above Formula (2); 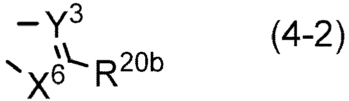
wherein X⁶, Y³, and R²¹ to R²⁴ have the same meaning as for X⁶, Y³, and R²¹ to R²⁴ in the above Formula (4); and R^{20b} denotes one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2).

4. The aromatic compound according to claim 3, wherein the aromatic compound represented by Formula (1-I) or Formula (1-II) is the compound represented by the above Formula (3-1) or the above Formula (3-2).

5. The aromatic compound according to claim 3 or 4, wherein, in the compound represented by one of the group consisting of Formulae (3-1) to (3-6), Formula (4-2) is Formula (4-3) below: 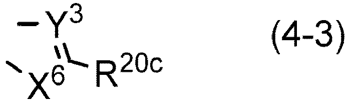 wherein X⁶, Y³, and R²¹ to R²⁴ have the same meaning as for X⁶, Y³, and R²¹ to R²⁴ in the above Formula (4); and R^{20c} denotes one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or a group represented by an aryl group having 4 to 26 carbon atoms.

6. The aromatic compound according to claim 3 or 4, wherein, in the compound represented by one of the group consisting of Formulae (3-1) to (3-6), Formula (4-2) is Formula (4-4) below: 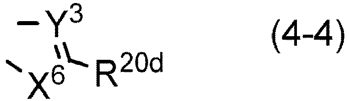 wherein X⁶, Y³, and R²¹ to R²⁴ have the same meaning as for X⁶, Y³, and R²¹ to R²⁴ in the above Formula (4) and R^{20d} is the group represented by the above Formula (2).

7. The aromatic compound according to any one of claims 1 to 4, wherein the aromatic compound represented by Formula (1-I) or Formula (1-II) is a compound represented by Formula (5) or Formula (5-2) below: 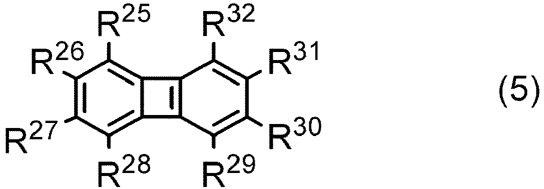 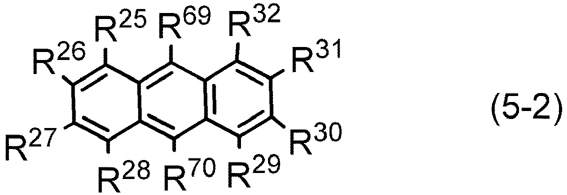
wherein only one of combinations consisting of adjacent two of R²⁵ to R²⁸ constitutes Formula (6) below and only one of combinations consisting of adjacent two of R²⁹ to R³² constitutes Formula (6-2) below, each of which forms a 5- or 6-membered ring; and
remainders of R²⁵ to R³² that do not constitute Formula (6) or Formula (6-2) below and R⁶⁹ and R⁷⁰ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2); 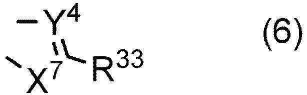
wherein X⁷ denotes an oxygen atom, a sulfur atom, a selenium atom, CR³⁴=CR³⁵, or NR³⁶;
Y⁴ denotes CR³⁷ or a nitrogen atom;
R³⁴ to R³⁷ each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2); and R³³ is the group represented by the above Formula (2); 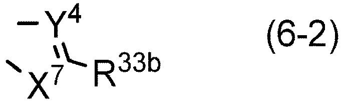
wherein X⁷, Y⁴, and R³⁴ to R³⁷ have the same meaning as for X⁷, Y⁴, and R³⁴ to R³⁷ in the above Formula (6); and R^{33b} denotes one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2).

8. The aromatic compound according to claim 7, wherein, in the aromatic compound represented by Formula (5) or Formula (5-2), Formula (6-2) is Formula (6-3) below: 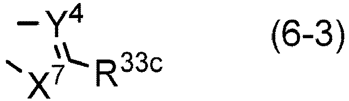 wherein X⁷, Y⁴, and R³⁴ to R³⁷ have the same meaning as for X⁷, Y⁴, and R³⁴ to R³⁷ in the above Formula (6); and R^{33c} denotes one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, or an aryl group having 4 to 26 carbon atoms.

9. The aromatic compound according to claim 7, wherein, in the aromatic compound represented by Formula (5) or Formula (5-2), Formula (6-2) is Formula (6-4) below: 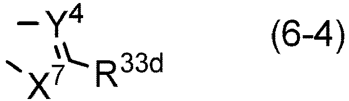 wherein X⁷, Y⁴, and R³⁴ to R³⁷ have the same meaning as for X⁷, Y⁴, and R³⁴ to R³⁷ in the above Formula (6); and R^{33d} is the group represented by the above Formula (2).

10. The aromatic compound according to claim 7, wherein the compound represented by Formula (5) or Formula (5-2) is a compound represented by one of the group consisting of Formulae (7-1) to (7-5): 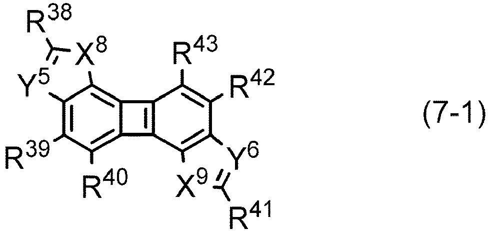 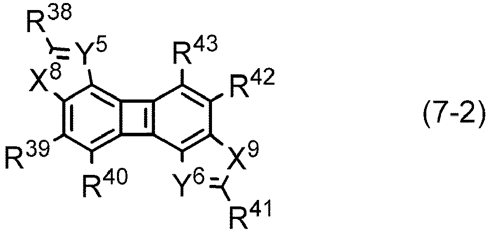 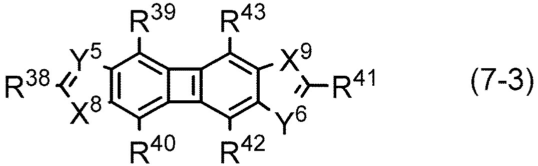 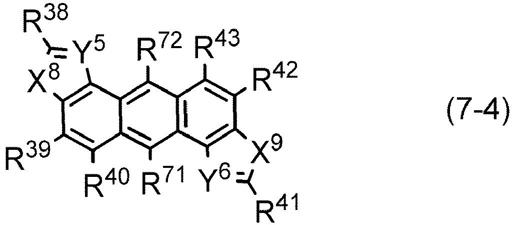 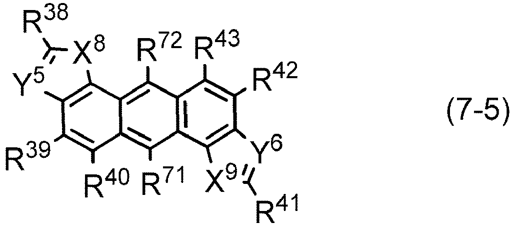
wherein X⁸ and X⁹ each independently denote an oxygen atom, a sulfur atom, a selenium atom, or NR⁴⁴;
Y⁵ and Y⁶ each independently denote CR⁴⁵ or a nitrogen atom;
R³⁸ to R⁴⁵, R⁷¹, and R⁷² each independently denote one of the group consisting of a hydrogen atom, a halogen atom, an alkyl group having 1 to 20 carbon atoms, an alkenyl group having 2 to 20 carbon atoms, an alkynyl group having 2 to 20 carbon atoms, an alkadienyl group having 4 to 22 carbon atoms, an alkadiynyl group having 4 to 22 carbon atoms, an aryl group having 4 to 26 carbon atoms, or the group represented by the above Formula (2) below; at least one of R³⁸ to R⁴⁵ is the group represented by the above Formula (2); and at least one of R³⁸ and R⁴¹ is the group represented by the above Formula (2).

11. The aromatic compound according to claim 10, wherein the compound represented by Formula (5) or Formula (5-2) is a compound represented by the above Formula (7-1), the above Formula (7-2), or the above Formula (7-5).

12. The aromatic compound according to claim 10 or 11, wherein R³⁸ and R⁴¹ each independently are one of the group consisting of the group represented by the above Formula (2), a hydrogen atom, a fluorine atom; and R³⁹, R⁴⁰, R⁴² to R⁴⁵, R⁷¹, and R⁷² are a hydrogen atom.

13. The aromatic compound according to any one of claims 10 to 12, wherein R³⁸ and R⁴¹ are the group represented by the above Formula (2); and R³⁹, R⁴⁰, R⁴² to R⁴⁵, R⁷¹, and R⁷² are a hydrogen atom.

14. A solution for forming an organic semiconductor layer, the solution comprising:
the aromatic compound according to any one of claims 1 to 13.

15. An organic semiconductor layer comprising:
the aromatic compound according to any one of claims 1 to 13.

16. An organic thin film transistor comprising:
the organic semiconductor layer according to claim 15.
